(19) Europäisches Patentamt
European Patent Office
Office européen des brevets

(11) EP 4 570 272 A1

(12) **EUROPEAN PATENT APPLICATION**
published in accordance with Art. 153(4) EPC

(43) Date of publication:
**18.06.2025 Bulletin 2025/25**

(21) Application number: **23852646.1**

(22) Date of filing: **10.08.2023**

(51) International Patent Classification (IPC):
***A61K 47/69*** *(2017.01)* ***A61K 9/51*** *(2006.01)*
***A61K 31/282*** *(2006.01)* ***A61K 39/395*** *(2006.01)*
***A61K 47/32*** *(2006.01)* ***A61K 47/68*** *(2017.01)*
***A61P 35/00*** *(2006.01)* ***C08F 220/10*** *(2006.01)*
***C08F 220/30*** *(2006.01)*

(52) Cooperative Patent Classification (CPC):
**A61K 9/51; A61K 31/282; A61K 39/395; A61K 47/32; A61K 47/68; A61K 47/69; A61P 35/00; C08F 220/10; C08F 220/30**

(86) International application number:
**PCT/JP2023/029356**

(87) International publication number:
**WO 2024/034684 (15.02.2024 Gazette 2024/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **10.08.2022 JP 2022128536**

(71) Applicant: **KOWA COMPANY, LTD.**
**Naka-ku**
**Nagoya-shi**
**Aichi 460-8625 (JP)**

(72) Inventors:
- **SUZUKI, Kenichi**
**Higashimurayama-shi, Tokyo 189-0022 (JP)**
- **YOSHIDA, Hideo**
**Higashimurayama-shi, Tokyo 189-0022 (JP)**
- **FUJIMAKI, Nobuhiro**
**Higashimurayama-shi, Tokyo 189-0022 (JP)**
- **HOSONO, Kahori**
**Higashimurayama-shi, Tokyo 189-0022 (JP)**

(74) Representative: **Wächtershäuser & Hartz Patentanwaltspartnerschaft mbB**
**Weinstraße 8**
**80333 München (DE)**

(54) **NEW DRUG COMPLEX**

(57) A novel copolymer utilizable for drug delivery technology.

The present invention relates to a drug complex in which a target recognition molecule is bonded to a copolymer X comprising structural units of (A), (B), and (C) .

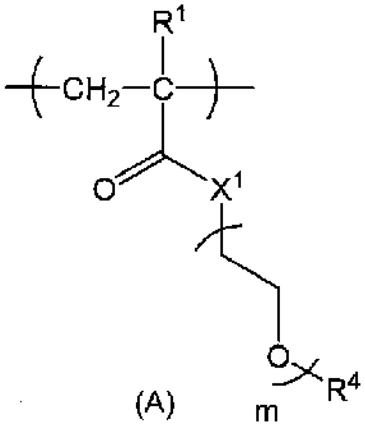

(A) ,

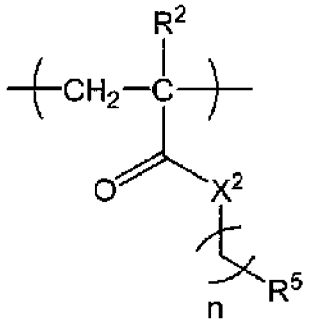

(B) ,

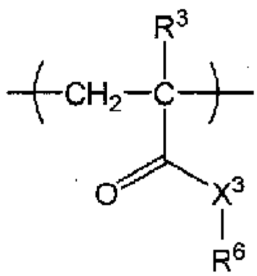

(C)

EP 4 570 272 A1

$R^1$, $R^2$, and $R^3$ are the same or different and represent hydrogen or $C_{1-3}$ alkyl, $R^4$ represents $C_{1-3}$ alkyl, $R^5$ represents hydrogen, $C_{1-18}$ alkyl, 3- to 8- membered cycloalkyl optionally having substituent, adamantyl, $C_{6-18}$ aryl optionally having substituent, or 5- to 10- membered heteroaryl group optionally having substituent, $X^1$, $X^2$, and $X^3$ are the same or different and represent oxygen, sulfur, or N-$R^7$, $R^6$ represents hydrogen, leaving group, or linker, $R^7$ represents hydrogen or $C_{1-3}$ alkyl group, m represents 1 to 100, and n represents 0 to 3.

FIG. 23

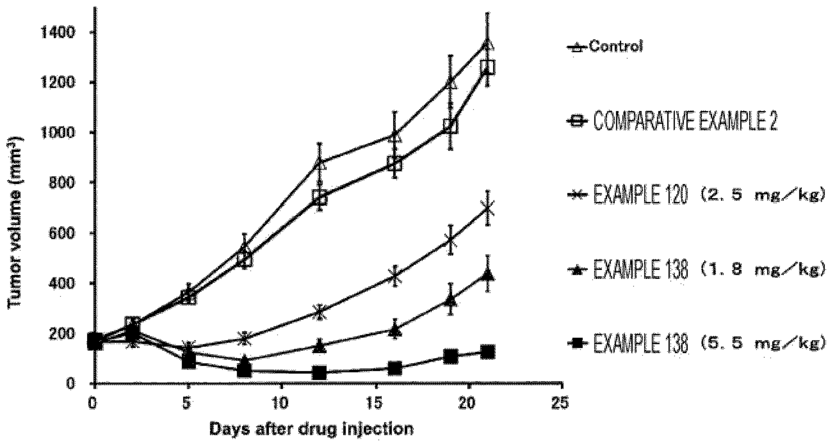

## Description

### Technical Field

The present invention relates to a novel copolymer useful for drug delivery technology. More specifically, the present invention relates to a copolymer for a drug delivery carrier targeting tumors, a pharmaceutical composition in which a physiologically active substance such as an anticancer agent is carried on copolymer, and a medicine containing the composition.

### Background Art

In recent years, research on drug delivery systems (DDS) has been energetically conducted as a technique for efficiently and safely delivering drugs to disease sites. Among them, there is an increasing demand for DDS employing nanoparticles as drug delivery carriers as a technique for enhancing the selectivity of drug accumulation by utilizing the structural characteristics of the disease site.

For example, in solid cancer tissue, since the structure of a neovascular vessel (tumor blood vessel) is immature as compared with a normal blood vessel, a cell gap of about several hundred nm is generated in the vascular endothelium, which allows the permeation of a large amount of substance. Due to this structural feature, it is known that a polymeric compound containing nanoparticles selectively permeates a tumor blood vessel and accumulates in solid cancer tissue. Furthermore, in solid cancer tissue, a lymphatic system involved in excretion of polymers malfunctions, so that permeated nanoparticles are continuously retained in the tissue (Enhanced permeability and retention effect, EPR effect). Since a general low molecular drug leaks out of a blood vessel due to membrane permeation of vascular cells, it is non-selectively distributed in tissue and does not accumulate in solid cancer tissue. According to the methodology of the EPR effect, nanoparticle-based drug delivery results in improved tissue selectivity to solid cancer in the distribution of a drug, since distribution to tissue is governed by the permeability of vascular endothelial cell gaps. Therefore, the EPR effect is a promising academic support in the development of nanotechnology-applied medicines (nanomedicine) targeting solid cancer.

It is believed that a drug delivery process in the EPR effect occurs through blood flow and that the extravasation process of nanoparticles is passive. Therefore, to maximize the accumulation of nanoparticles in solid cancer, it is important to impart a molecular design that can withstand long-term blood retention to the constituent components of nanoparticles used as drug delivery carriers. Drug delivery carriers are therefore required to have the ability to avoid barriers such as nonspecific interactions with blood components, foreign body recognition by the reticuloendothelial system (RES) in the liver, spleen, and lungs, and glomerular filtration in the kidneys. In addition, it is known that these barriers can be overcome by optimizing particle properties such as particle diameter and surface modification with a biocompatible polymer. For example, it is desirable that the particle diameter of the drug delivery carrier be greater than about 6 nm, which is the threshold for renal clearance, and less than 200 nm, which may avoid recognition by the RES.

The particle diameter of the drug delivery carrier is also known to affect tissue permeation at a disease site. For example, the anticancer activity of drug-encapsulating nanoparticles with particle diameters measuring 30 nm, 50 nm, 70 nm, and 100 nm, which exhibit equivalent blood retention, has been compared and studied, and it has been revealed that drug-encapsulating nanoparticles with a particle diameter of 30 nm reach the deep parts of a disease site and thus exhibit the highest therapeutic effect (Non-Patent Literature 1). Therefore, it would be desirable for the particle diameter of nanoparticles for a drug delivery carrier targeting solid cancer to be as small as possible within the range that avoids renal clearance.

As nanoparticles for drug delivery carriers, methods using colloidal dispersions such as liposomes, emulsions, or nanoparticles, methods using biologically derived raw materials such as albumin, methods using natural polymers such as natural polysaccharides, or methods using synthetic polymers, have been developed. Among them, synthetic polymers are widely used as constituents of drug delivery carriers because it is possible to prepare nanoparticles whose particle diameter is precisely controlled by appropriately selecting monomers as constituent components and synthesis methods.

For example, a method for utilizing an amphiphilic block copolymer composed of a hydrophilic segment and a hydrophobic segment as a drug delivery carrier is disclosed. The block copolymer spontaneously associates in an aqueous medium by, for example, hydrophobic interaction between molecules as a driving force to form core-shell type nanoparticles (polymeric micelles). It is known that it is possible to encapsulate a low molecular drug in or bind it to the hydrophobic segment of the polymeric micelle, and the obtained drug-encapsulating polymeric micelle exhibits high blood stability, and due to selective accumulation in solid cancer through the EPR effect, high anticancer activity as compared with the administration of a solution of a low molecular drug (Patent Literature 1). However, since the polymeric micelle is an assembly of multiple molecules, a particle diameter of about 30 nm is the lower limit value that can be prepared, and it is difficult to finely control the size in the vicinity of a particle diameter of 10 nm that can avoid the influence of renal clearance.

Meanwhile, among nanoparticles formed of a synthetic polymer, those which form particles by, for example,

chemical cross-linking, hydrophobic interaction, or ionic bonding within a single chain as a driving force (hereinafter abbreviated as single chain nanoparticles (SCNPs)) are known to form nanoparticles having a small particle diameter of 20 nm or less (Non-Patent Literature 2). Therefore, although SCNPs are expected to be useful as drug delivery carriers, a technique for precisely controlling their particle diameter has not been found so far.

Another drug delivery technology is an antibody-drug conjugate (ADC) that allows target delivery of a cytotoxic agent (drug) to antigen-expressing tumor cells (Non-Patent Literatures 3 to 5). The ADC has three components: an antibody (Ab), a linker, and a drug. In order to achieve topical delivery to the target, the drug is linked or conjugated to the antibody. A general conjugation method involves chemical modification via an amino group of a lysine side chain of the antibody or a cysteine sulfhydryl group obtained by reducing an interchain disulfide bond. The design of the ADC remains challenging and a plurality of elements (e.g., selection of antibodies, linker stability, drug/toxin (payload), and cleavage kinetics thereof) must be controlled. Here, an important parameter is the number of payloads to a single antibody (drug-antibody ratio (DAR)). An antibody to which many drugs/toxin molecules are bonded exhibits impaired binding to a target antigen or rapid in vivo clearance from the bloodstream, and generally only a limited number of the drugs/toxin molecules can be bonded to one antibody (typically, the DAR is 4 to 6). As a result, a highly toxic agent with an IC50 of less than 1 nM (e.g., calicheamicin or auristatin monomethyl ester (MMAE)) must be used to obtain efficacy sufficient to kill or damage the target cells (Non-Patent Literatures 6 and 7). Accordingly, if a small part of the conjugate acts on non-target cells, a serious off-target toxicity may appear. In this way, a new approach that combines high efficacy with improved tolerability has been desired.

## Citation List

### Patent Literature

Patent Literature 1: JP 3270592 B2

### Non-Patent Literature

Non-Patent Literature 1: H. Cabral et al., Nat. Nanotechnol. 6 815-823 (2011)

Non-Patent Literature 2: Jose A. Pomposo, Single-Chain Polymer Nanoparticles: Synthesis, Characterization, Simulations, and Applications (2017)

Non-Patent Literature 3: Chari, R.V. et al., Angew.Chem.Int.Ed. 53, 3796-3827 (2014)

Non-Patent Literature 4: Jagadeesh, D., Smith, M.R., Curr.Treat.Options Oncol. 17, 55 (2016)

Non-Patent Literature 5: Ducry, L., Stump, B., Bioconjugate Chem. 21, 5-13 (2010)

Non-Patent Literature 6: Casi, G., Neri, D., J.Controlled Release 161, 422-428 (2012)

Non-Patent Literature 7: Wu, A. M., Senter, P.D., Nat.Biotechnol. 23, 1137-1146 (2005)


## Summary of Invention

### Technical Problem

It is an object of the present invention to provide a copolymer for a drug delivery carrier targeting tumor. More specifically, it is an object of the present invention to provide a copolymer for a drug delivery carrier that can improve blood retention and/or tumor accumulation of a drug.

### Solution to Problem

To achieve the above-mentioned objects, the present inventors conducted extensive research and discovered that a terpolymer of an acrylic acid derivative has the property of forming SCNPs in water. Furthermore, the present inventors succeeded in creating a novel polymer for a drug delivery carrier, which is capable of precisely controlling the particle diameter of SCNPs at a minute scale of 20 nm or less and about 10 nm, and has high tumor accumulation. When a drug complex in which an anticancer agent was carried on or bonded to the polymer was administered to a mouse model with a subcutaneously implanted cancer, it exhibited an excellent antitumor effect.

The present invention relates to the following inventions:

[1] A copolymer in which a target recognition molecule is bonded to a copolymer X comprising structural units represented by the following formulas (A), (B), and (C) :

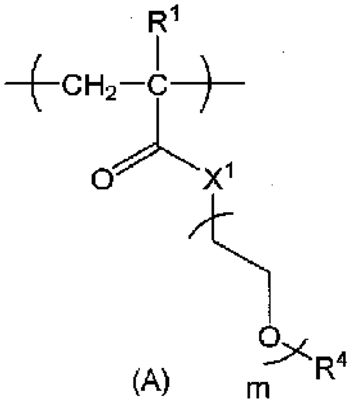
(A) ,
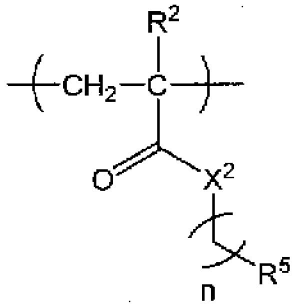
(B) ,
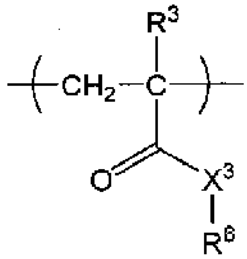
(C)

wherein, $R^1$, $R^2$, and $R^3$ are the same or different and represent a hydrogen atom or a $C_{1-3}$ alkyl group, $R^4$ represents a $C_{1-3}$ alkyl group, $R^5$ represents a hydrogen atom, a $C_{1-18}$ alkyl group, a 3- to 8-membered cycloalkyl group optionally having a substituent, an adamantyl group, a $C_{6-18}$ aryl group optionally having a substituent, or a 5-to 10-membered heteroaryl group optionally having a substituent, $X^1$, $X^2$, and $X^3$ are the same or different and represent an oxygen atom, a sulfur atom, or N-$R^7$, $R^6$ represents a hydrogen atom, a leaving group, or a linker; $R^7$ represents a hydrogen atom or a $C_{1-3}$ alkyl group, m represents an integer of 1 to 100, and n represents an integer of 0 to 3.

[2] The copolymer according to [1], wherein the copolymer X is a copolymer formed by polymerization of three types of monomers represented by the following general formulas (1) to (3):

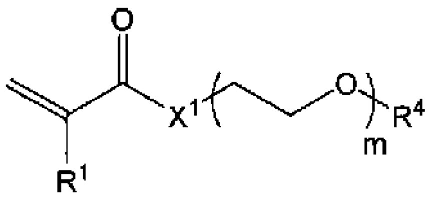
(1) ,
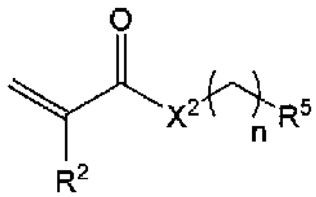
(2) ,
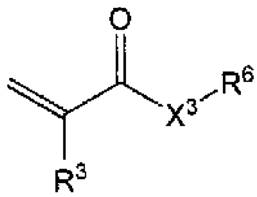
(3)

wherein, $R^1$, $R^2$, and $R^3$ are the same or different and represent a hydrogen atom or a $C_{1-3}$ alkyl group, $R^4$ represents a $C_{1-3}$ alkyl group, $R^5$ represents a hydrogen atom, a $C_{1-18}$ alkyl group, a 3- to 8-membered cycloalkyl group optionally having a substituent, an adamantyl group, a $C_{6-18}$ aryl group optionally having a substituent, or a 5-to 10-membered heteroaryl group optionally having a substituent, $X^1$, $X^2$, and $X^3$ are the same or different and represent an oxygen atom, a sulfur atom, or N-$R^7$, $R^6$ represents a hydrogen atom, a leaving group, or a linker, $R^7$ represents a hydrogen atom or a $C_{1-3}$ alkyl group, m represents an integer of 1 to 100, and n represents an integer of 0 to 3.

[3] The copolymer according to [1] or [2], wherein $R^1$ is a hydrogen atom.

[4] The copolymer according to any one of [1] to [3], wherein $R^2$ is a hydrogen atom.

[5] The copolymer according to any one of [1] to [4], wherein $R^3$ is a hydrogen atom.

[6] The copolymer according to any one of [1] to [5], wherein $R^4$ is a methyl group.

[7] The copolymer according to any one of [1] to [6], wherein $R^5$ is a $C_{6-18}$ aryl group optionally having a substituent.

[8] The copolymer according to any one of [1] to [7], wherein $R^5$ is a phenyl group.

[9] The copolymer according to any one of [1] to [8], wherein $R^6$ is a hydrogen atom.

[10] The copolymer according to any one of [1] to [8], wherein the leaving group of $R^6$ is a group represented by the following formula (4).

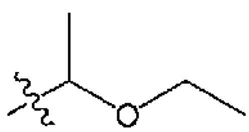
(4)

[11] The copolymer according to any one of [1] to [8], wherein the linker of $R^6$ is a group represented by the following formula (5):

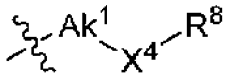

(5)

wherein, $R^8$ represents a hydrogen atom or a drug, $Ak^1$ represents a $C_{1-7}$ alkylene bond, and $X^4$ represents an oxygen atom, a sulfur atom, or $-N(R^7)-$ ($R^7$ represents a hydrogen atom or a $C_{1-3}$ alkyl group).

[12] The copolymer according to any one of [1] to [11], wherein $X^1$ is an oxygen atom.

[13] The copolymer according to any one of [1] to [12], wherein $X^2$ is an oxygen atom.

[14] The copolymer according to any one of [1] to [13], wherein $X^3$ is an oxygen atom or NH.

[15] The copolymer according to any one of [1] to [14], wherein m is an integer of 4 to 22.

[16] The copolymer according to any one of [1] to [15], wherein n is 1.

[17] The copolymer according to any one of [1] to [16], wherein a ratio of structural units (A), (B), and (C) is composed of 0.01 to 100 parts by mass of (B) and 0.1 to 100 parts by mass of (C) with respect to 1 part by mass of (A).

[18] The copolymer according to any one of [2] to [16], wherein 0.01 to 100 parts by mass of monomer (2) and 0.1 to 100 parts by mass of monomer (3) are polymerized with respect to 1 part by mass of monomer (1).

[19] The copolymer according to any one of [1] to [18], wherein the copolymer has a number average molecular weight of 5 000 to 150 000.

[20] The copolymer according to any one of [1] to [19], wherein the target recognition molecule is an antibody.

[21] The copolymer according to [20], wherein the antibody is an anti-EGFR antibody, an anti-Her2 antibody, an anti-CD20 antibody, an anti-CD276 antibody, an anti-MUC1 antibody, an anti-PD-L1 antibody, or an anti-TROP-2 antibody.

[22] The copolymer according to [20], wherein the antibody is cetuximab, panitumumab, necitumumab, amivantamab, panitumumab, trastuzumab, pertuzumab, margetuximab, rituximab, ibritumomab, tositumomab, ofatumumab, obinutuzumab, clivatuzumab, gatipotuzumab, ifinatamab, mirzotamab, vobramitamab, atezolizumab, avelumab, durvalumab, sacituzumab, or functional fragments thereof.

[23] A drug complex including the copolymer according to any one of [1] to [22] and a drug.

[24] The drug complex according to [23], wherein the drug is an antimetabolite, an alkylating agent, an anthracycline, an antibiotic, a mitotic inhibitor, a topoisomerase inhibitor, a proteasome inhibitor, or an anti-hormone agent.

[25] The drug complex according to [23], wherein the drug is DM0, DM1, DM2, DM3, DM4, emtansine, auristatin E, auristatin phenylalanine phenylenediamine (AFP), monomethyl auristatin E, monomethyl auristatin D, monomethyl auristatin F, paclitaxel, docetaxel, irinotecan, topotecan, nogitecan, amsacrine, etoposide, teniposide, mizantrone, SN-38, exatecan, or deruxtecan.

[26] The drug complex according to any one of [23] to [25], wherein a bond of the target recognition molecule or the drug to the copolymer X is a covalent bond or a noncovalent bond.

[27] The drug complex according to any one of [23] to [26], wherein the bond of the target recognition molecule or the drug to the copolymer X is represented by the following formula (a):

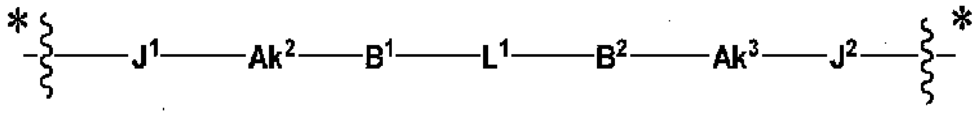

(a)

wherein, $J^1$ is a bond to the target recognition molecule or the drug, $J^2$ is a bond to the copolymer X, $Ak^2$ and $Ak^3$ each independently represent a single bond or a $C_{1-7}$ alkylene bond, $B^1$ and $B^2$ each independently represent a single bond, an amide bond, or an ester bond, $L^1$ represents a single bond, $-(CH_2CH_2O)_oCH_2CH_2-$, phenylene, cyclohexylene, -NH-peptide-CO-, or phenylene-NH-peptide-CO-, and o represents an integer of 0 to 100.

[28] A single chain nanoparticle including the copolymer or the drug complex according to any one of [1] to [27].

[29] A pharmaceutical composition including the copolymer or the drug complex according to any one of [1] to [28].

Advantageous Effects of Invention

As will be evident from Examples described below, an SCNP obtained by self-association of the copolymer of the present invention carrying or binding to an anticancer agent, exhibited a tumor growth suppressing effect in a mouse tumor-bearing model, and therefore can be applied as a therapeutic agent for malignant tumor. Since the SCNP obtained

by self-association of the copolymer of the present invention carrying or binding to the anticancer agent has a high DAR as compared with an existing ADC, and has a high tumor growth suppressing effect at a low dose, it is possible to provide a therapeutic agent for malignant tumor capable of achieving both enhancement of a pharmacological action and suppression of side effects. Then, the copolymer to which the target recognition molecule of the present invention is bonded is useful as a drug delivery utilizing a target-specific target recognition molecule.

Brief Description of Drawings

Fig. 1 is a diagram showing a $^1$H-NMR spectrum of a copolymer obtained in Example 1 measured by using nuclear magnetic resonance (NMR).
Fig. 2 is a diagram showing a chromatogram of a copolymer obtained in Example 1 obtained by gel permeation chromatography (GPC).
Fig. 3 is a diagram showing a particle diameter measurement result (scattering intensity distribution) in dynamic light scattering (DLS) for a copolymer before encapsulating DACHPt (Example 69) and a DACHPt-encapsulating SCNP (Example 70).
Fig. 4 is a diagram showing a $^1$H-NMR spectrum of $N_3$-poly[(benzyl acrylate)-co-(poly(ethylene glycol)methyl ether acrylate)-co-(acrylic acid)] obtained in Example 71, measured by using the nuclear magnetic resonance (NMR).
Fig. 5 is a diagram showing a chromatogram obtained by the gel permeation chromatography (GPC) for $N_3$-poly[(benzyl acrylate)-co-(poly(ethylene glycol)methyl ether acrylate)-co-(acrylic acid)] obtained in Example 71.
Fig. 6 is a diagram showing a UV spectrum of $N_3$-poly[(benzyl acrylate)-co-(poly(ethylene glycol)methyl ether acrylate)-co-(acrylic acid)]-isobutyronitrile obtained in Example 92, measured by using UV spectrum measurement (UV).
Fig. 7 is a diagram showing a $^1$H-NMR spectrum of a DM1-cysteamine-bonded copolymer obtained in Example 96, measured by using the nuclear magnetic resonance (NMR).
Fig. 8 is a diagram showing a $^1$H-NMR spectrum of a DM1-N-4-APM-bonded copolymer obtained in Example 108, measured by using the nuclear magnetic resonance (NMR).
Fig. 9 is a diagram showing a $^1$H-NMR spectrum of a 1,4-diaminobutane-bonded copolymer obtained in Example 109, measured by using the nuclear magnetic resonance (NMR).
Fig. 10 is a diagram showing a $^1$H-NMR spectrum of a DM1-MHA-1,4-diaminobutane-bonded copolymer obtained in Example 117, measured by using the nuclear magnetic resonance (NMR).
Fig. 11 is a diagram showing a $^1$H-NMR spectrum of a DM1-SPDP-1,4-diaminobutane-bonded copolymer obtained in Example 118, measured by using the nuclear magnetic resonance (NMR).
Fig. 12 is a diagram showing a $^1$H-NMR spectrum of a DM1-SMCC-1,4-diaminobutane-bonded copolymer obtained in Example 122, measured by using the nuclear magnetic resonance (NMR).
Fig. 13 is a diagram showing a $^1$H-NMR spectrum of a DM1-CL-031-1,4-diaminobutane-bonded copolymer obtained in Example 124, measured by using the nuclear magnetic resonance (NMR).
Fig. 14 is a diagram showing a $^1$H-NMR spectrum of a DM1-CL-018-1,4-diaminobutane-bonded copolymer obtained in Example 125, measured by using the nuclear magnetic resonance (NMR).
Fig. 15 is a diagram showing a $^1$H-NMR spectrum of a DM1-CL-038-1,4-diaminobutane-bonded copolymer obtained in Example 126, measured by using the nuclear magnetic resonance (NMR).
Fig. 16 is a diagram showing a $^1$H-NMR spectrum of a DM1-CL-047-1,4-diaminobutane-bonded copolymer obtained in Example 127, measured by using the nuclear magnetic resonance (NMR).
Fig. 17 is a diagram showing a $^1$H-NMR spectrum of a SN-38-CO-1,4-diaminobutane-bonded copolymer obtained in Example 128, measured by using the nuclear magnetic resonance (NMR).
Fig. 18 is a diagram showing a $^1$H-NMR spectrum of a deruxtecan-SPDP-1,4-diaminobutane-bonded copolymer obtained in Example 129, measured by using the nuclear magnetic resonance (NMR).
Fig. 19 is a diagram showing a $^1$H-NMR spectrum of a 4-hydroxybutylamine-bonded copolymer obtained in Example 130, measured by using the nuclear magnetic resonance (NMR) .
Fig. 20 is a diagram showing a $^1$H-NMR spectrum of a staurosporine (STS)-bonded copolymer obtained in Example 131, measured by using the nuclear magnetic resonance (NMR) .
Fig. 21 is a diagram showing a $^1$H-NMR spectrum of an exatecan-PAB-Cit-Val-Ahx-bonded copolymer obtained in Example 158, measured by using the nuclear magnetic resonance (NMR).
Fig. 22 is a diagram showing a change in relative tumor volume when an oxaliplatin solution or the DACHPt-encapsulating SCNP (Example 70) was administered 3 times every other day to a model mouse having a back subcutaneously transplanted with a mouse colon cancer cell line (C26).
Fig. 23 is a diagram showing a change in tumor volume when physiological saline or cetuximab-bonded micelle drug

complex (Example 138) was administered to a model mouse having a right ventral portion subcutaneously transplanted with a mouse EGFR-positive human colon cancer cell line HT-29.

Fig. 24 is a diagram showing a change in tumor volume when the physiological saline or trastuzumab-bonded micelle drug complex (Example 151) was administered to a model mouse having a right ventral portion subcutaneously transplanted with a mouse HER2-positive human gastric cancer cell line NCI-N87.

Fig. 25 is a diagram showing a change in tumor volume when the physiological saline or cetuximab-bonded micelle drug complex (Example 160 or Example 161) was administered to a model mouse having a right ventral portion subcutaneously transplanted with a mouse EGFR-positive human breast cancer cell line MDA-MB-468.

Fig. 26 is a diagram showing a change in tumor volume when the physiological saline or cetuximab-bonded micelle drug complex (Example 162 or Example 163) was administered to a model mouse having a right ventral portion subcutaneously transplanted with an EGFR-positive human colon cancer cell line HCT-116 having KRAS mutation (G13D).

Description of Embodiments

Terms in the present description are used in the meanings commonly used in the field unless otherwise specified. Hereinafter, the present invention will be described in more detail.

In the present description, the term "nanoparticle" refers to a structure showing a particle diameter of 100 nm or less.

In the present description, the term "single chain nanoparticle (SCNP)" refers to a nanoparticle formed by using, for example, chemical cross-linking, hydrophobic interaction, or ionic bonding in a single chain as a driving force. The SCNP often indicates a nanoparticle having a relatively small particle diameter of 20 nm or less among nanoparticles.

In the present description, the term "initiator" means an initiator for thermal radical polymerization such as an azo compound or a peroxide.

In the present description, the term "chain transfer agent" refers to a compound that causes a chain transfer reaction in radical polymerization, and is preferably a compound having a thiocarbonyl group.

In the present description, the term "$C_{1-3}$ alkyl group" means a linear or branched alkyl group having 1 to 3 carbon atoms, and examples thereof include a methyl group, an ethyl group, an n-propyl group, and an isopropyl group.

In the present description, the term "$C_{1-18}$ alkyl group" means a linear or branched, alkyl group having 1 to 18 carbon atoms, and examples thereof include a methyl group, an ethyl group, an n-propyl group, an isopropyl group, an n-butyl group, an isobutyl group, a sec-butyl group, a tert-butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, and an octadecyl group.

In the present description, the term "3- to 8-membered cycloalkyl group optionally having a substituent" means a cyclic alkyl group having 3 to 8 carbon atoms, and examples thereof include a cyclopropyl group, a cyclobutyl group, a cyclopentyl group, a cyclohexyl group, a cycloheptyl group, and a cyclooctyl group. The substituent is not particularly limited, and examples thereof include a halogen atom, an alkyl group having 1 to 6 carbon atoms, an alkenyl group having 2 to 6 carbon atoms, an alkynyl group having 2 to 6 carbon atoms, a hydroxyl group, an alkoxy group having 1 to 6 carbon atoms, an amino group, an alkylamino group having 1 to 6 carbon atoms, a di-alkylamino group having 1 to 6 carbon atoms in which alkyl groups are the same or different, a thiol group, an alkylthio group having 1 to 6 carbon atoms, a carboxyl group, an alkoxycarbonyl group having 1 to 6 carbon atoms, and a carbamoyl group.

In the present description, the term "$C_{6-18}$ aryl group optionally having a substituent" means a monocyclic or polycyclic condensed aromatic hydrocarbon group, and examples thereof include a phenyl group, a naphthyl group, an anthracenyl group, a phenanthrenyl group, a triphenylenyl group, a pyrenyl group, a chrysenyl group, and a naphthacenyl group. Further, a "$C_{6-14}$ aryl group optionally having a substituent" means a monocyclic or polycyclic condensed aromatic hydrocarbon group, and examples thereof include a phenyl group, a naphthyl group, an anthracenyl group, and a phenanthrenyl group. The substituent is not particularly limited, and examples thereof include a halogen atom, an alkyl group having 1 to 6 carbon atoms, an alkenyl group having 2 to 6 carbon atoms, an alkynyl group having 2 to 6 carbon atoms, a hydroxyl group, an alkoxy group having 1 to 6 carbon atoms, an amino group, an alkylamino group having 1 to 6 carbon atoms, a di-alkylamino group having 1 to 6 carbon atoms in which alkyl groups are the same or different, a thiol group, an alkylthio group having 1 to 6 carbon atoms, a carboxyl group, an alkoxycarbonyl group having 1 to 6 carbon atoms, and a carbamoyl group.

In the present description, the term "5- to 10-membered heteroaryl group optionally having a substituent" means a 5- to 10-membered, monocyclic aromatic heterocyclic group or condensed aromatic heterocyclic group containing 1 to 4 heteroatoms selected from a nitrogen atom, an oxygen atom, and a sulfur atom, other than a carbon atom, as atoms constituting the ring. Examples of the monocyclic aromatic heterocyclic group include a furyl group, a thienyl group, a pyrrolyl group, a pyridinyl group, a pyrazinyl group, a pyrimidinyl group, a pyridazinyl group, an imidazolyl group, a pyrazyl group, a thiallyl group, an oxazolyl group, an isoxazolyl group, a 1,3,4-thiadiazolyl group, a 1,2,3-triazolyl group, a 1,2,4-triazolyl group, and a tetrazolyl group. Examples of the condensed aromatic heterocyclic group include a benzofuranyl group, a benzothiophenyl group, a quinoxalinyl group, an indolyl group, an isoindolyl group, an isobenzofuranyl group, a chromanyl group, a benzimidazolyl group, a benzothiazolyl group, a benzoxazolyl group, a quinolyl group, and an isoquinolinyl group. The term "6- to 10-membered heteroaryl group optionally having a substituent" means a 6- to 10-membered, monocyclic aromatic heterocyclic group or condensed aromatic heterocyclic group containing 1 to 4 heteroatoms selected from a nitrogen atom, an oxygen atom, and a sulfur atom, other than a carbon atom, as the atoms constituting the ring. Examples of the monocyclic aromatic heterocyclic group include a pyridinyl group, a pyrazinyl group, a pyrimidinyl group, and a pyridazinyl group. Examples of the condensed aromatic heterocyclic group include a benzofuranyl group, a benzothiophenyl group, a quinoxalinyl group, an indolyl group, an isoindolyl group, an isobenzofuranyl group, a chromanyl group, a benzimidazolyl group, a benzothiazolyl group, a benzoxazolyl group, a quinolyl group, and an isoquinolinyl group. The substituent is not particularly limited, and examples thereof include a halogen atom, an alkyl group having 1 to 6 carbon atoms, an alkenyl group having 2 to 6 carbon atoms, an alkynyl group having 2 to 6 carbon atoms, a hydroxyl group, an alkoxy group having 1 to 6 carbon atoms, an amino group, an alkylamino group having 1 to 6 carbon atoms, a di-alkylamino group having 1 to 6 carbon atoms in which alkyl groups are the same or different, a thiol group, an alkylthio group having 1 to 6 carbon atoms, a carboxyl group, an alkoxycarbonyl group having 1 to 6 carbon atoms, and a carbamoyl group.

In the present description, examples of the "halogen atom" include a fluorine atom, a chlorine atom, a bromine atom, and an iodine atom.

In the present description, the term "$C_{1-7}$ alkylene bond" means a linear or branched, alkylene group having 1 to 7 carbon atoms, which may be substituted, and examples thereof include a methylene group, an ethylene group, a propylene group, a butylene group, a pentylene group, a hexylene group, a heptylene group, $-CH(CH_3)-$, $-C(CH_3)_2-$, $-CH(CH_2CH_3)-$, $-CH(CH_3)CH_2-$, $-CH(CH_2CH_2CH_3)-$, $-CH(CH_2(CH_3)_2)-$, $-C(CH_3)(CH_2CH_3)-$, $-C(CH_3)_2CH_2-$, $-CH(CH_2CH_3)CH_2$, $-CH(CH_3)CH(CH_3)-$, $-CH(CH_3)CH_2CH_2-$, $-CH_2CH(CH_3)CH_2-$, $-CH(CH_2CH_2CH_2CH_3)-$, $-C(CH_3)(CH_2CH_2CH_3)-$, $-C(CH_2CH_3)_2-$, $-CH(CH_2CH_2CH_3)CH_2-$, $-CH(CH_2(CH_3)_2)CH_2-$, $-C(CH_3)(CH_2CH_3)CH_2-$, $-C(CH_3)_2CH(CH_3)-$, $-CH(CH_2CH_3)CH(CH_3)-$, $-C(CH_3)_2CH_2CH_2-$, $-CH(CH_2CH_3)CH_2CH_2-$, $-CH_2CH(CH_2CH_3)CH_2-$, $-CH(CH_3)CH(CH_3)CH_2-$, $-CH(CH_3)CH_2CH(CH_3)-$, $-CH(CH_2CH_2CH_2CH_2CH_3)-$, $-C(CH_3)(CH_2CH_2CH_2CH_3)-$, $-C(CH_2CH_3)(CH_2CH_2CH_3)-$, $-C(CH_2CH_3)(CH_2(CH_3)_2)-$, $-CH(CH_2CH_2CH_2CH_3)CH_2-$, $-CH(CH(CH_3)CH_2CH_3)CH_2-$, $-CH(CH_2CH(CH_3)CH_3)CH_2-$, $-CH(CH_2CH_2(CH_3)_2)CH_2-$, $-C(CH_3)(CH_2CH_2CH_3)CH_2-$, $-C(CH_2CH_3)_2CH_2-$, $-CH(CH_2(CH_3)_2)CH_2-$, $-C(CH_3)(CH_2CH_3)CH(CH_3)-$, $-CH(CH_2CH_3)C(CH_3)_2-$, $-CH(CH_2CH_3)CH(CH_2CH_3)-$, $-C(CH_3)_2C(CH_3)_2-$, $-CH(CH_2CH_2CH_3)CH_2CH_2-$, $-CH(CH_2(CH_3)_2)CH_2CH_2-$, $-CH_2CH(CH_2CH_2CH_3)CH_2-$, $-CH_2CH(CH_2(CH_3)_2)CH_2-$, $-C(CH_3)(CH_2CH_3)CH_2CH_2-$, $-CH(CH_2CH_3)CH(CH_3)CH_2-$, $-CH(CH_2CH_3)CH_2CH(CH_3)-$, $-CH_2CH(CH_2CH_3)CH(CH_3)-$, $-CH(CH_3)CH(CH_3)CH(CH_3)-$, $-C(CH_3)_2CH(CH_3)CH_2-$, $-C(CH_3)_{2C}H_2CH(CH_3)-$, $-CH(CH_2CH_3)CH_2CH_2CH_2-$, $-CH_2CH(CH_2CH_3)CH_2CH_2-$, $-C(CH_3)_2CH_2CH_2CH_2-$, $-CH(CH_3)CH(CH_3)CH_2CH_2-$, $-CH(CH_3)CH_2CH(CH_3)CH_2-$, $-CH(CH_3)CH_2CH_2CH(CH_3)-$, $-CH(CH_3)CH_2CH_2CH_2CH_2-$, $-CH_2CH(CH_3)CH_2CH_2CH_2-$, $-CH_2CH_2CH(CH_3)CH_2CH_2-$, $-C(CH_2CH_3)(CH_2CH_2CH_2CH_3)-$, $-C(CH_2CH_2CH_3)_2-$, $-C(CH_3)(CH_2CH_2CH_2CH_3)CH_2-$, $-C(CH_3)(CH(CH_3)CH_2CH_3)CH_2-$, $-C(CH_3)(CH_2CH(CH_3)CH_3)CH_2-$, $-C(CH_3)(CH_2CH_2(CH_3)_2)CH_2-$, $-CH(CH_2CH_2CH_2CH_3)CH(CH_3)-$, $-CH(CH(CH_3)CH_2CH_3)CH(CH_3)-$, $-CH(CH_2CH(CH_3)CH_3)CH(CH_3)-$, $-CH(CH_2CH_2(CH_3)_2)CH(CH_3)-$, $-C(CH_3)(CH_2CH_2CH_3)CH(CH_3)-$, $-C(CH_2CH_3)_2CH(CH_3)-$, $-CH(CH_2(CH_3)_2)CH(CH_3)-$, $-C(CH_3)(CH_2CH_3)C(CH_3)_2-$, $-C(CH_3)(CH_2CH_2CH_3)CH_2CH_2-$, $-CH(CH_2CH_2CH_3)CH(CH_3)CH_2-$, $-CH(CH_2CH_2CH_3)CH_2CH(CH_3)-$, $-CH_2CH(CH_2CH_2CH_3)CH(CH_3)-$, $-C(CH_3)(CH_2CH_3)CH(CH_3)CH_2-$, $-C(CH_3)(CH_2CH_3)CH_2CH(CH_3)-$, $-CH(CH_2CH_3)C(CH_3)_2CH_2-$, $-CH(CH_2CH_3)CH(CH_3)CH(CH_3)-$, $-CH(CH_2CH_3)CHCH(CH_3)_2-$, $-C(CH_3)_2CH(CH_2CH_3)CH_2-$, $-CH(CH_3)C(CH_3)(CH_2CH_3)CH_2-$, $-CH(CH_3)CH(CH_2CH_3)CH(CH_3)-$, $-C(CH_3)_2CH(CH_3)CH(CH_3)-$, $-CH(CH_3)C(CH_3)_2CH(CH_3)-$, $-C(CH_3)_2CH(CH_3)CH_2CH_2-$, $-C(CH_3)_2CH_2CH(CH_3)CH_2-$, $-C(CH_3)_2CH_2CH_2CH(CH_3)-$, $-CH(CH_3)C(CH_3)_2CH_2CH_2-$, $-CH(CH_3)CH(CH_3)CH(CH_3)CH_2-$, $-CH(CH_3)CH(CH_3)CH_2CH(CH_3)-$, $-CH(CH_3)CH(CH_3)CH_2CH_2CH_2-$, $-CH(CH_3)CH_2CH(CH_3)CH_2CH_2-$, $-CH(CH_3)CH_2CH_2CH(CH_3)CH_2-$, $-CH(CH_3)CH_2CH_2CH_2CH(CH_3)-$, $-C(CH_3)_2CH_2CH_2CH_2CH_2-$, and $-CH(CH_2CH_3)CH_2CH_2CH_2CH_2$. The substituent is not particularly limited, and can substitute any hydrogen atom, and examples thereof include a phenoxy group, a carboxylic acid, a sulfonic acid, a phosphoric acid, a hydroxyl group, and a thiol group.

In the present description, the term "target recognition molecule" means a molecule that recognizes and specifically binds to a marker or a receptor (e.g., a transmembrane protein, a surface insolubilized protein, or a proteoglycan) expressed on a cell surface. The target recognition molecule is a molecule that specifically recognizes a target specific to or overexpressed in cancer cells necessary for cancer growth or metastasis, and examples thereof include antibodies, lipocalins (e.g., anticalins), proteins (e.g., interferons, lymphokines, growth factors, or colony stimulating factors), peptides (e.g., an LHRH receptor targeting peptide, or an EC-1 peptide), and peptidomimetics. In addition to having a bond specificity, the target recognition molecule may also have certain therapeutic effects, such as an antiproliferative activity (cell proliferation inhibitory and/or cytotoxicity), on a target cell or pathway. The target recognition molecule may be carried on the copolymer by an action such as electrostatic interaction, hydrogen bond, hydrophobic interaction, or covalent bond to the copolymer X of the present invention. The target recognition molecule can be modified

to an extent that the binding specificity is maintained, and can be bonded to the copolymer X via a chemically reactive group (e.g., carboxylic acid, primary amine, secondary amine, or thiol), a chemically reactive amino acid residue, or a side chain thereof (e.g., tyrosine, histidine, cysteine, lysine). Hereinafter, the copolymer X of the present invention bonded to or carried with the target recognition molecule may be referred to as a "target-recognizable copolymer".

In the present description, the term "antibody" is a molecule having a characteristic of immunospecifically binding to a target antigen and having a sequence derived from an immunoglobulin such as IgG, IgM, IgA, IgD, or IgE, or functional fragments thereof. Monoclonal antibodies, chimeric antibodies, recombinant antibodies, or humanized antibodies are included. The antibody may have an idiotype, and examples of an antigen binding fragment include a Fab region, an $F(ab')_2$ fragment, a pFc' fragment, and a Fab' fragment. The Fab region includes one constant domain and one variable domain derived from heavy and light chains of the antibody. The Fc fragment and the Fab fragment are fragments derived from an immunoglobulin cleaved by an enzyme papain. The $F(ab')_2$ fragment and the pFc' fragment are fragments derived from an immunoglobulin cleaved by an enzyme pepsin. The Fab' fragment is obtained by reducing the $F(ab')_2$ fragment under mild conditions. As a further form, it is also possible to use an Fc-fused protein in which a functional protein such as a receptor extracellular domain and an Fc domain of the immunoglobulin are fused. It is also possible to use a bispecific antibody capable of binding to two types of antigens or a multi-specific antibody having an additional antigen-binding site. Moreover, chemical or biological modification can also be made. Where gene recombination technology is used, it is sufficient that characteristics of an original amino acid sequence are maintained, and deletion, substitution, insertion, or addition of one or more amino acids is allowed simultaneously or separately. For example, the amino acid sequence is an amino acid sequence having a homology of 80% or more, preferably 90% or more, and more preferably, an amino acid sequence having a homology of 95% or more.

In the present description, the term "pharmaceutical composition" means one in which active ingredients (drug, physiologically active substance) that can be used for diagnosis, prevention, or treatment of a disease are carried on the copolymer X, the target-recognizable copolymer, or the drug complex of the present invention by an action such as electrostatic interaction, hydrogen bond, hydrophobic interaction, or covalent bond. Examples of the carrying form include a form in which the drug is present on the particle surface, a form in which the drug is contained in the nanoparticle, and a combination form thereof where the copolymer X, the target-recognizable copolymer, or the drug complex forms the nanoparticle.

One embodiment of the present invention is a copolymer or a drug complex in which a target recognition molecule is bonded to a copolymer X comprising structural units represented by the following formulas (A), (B), and (C) .

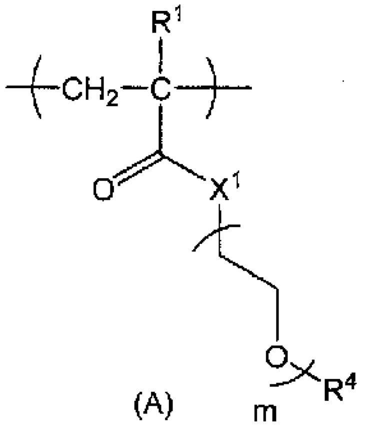
(A) ,

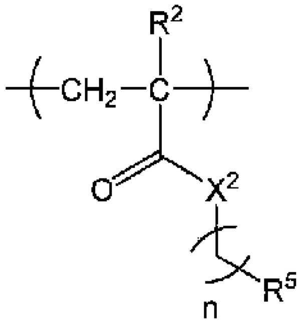
(B) ,

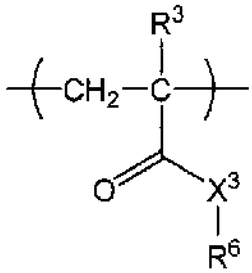
(C)

wherein, $R^1$, $R^2$, and $R^3$ are the same or different and represent a hydrogen atom or a $C_{1-3}$ alkyl group, $R^4$ represents a $C_{1-3}$ alkyl group, $R^5$ represents a hydrogen atom, a $C_{1-18}$ alkyl group, a 3- to 8-membered cycloalkyl group optionally having a substituent, an adamantyl group, a $C_{6-18}$ aryl group optionally having a substituent, or a 5-to 10-membered heteroaryl group optionally having a substituent, $X^1$, $X^2$, and $X^3$ are the same or different and represent an oxygen atom, a sulfur atom, or N-$R^7$, $R^6$ represents a hydrogen atom, a leaving group, or a linker, $R^7$ represents a hydrogen atom or a $C_{1-3}$ alkyl group, m represents an integer of 1 to 100, and n represents an integer of 0 to 3.

In the copolymer X of the present invention, the structural unit (A) functions as a unit that imparts hydrophilicity, and the structural unit (B) functions as a unit that imparts hydrophobicity. Further, the structural unit (C) functions as a scaffold in which the active ingredient (drug, or physiologically active substance) is bonded to the copolymer X or the target-recognizable copolymer. Having these three structural units serves to imparting the copolymer X, the target-recognizable copolymer, or the drug complex of the present invention a property of forming SCNP in water, and to facilitating the formed SCNP to precisely control particle diameter at a minute scale of 20 nm or less, and to function as a drug delivery carrier having high tumor accumulation.

$R^1$ in the structural unit (A) represents a hydrogen atom or a $C_{1-3}$ alkyl group, and is preferably a hydrogen atom or a methyl group, preferably a hydrogen atom, an ethyl group, or a propyl group, and more preferably a hydrogen atom.

$X^1$ represents an oxygen atom, the sulfur atom, or N-$R^7$, and is preferably an oxygen atom, a sulfur atom, or NH, and more preferably an oxygen atom.

m represents an integer of 1 to 100, is preferably an integer of 3 to 100, and from a viewpoint of imparting good hydrophilicity, preferably 3 to 80, more preferably 4 to 60, still more preferably 4 to 40, and even more preferably 4 to 22.

$R^4$ represents a $C_{1-3}$ alkyl group, specifically a methyl group, an ethyl group, an n-propyl group or an isopropyl group, preferably a methyl group or an ethyl group, and more preferably a methyl group.

$R^2$ in the structural unit (B) represents a hydrogen atom or a $C_{1-3}$ alkyl group, and is preferably a hydrogen atom or a methyl group, preferably a hydrogen atom, an ethyl group, or a propyl group, and more preferably a hydrogen atom.

$X^2$ represents an oxygen atom, a sulfur atom, or N-$R^7$, and is preferably an oxygen atom, a sulfur atom, or NH, and more preferably an oxygen atom.

n represents an integer of 0 to 3, preferably an integer of 1 to 3, and more preferably 1.

$R^5$ represents a hydrogen atom, a $C_{1-18}$ alkyl group, a 3- to 8-membered cycloalkyl group optionally having a substituent, an adamantyl group, a $C_{6-18}$ aryl group optionally having a substituent, or a 5- to 10-membered heteroaryl group optionally having a substituent, and from a viewpoint of imparting the hydrophobicity to the structural unit (B), preferably a $C_{1-18}$ alkyl group, a 3- to 8-membered cycloalkyl group optionally having a substituent, an adamantyl group, a $C_{6-18}$ aryl group optionally having a substituent, or a 5- to 10-membered heteroaryl group optionally having a substituent, more preferably a $C_{1-18}$ alkyl group, a 3- to 8-membered cycloalkyl group optionally having a substituent, an adamantyl group, a $C_{6-18}$ aryl group optionally having a substituent, or a 5- to 10-membered heteroaryl group optionally having a substituent, and still more preferably a $C_{1-18}$ alkyl group, a 3- to 8-membered cycloalkyl group, an adamantyl group, or a $C_{6-18}$ aryl group. Meanwhile, a 3- to 8-membered cycloalkyl group optionally having a substituent, an adamantyl group, a $C_{6-14}$ aryl group optionally having a substituent, or a 6- to 10-membered heteroaryl group optionally having a substituent is also preferable. Here, the substituent is preferably one or more types selected from a halogen atom, an alkyl group having 1 to 6 carbon atoms, an alkenyl group having 2 to 6 carbon atoms, and an alkynyl group having 2 to 6 carbon atoms.

$R^3$ in the structural unit (C) represents a hydrogen atom or a $C_{1-3}$ alkyl group, and is preferably a hydrogen atom or a methyl group, preferably a hydrogen atom, an ethyl group, or a propyl group, and more preferably a hydrogen atom.

$X^3$ represents an oxygen atom, a sulfur atom, or N-$R^7$, and is preferably an oxygen atom, a sulfur atom, or NH, and more preferably an oxygen atom or NH.

$R^6$ represents a hydrogen atom, a leaving group, or a linker. The leaving group is a group that can detach when the structural unit (C) binds to the drug (physiologically active substance), and the linker is a group that can be used for crosslinking when the structural unit (C) binds to the drug (physiologically active substance). As the leaving group or linker, a $C_{1-18}$ alkyl group optionally having a substituent, a 3- to 8-membered cycloalkyl group optionally having a substituent, or a $C_{7-19}$ aralkyl group optionally having a substituent is preferable. Here, examples of the substituent include a halogen atom, an alkyl group having 1 to 6 carbon atoms, an alkenyl group having 2 to 6 carbon atoms, an alkynyl group having 2 to 6 carbon atoms, a hydroxyl group, an alkoxy group having 1 to 6 carbon atoms, an amino group, an alkylamino group having 1 to 6 carbon atoms, a di-alkylamino group having 1 to 6 carbon atoms in which alkyl groups are the same or different, a thiol group, an alkylthio group having 1 to 6 carbon atoms, a carboxyl group, an alkoxycarbonyl group having 1 to 6 carbon atoms, and a carbamoyl group. Among these groups, the linker is preferably a group having a functional group such as a hydroxyl group, an amino group, a thiol group, or a carboxyl group as a substituent.

Preferred specific examples of the leaving group of $R^6$ include a group represented by the following formula (4):

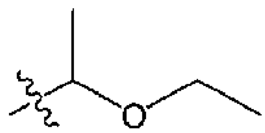

(4)

Preferred specific examples of the linker of $R^6$ include a group represented by the following formula (5):

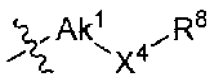

(5)

wherein, $R^8$ represents a hydrogen atom or a drug, $Ak^1$ represents a $C_{1-7}$ alkylene bond, and $X^4$ represents an oxygen

atom, a sulfur atom, or -N($R^7$)- ($R^7$ represents a hydrogen atom or a $C_{1-3}$ alkyl group). A group represented by formula (5) in which $X^4$ represents an oxygen atom, a sulfur atom, or NH is more preferable.

The copolymer X of the present invention is the copolymer having the structural units represented by formulas (A), (B) and (C). The copolymer X may be a random copolymer or a block copolymer, and is preferably a random copolymer. As for a composition ratio of each structural unit in one molecule, when (A) is 1 part by mass, preferably (B) is 0.01 to 100 parts by mass and (C) is 0.1 to 100 parts by mass; more preferably (B) is 0.05 to 18 parts by mass and (C) is 0.1 to 20 parts by mass, and particularly preferably (B) is 0.05 to 4 parts by mass and (C) is 0.1 to 16 parts by mass.

A polymerization degree of the copolymer X of the present invention is not particularly limited, and it is preferably 5 000 to 150 000, and more preferably 8 000 to 150 000 as a number average molecular weight.

In the copolymer of the present invention, as described above, the monomer represented by general formula (1) functions as a unit that imparts the hydrophilicity, and the monomer represented by general formula (2) functions as a unit that imparts the hydrophobicity. Further, the monomer represented by general formula (3) functions as a scaffold to which the drug and the copolymer are bonded. Examples of the monomer functioning as the hydrophobic unit represented by general formula (2) include monomers represented by the following formulas.

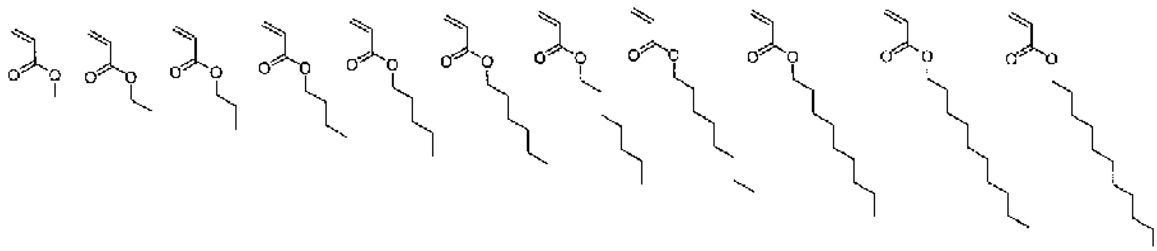

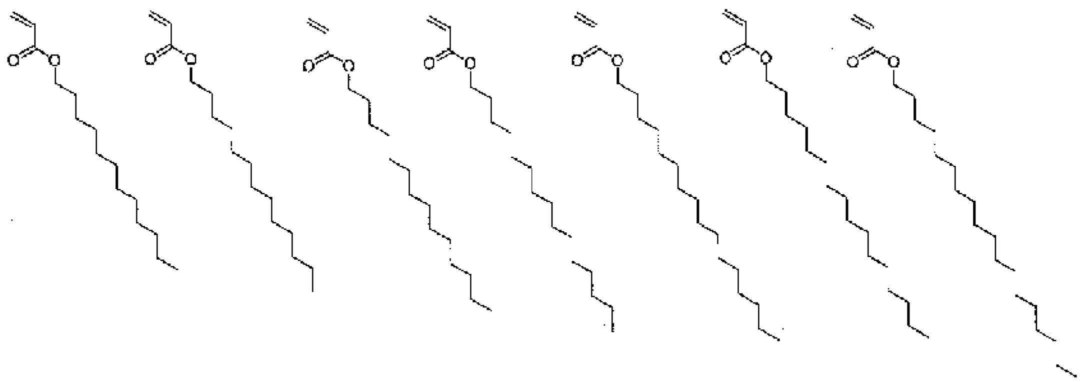

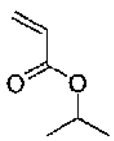 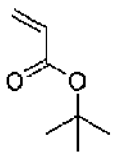 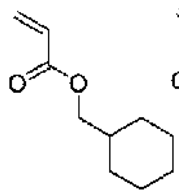 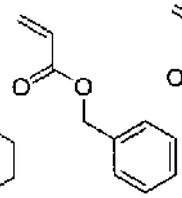 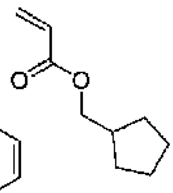 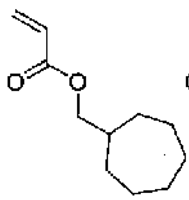 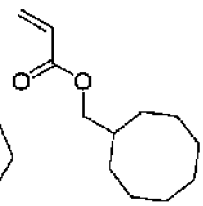

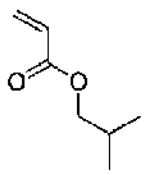 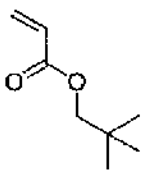 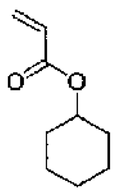 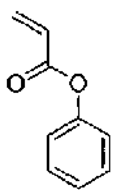 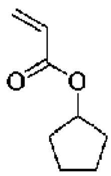 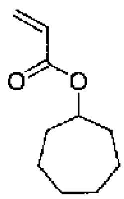 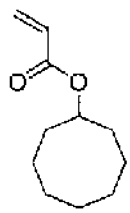

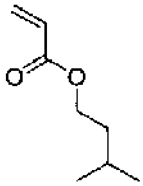 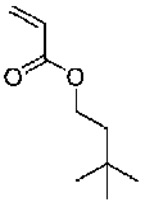 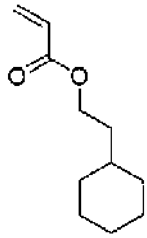 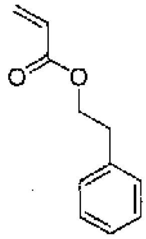 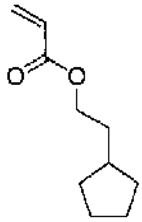 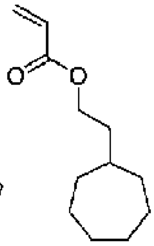 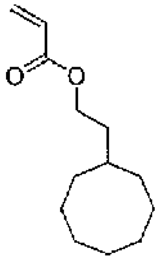

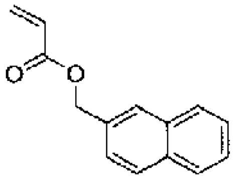 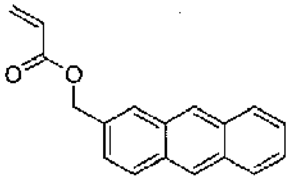 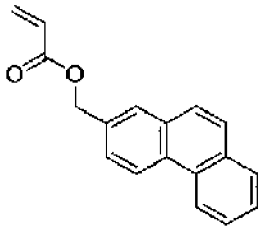 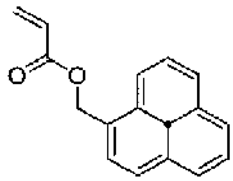

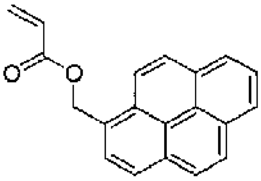 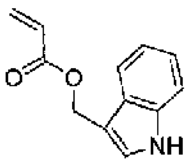 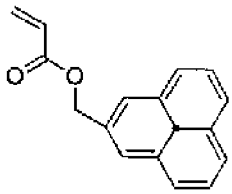 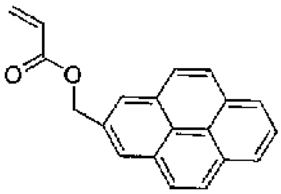

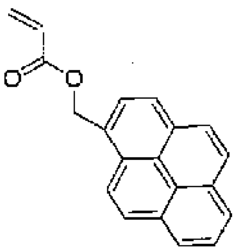 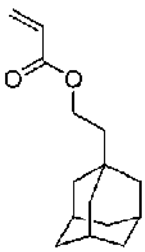

No text.

In general formula (1), $R^1$ represents a hydrogen atom or a $C_{1-3}$ alkyl group, and is preferably a hydrogen atom or a methyl group, preferably a hydrogen atom, an ethyl group, or a propyl group, and more preferably a hydrogen atom.

In general formula (2), $R^2$ represents a hydrogen atom or a $C_{1-3}$ alkyl group, and is preferably a hydrogen atom or a methyl group, preferably a hydrogen atom, an ethyl group, or a propyl group, and more preferably a hydrogen atom.

In general formula (3), $R^3$ represents a hydrogen atom or a $C_{1-3}$ alkyl group, and is preferably a hydrogen atom or a methyl group, preferably a hydrogen atom, an ethyl group, or a propyl group, and more preferably a hydrogen atom.

In general formula (1), $R^4$ represents a $C_{1-3}$ alkyl group, specifically a methyl group, an ethyl group, an n-propyl group or an isopropyl group, preferably a methyl group or an ethyl group, and more preferably a methyl group.

In general formula (1), $X^1$ represents an oxygen atom, a sulfur atom, or N-$R^7$, and is preferably an oxygen atom, a sulfur atom, or NH, and more preferably an oxygen atom.

In general formula (1), m represents an integer of 1 to 100, and is preferably an integer of 3 to 100, and preferably 3 to 80, more preferably 4 to 60, still more preferably 4 to 40, and still more preferably 4 to 22 from the viewpoint of imparting good hydrophilicity.

In general formula (2), $R^5$ represents a hydrogen atom, a $C_{1-18}$ alkyl group, a 3- to 8-membered cycloalkyl group optionally having a substituent, an adamantyl group, a $C_{6-18}$ aryl group optionally having a substituent, or a 5-to 10-membered heteroaryl group optionally having a substituent, and from the viewpoint of imparting hydrophobicity to the structural unit (B), a $C_{1-18}$ alkyl group, a 3- to 8-membered cycloalkyl group optionally having a substituent, an adamantyl group, a $C_{6-18}$ aryl group optionally having a substituent, or a 5- to 10-membered heteroaryl group optionally having a substituent is preferable, a $C_{1-18}$ alkyl group, a 3- to 8-membered cycloalkyl group optionally having a substituent, an adamantyl group, a $C_{6-18}$ aryl group optionally having a substituent, or a 5- to 10-membered heteroaryl group optionally having a substituent is more preferable, and a $C_{1-18}$ alkyl group, a 3- to 8-membered cycloalkyl group, an adamantyl group,

or a $C_{6-18}$ aryl group is still more preferable. In addition, a 3- to 8-membered cycloalkyl group optionally having a substituent, an adamantyl group, a $C_{6-14}$ aryl group optionally having a substituent, or a 6-to 10-membered heteroaryl group optionally having a substituent is also preferable. Here, the substituent is preferably one or two or more selected from a halogen atom, an alkyl group having 1 to 6 carbon atoms, an alkenyl group having 2 to 6 carbon atoms, and an alkynyl group having 2 to 6 carbon atoms.

In general formula (2), $X^2$ represents an oxygen atom, a sulfur atom, or N-$R^7$, and is preferably an oxygen atom, a sulfur atom, or NH, and more preferably an oxygen atom.

In general formula (2), n represents an integer of 0 to 3, preferably an integer of 1 to 3, and more preferably 1.

In general formula (3), $R^6$ represents a hydrogen atom, a leaving group, or a linker. As the leaving group or linker, a $C_{1-18}$ alkyl group optionally having a substituent, a 3- to 8-membered cycloalkyl group optionally having a substituent, or a $C_{7-19}$ aralkyl group optionally having a substituent is preferable. Here, examples of the substituent include a halogen atom, an alkyl group having 1 to 6 carbon atoms, an alkenyl group having 2 to 6 carbon atoms, an alkynyl group having 2 to 6 carbon atoms, a hydroxyl group, an alkoxy group having 1 to 6 carbon atoms, an amino group, an alkylamino group having 1 to 6 carbon atoms, a di-alkylamino group having 1 to 6 carbon atoms in which alkyl groups are the same or different, a thiol group, an alkylthio group having 1 to 6 carbon atoms, a carboxyl group, an alkoxycarbonyl group having 1 to 6 carbon atoms, and a carbamoyl group. Among these groups, as the linker, a group having a functional group such as a hydroxyl group, an amino group, a thiol group, or a carboxyl group as a substituent is preferable.

Preferred specific examples of the leaving group of $R^6$ include a group represented by the following formula (4):

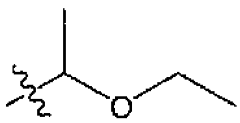

(4)

Preferred specific examples of the linker of $R^6$ include a group represented by the following formula (5):

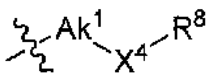

(5)

wherein, $R^8$ represents a hydrogen atom or a drug, $Ak^1$ represents a $C_{1-7}$ alkylene bond, and $X^4$ represents an oxygen atom, a sulfur atom, or -N($R^7$)- ($R^7$ represents a hydrogen atom or a $C_{1-3}$ alkyl group). A group represented by formula (5) in which $X^4$ represents an oxygen atom, a sulfur atom, or NH is preferable.

In general formula (3), $X^3$ represents an oxygen atom, a sulfur atom, or N-$R^7$, and is preferably an oxygen atom, a sulfur atom, or NH, and more preferably an oxygen atom or NH.

The copolymer X of the present invention is formed by copolymerizing three monomers represented by general formulas (1) to (3). The copolymerization may be random copolymerization or block copolymerization, and those formed by random copolymerization are preferable. As for a blending ratio of the three monomers, it is preferable that 0.01 to 100 parts by mass of monomer (2) and 0.1 to 100 parts by mass of monomer (3) are polymerized, it is more preferable that 0.05 to 18 parts by mass of monomer (2) and 0.1 to 20 parts by mass of monomer (3) are polymerized, and it is particularly preferable that 0.05 to 4 parts by mass of monomer (2) and 0.1 to 16 parts by mass of monomer (3) are polymerized, with respect to 1 part by mass of monomer (1).

In addition, "solvates" in which various solvents are coordinated are also included in the copolymer X of the present invention. In the present description, examples of the "solvate" include a hydrate and an ethanolate. The solvent may be coordinated to the copolymer X of the present invention in any number.

The copolymer X of the present invention can be produced by various known methods. The production method is not particularly limited, and for example, the copolymer X can be produced according to a basic polymer synthesis method described below.

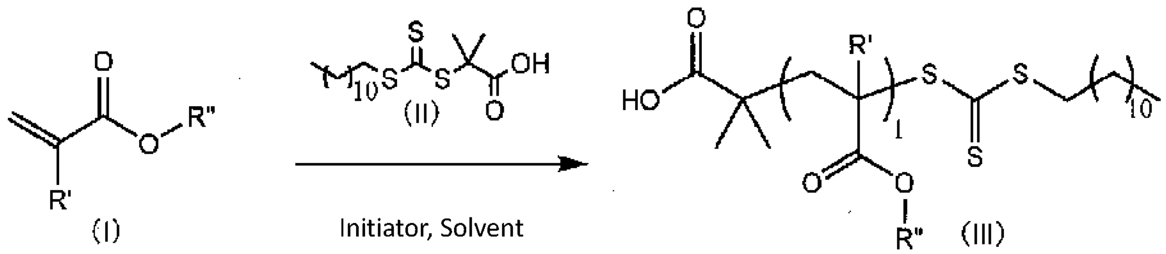


wherein R' represents a hydrogen atom or a $C_{1-3}$ alkyl group, and R" represents the group represented by $R^4$, $R^5$, or $R^6$.

This reaction shows a step of producing a polymer (III) by reacting a monomer (I) with a chain transfer agent (II) and an initiator. This reaction can be performed in the absence of a solvent or in a solvent such as alcohols such as methanol, ethanol, 1-propanol, and 2-propanol; ethers such as diethyl ether, tetrahydrofuran, and 1,4-dioxane; aromatic hydrocarbons such as benzene, toluene, and xylene; halogenated hydrocarbons such as dichloromethane, chloroform, and 1,2-dichloroethane; N,N-dimethylformamide; N,N-dimethylacetamide; N-methylpyrrolidone; acetonitrile; and ethyl acetate, and it is preferable to use aromatic hydrocarbons such as toluene and xylene as a solvent.

As the chain transfer agent, it is possible to use 2-(dodecylthiocarbonothioylthio)-2-methylpropionic acid (DDMAT), cyanomethyl dodecyltrithiocarbonate (CDTTC), 2-cyano-2-propyldodecyl trithiocarbonate (CPDTTC), 4-cyano-4-[(dodecylsulfanyl-thiocarbonyl)sulfanyl]pentanoic acid (CDSPA), 2-(dodecylthiocarbonothioylthio)-2-methylpropionic acid 3-azido-1-propanol ester ($N_3$-CTA), $N_3$-$PEG_m$Ester-CTA (m is as defined above), for example, 2-(dodecylthiocarbonothioylthio)-2-methylpropionic acid 2-(2-(2-azidoethoxy)ethoxy)ethyl ester ($N_3$-PEG2Ester-CTA), 2-(dodecylthiocarbonothioylthio)-2-methylpropionic acid 17-azido-3,6,9,12,15-pentaoxaheptadecan-1-ol ester ($N_3$-PEG5Ester-CTA), 2-(dodecylthiocarbonothioylthio)-2-methylpropionic acid 23-azido-3,6,9,12,15,18,21-heptaoxatricosan-1-ol ester ($N_3$-PEG7Ester-CTA), $N_3$-$PEG_m$Amide-CTA (m is as described above), for example, N-(8-azido-3,6-dioxaoctan-1-yl)-2-(dodecylthiocarbonothioylthio)-2-methylpropanamide ($N_3$-PEG2Amide-CTA), N-(17-azido-3,6,9,12,15-pentaoxaheptadecan-1-yl)-2-(dodecylthiocarbonothioylthio)-2-methylpropanamide ($N_3$-PEG5Amide-CTA), or N-(23-azido-3,6,9,12,15,18,21-heptaoxatricosan-1-yl)-2-(dodecylthiocarbonothioylthio)-2-methylpropanamide ($N_3$-PEG7Amide-CTA), and it is preferable to use DDMAT or $N_3$-CTA, and more preferably $N_3$-CTA. Where polymerization is carried out by using a chain transfer agent, the copolymer X of the present invention has a structure in which a part or all of a structure of the chain transfer agent is partially bonded. Where the copolymer X includes a structure of a chain transfer agent, the structure may be removed by an appropriate method.

As the initiator, an azo polymerization initiator such as 2,2'-azobis-isobutyronitrile (AIBN), 1,1'-azobis(cyclohexanecarbonitrile) (ACHN), 2,2'-azobis-2-methylbutyronitrile (AMBN), 2,2'-azobis-2,4-dimethylvaleronitrile (ADVN), or dimethyl 2,2'-azobis(2-methylpropionate) (MAIB) can be used, and AIBN is preferably used.

The reaction temperature is 0 to 300°C, preferably 0 to 150°C, and more preferably 1 to 100°C, and the reaction time is 1 minute to 48 hours, and preferably 5 minutes to 24 hours. In this reaction, a random copolymerized copolymer X can be produced by carrying out the reaction in the coexistence of monomers (I) having different structures.

For example, if the $N_3$-CTA having an azide group is used as the chain transfer agent, the copolymer X having an end at which the azide group is present will be obtained, which is advantageous for production of the target-recognizable copolymer by a click reaction described later.

As the target recognition molecule that binds to the copolymer X, an antibody that recognizes a marker or a receptor expressed on the cell surface, a mutant (modified) form thereof, or a functional fragment (fragmented antibody) thereof is preferable, and specifically, examples of the target recognition molecule can include antibodies or mutant (modified) bodies or functional fragments thereof that target one or more of BCMA, BLyS, CA-125, CCR4, CD3, CD19, CD20, CD22, CD25, CD30, CD33, CD38, CD40L, CD52, CD74, CD79b, CEACAM5, CEACAM6, CSAp, CTLA-4, CXCR4, EGFR, EpCAM, ErbB2(HER2), GD2, gp100, HLA-DR, IGF-1R, IL-6R, cMET, MUC1, Nectin-4, PD-1, PD-L1, PDGFR, SLAMF7, PSMA, TAG-72, TF, TNF-$\alpha$, TROP-2(EGP-1), VEGF, VEGFR1, VEGFR2, $\alpha$4 integrin, $\alpha$ fetoprotein (AFP), fibrin, CAIX, A33, B7, CA125, CCL19, CD2, CD4, CD8, CD11A, CD14, CD15, CD16, CD18, CD23, CD32b, CD37, CD40, CD44, CD45, CD54, CD55, CD59, CD64, CD66, CD70, CD80, CD95, CD138, CD147, CD154, CD276, EGFRvIII, FGF, Flt-1, FR$\alpha$, HMGB-1, IL-4R, IL-12, IL-15, IGF-1, IGF-2, MIF, TRAG-3, MCP-1, CD67, CD70L, CD79a, CD132, CD133, CDC27, CDK-4/m, CDKN2A, CXCR7, CXCL12, HIF-1$\alpha$, EGP-2, ILGF-1R, SAGE, S100, Survivin, Survivin-2B, TAC, tenascin, TRAIL-R, Tn antigen, Thomsen-Friedenreich antigen, WT-1, bcl-2, bcl-6, or Kras.

Here, examples of preferable targets can include antibodies targeting one or more selected from the group consisting of CD20, CD276, MUC1, EGFR, HER2, PD-L1, and TROP-2, or mutant (modified) forms or functional fragments thereof.

Examples of preferable individual antibodies include alemtuzumab (anti-CD52), bevacizumab (anti-VEGF), ramucirumab (anti-VEGFR2), cetuximab (anti-EGFR), gemtuzumab (anti-CD33), panitumumab (anti-EGFR), necitumumab (anti-EGFR), amivantamab (anti-EGFR/cMET), rituximab (anti-CD20), ibritumomab (anti-CD20), tositumomab (anti-CD20), ofatumumab (anti-CD20), mosunetuzumab (anti-CD20/CD3), trastuzumab (anti-ErbB2), pertuzumab (anti-HER2), margetuximab (anti-HER2), patritumab (anti-HER3), lambrolizumab (anti-PD-1), nivolumab (anti-PD-1), pembrolizumab (anti-PD-1), cemiplimab (anti-PD-1), dostarlimab (anti-PD-1), toripalimab (anti-PD-1), atezolizumab (anti-PD-L1), avelumab (anti-PD-L1), durvalumab (anti-PD-L1), Ipilimumab (anti-CTLA-4), abagovomab (anti-CA-125), adecatumumab (anti-EpCAM), belantamab (anti-BCMA), mogamulizumab (anti-CCR4), catumaxomab (anti-CD3/EpCAM), edrecolomab (anti-EpCAM), blinatumomab (anti-CD19/CD3), tafasitamab (anti-CD19), loncastuximab (anti-CD19), inotuzumab (anti-CD22), moxetumomab (anti-CD22), brentuximab (anti-CD30), polatuzumab (anti-CD79b), dinutuximab (anti-GD2), naxitamab (anti-GD2), tebentafusp (anti-gp100/CD3), enfortumab (anti-Nectin-4), olaratumab (anti-PDGFR), elotuzumab (anti-SLAMF7), tisotumab (anti-TF), sacituzumab (anti-TROP-2), tocilizumab (also known as atlizumab: anti-IL-6 receptor), obinutuzumab (also known as GA101: anti-CD20), CC49 (anti-TAG-72), AB-PG1-XG1-026 (anti-PSMA, US 8,114,965, deposited as ATCC PTA-4405 and PTA-4406), D2/B (anti-PSMA, WO 2009/130575 A), daclizumab (anti-CD25), muromonab-CD3 (anti-CD3), natalizumab (anti-$\alpha$4 integrin), infliximab (anti-TNF-$\alpha$), certolizumab pegol (anti-TNF-$\alpha$), adalimumab (anti-TNF-$\alpha$), dapirolizumab pegol (anti-CD40L), letolizumab (anti-CD40L), ruplizumab (anti-CD40L), belimumab (anti-BLyS), 59D8 (anti-fibrin), biciromab (also known as T2G1s: anti-fibrin), MH1 (anti-fibrin), felzartamab (anti-CD38), isatuximab (anti-CD38), daratumumab (anti-CD38), hR1 (anti-IGF-1R, US Publication No. 2010/226,884), clivatuzumab (anti-MUC1), gatipotuzumab (anti-MUC1), veltuzumab (also known as hA20: anti-CD20, US 7,151,164), hA19 (anti-CD19, US 7,109,304), hIMMU31 (anti-AFP, US 7,300,655), milatuzumab (also known as hLL1 (anti-CD74, US 7,312,318), epratuzumab (also known as hLL2: anti-CD22, US 7,074,403), hMu-9 (anti-CSAp, US 7,387,773), hL243 (anti-HLA-DR, US 7,612,180), labetuzumab (also known as hMN-14: anti-CEACAM5, US 6,676,924), hMN-3 and hMN-15 (anti-CEACAM6, US 7 541 440), Ab124 and Ab125 (anti-CXCR4, US 7,138,496), G250 (anti-CAIX), A33 (anti-A33), galiximab (anti-B7), OC125 (anti-CA125), abagovomab (anti-CA125), CAP-100 (anti-CCL19), TRX-3 (anti-CD2), IT-1208 (anti-CD4), zanolimumab (anti-CD4), crefmirlimab (anti-CD8), afelimomab (anti-CD11A), cytolin (anti-CD11A), efalizumab (anti-CD11A), odulimomab (anti-CD11A), atibuclimab (anti-CD14), fanolesomab (anti-CD15), GTB-4550 (anti-CD16), erenumab (anti-CD18), odulimomab (anti-CD18), rovelizumab (anti-CD18), lumiliximab (anti-CD23), obexelimab (anti-CD32b), BI-1206 (anti-CD32b), HuMax-CD32b (anti-CD32b), NVS-32b (anti-CD32b), NNV-003 (anti-CD37), lilotomab (anti-CD37), K7153A (anti-CD37), iscalimab (anti-CD40), ChiLob7/4 (anti-CD40), CDX-1140 (anti-CD40), TNX-1500 (anti-CD40L), TES-23 (anti-CD44), bivatuzumab (anti-CD44), actimab-B (anti-CD45), BI-505 (anti-CD54), enlimomab (anti-CD54), MOR-101 (anti-CD54), MOR-102 (anti-CD54), Onyvax-105 (anti-CD55), GB-262 (anti-CD55), PAT-SC1 (anti-CD55), VG-102 (anti-CD55), AR36A36.11.1 (anti-CD59), KNP-302 (anti-CD59), MDX-210 (anti-CD64), MDX-220 (anti-CD64), tinurilimab (anti-CD66c), cusatuzumab (anti-CD70), MDX-1411 (anti-CD70), cosibelimab (anti-PD-L1), galiximab (anti-CD80), novotarg (anti-CD95), DOM-1112 (anti-CD138), indatuximab (anti-CD138), gavilimomab (anti-CD147), letolizumab (anti-CD154), ABI-793 (anti-CD154), DOM-0800 (anti-CD154), ifinatamab (anti-CD276), mirzotamab (anti-CD276), vobramitamab (anti-CD276), AMG-596 (anti-EGFRvIII), bemarituzumab (anti-FGF), burosumab (anti-FGF), U3-1784 (anti-FGF), aprutumab (anti-FGF), icrucumab (anti-Flt-1/VEGFR), farletuzumab (anti-FR$\alpha$), mirvetuximab (anti-FR$\alpha$), girentuximab (anti-CAIX), MEDI-541 (anti-HMGB-1), dupilumab (anti-IL-4R), levilimab (anti-IL-6R), SANT-7 (anti-IL-6R), vobarilizumab (anti-IL-6R), sarilumab (anti-IL-6R), clazakizumab (anti-IL-6R), TZLS-501 (anti-IL-6R), ustekinumab (anti-IL-12), briakinumab (anti-IL-12), ordesekimab (anti-IL-15), cixutumumab (anti-IGF-1), figitumumab (anti-IGF-1), teprotumumab (anti-IGF-1), dalotuzumab (anti-IGF-1), ganitumab (anti-IGF-1), robatumumab (anti-IGF-1), AVE1642 (anti-IGF-1), dusigitumab (anti-IGF-1/2), istiratumab (anti-IGF-1), xentuzumab (anti-IGF-1), imalumab (anti-MIF), relatlimab (anti-TRAG-3), carlumab (anti-MCP-1), alacizumab pegol (anti-VEGFR-2), brolucizumab (anti-VEGFR), gentuximab (anti-VEGFR), and olinvacimab (anti-VEGFR-2).

The linker which binds to the copolymer X may be any linker as long as it links (interacts with) the copolymer X with the drug or the target recognition molecule, and may be an amino acid residue, a bifunctional derivative, a bond using a bioorthogonal reaction, an alkylene bond, a polyethylene glycol (PEG) bond, a disulfide bond, or a thioether bond.

Examples of the linker having a cleavage site (e.g., a cathepsin B cleavage site, a cathepsin C cleavage site, or a cathepsin D cleavage site) by a protease include peptides in which amino acid residues are, for example, alanine, phenylalanine, glycine, valine, lysine, citrulline, serine, a glutamic acid, and an aspartic acid. For example, the linkers include dipeptide, tripeptide, tetrapeptide, or pentapeptide, and the amino acid residue may be a natural or non-natural amino acid residue. Examples thereof include valine-citrulline (ve or val-cit), valine-alanine (va or val-ala), valine-lysine (val-lys), phenylalanine-alanine (phe-ala), phenylalanine-lysine (fk or phe-lys), phenylalanine-citrulline (phe-cit), phenylalanine-phenylalanine-lysine (phe-phe-lys), alanine-phenylalanine (af or ala-phe), alanine-lysine (ala-lys), glycine-glycine (gly-gly), glycine-alanine-phenylalanine (gly-ala-phe), glycine-valine-citrulline (gly-val-cit), glycine-glycine-glycine (gly-gly-gly), glycine-phenylalanine-lysine (gly-phe-lys), glycine-phenylalanine-leucine-glycine (gly-phe-leu-gly), glycine-glycine-phenylalanine-glycine (gly-gly-phe-gly), leucine-citrulline (leu-cit), isoleucine-citrulline (ile-cit), tryptophan-citrulline (trp-cit), or alanine-leucine-alanine-leucine (ala-leu-ala-leu) .

Examples of the linker that forms a carbamate group/carbonate group with the drug or the antibody via peptide-p-aminobenzyl alcohol ("peptide-PAB") include valine-citrulline-p-aminobenzyl carbamate, maleimidocaproyl-p-aminobenzyl carbamate, maleimidocaproyl-phenylalanine-lysine-p-aminobenzyl carbamate, or maleimidocaproyl-valine-citrulline-p-aminobenzyl carbamate.

Examples of the bifunctional derivative include N-[β-maleimidopropyloxy]succinimide ester (BMPS), [N-ε-maleimidocaproyloxy]succinimide ester (EMCS), N-[γ-maleimidobutyryloxy]succinimide ester (GMBS), m-maleimidobenzoyl-N-hydroxysuccinimide ester (MBS), [N-ε-maleimidocaproyloxy]sulfosuccinimide ester (sulfo-EMCS), N-[γ-maleimidobutyryloxy]sulfosuccinimide ester (sulfo-GMBS), m-maleimidobenzoyl-N-hydroxysulfosuccinimide ester (sulfo-MBS), N-succinimidyl-3-(2-pyridyldithio)propionate (SPDP), succinimidyl-4-(N-maleimidomethyl)cyclohexane-1-carboxylate (SMCC), N-succinimidyl-4-(2-pyridylthio)pentanoate (SPP), iminothiolane (IT), imidoesters (e.g., dimethyladipimidate HCl), active esters (e.g., disuccinimidyl sulfate), aldehydes (e.g., glutaraldehyde), bis-azide compounds (e.g., bis(p-azidobenzoyl)hexanediamine), bis-diazonium derivatives (for example, bis-(p-diazoniumbenzoyl)-ethylenediamine), diisocyanates (e.g., toluene 2,6-diisocyanate), his-active fluorine compounds (e.g., 1,5-difluoro-2,4-dinitrobenzene), DBCO-NHS Ester, DBCO-C6-NHS Ester, DBCO-Sulfo-NHS Ester, DBCO-PEG4-NHS Ester, DBCO-PEG5-NHS Ester, Sulfo DBCO-TFP Ester, Sulfo DBCO-PEG4-TFP Ester, DBCO-PEG5 -TFP Ester, DBCO-STP Ester, DBCO Acid, DBCO-C6-Acid, DBCO-PEG5-Acid, DBCO Amine, DBCO-PEG4-Amine, Sulfo DBCO-Amine, DBCO Maleimide, Sulfo DBCO-Maleimide, DBCO-PEG4- Maleimide, BCN-PEG3-Val-Cit, DBCO-PEG4-Val-Cit-PAB-PNP, or TCO-PEG4-Val-Cit-PAB-PNP.

Examples thereof include a triazole and oxime/hydrazone bond formed by the bioorthogonal reaction, for example, a Huisgen reaction between the azide and the alkyne.

Moreover, for example, an alkylene bond, a polyethylene glycol (PEG) bond, a disulfide bond, or a thioether bond can be singly used or in combination.

A preferable combination is represented by the following formula (a):

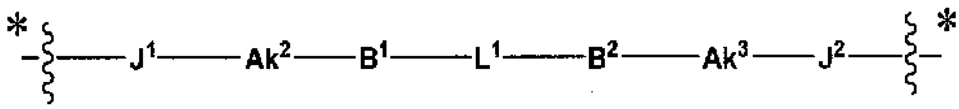

(a)

wherein, $J^1$ is a bond to the target recognition molecule or the drug, $J^2$ is a bond to the copolymer X, $Ak^2$ and $Ak^3$ each independently represent a single bond or a $C_{1-7}$ alkylene bond, $B^1$ and $B^2$ each independently represent a single bond, an amide bond, or an ester bond, $L^1$ represents a single bond, $-(CH_2CH_2O)_oCH_2CH_2-$, phenylene, cyclohexylene, -NH-peptide-CO-, or phenylene-NH-peptide-CO-, and o represents an integer of 0 to 100.

In general formula (a), $J^1$ is a bond to the target recognition molecule or the drug, and examples thereof can include -CO-, -S-, -CO-O-, $-CO-Ak^4-O-$, or the following formula (a'):

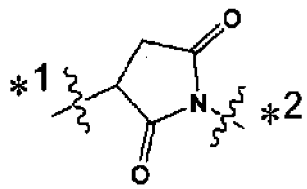

(a')

wherein, $*^1$ represents a bond to the target recognition molecule or the drug, and $*^2$ represents a bond to $Ak^2$.

In general formula (a), $Ak^2$, $Ak^3$, and $Ak^4$ each independently represent a single bond or a $C_{1-7}$ alkylene bond.

In general formula (a), $B^1$ and $B^2$ each independently represent a single bond, an amide bond, or an ester bond.

In general formula (a), $L^1$ represents a single bond, $-(CH_2CH_2O)_oCH_2CH_2-$, phenylene, cyclohexylene, -NH-peptide-CO-, or phenylene-NH-peptide-CO-, and o represents an integer of 0 to 100. The peptide contains 2 to 5 amino acid residues.

In general formula (a), $J^2$ is the bond to the copolymer X, and represents a single bond, a triazole-containing bond formed by, for example, the Huisgen reaction between an azide and a functional group containing an alkyne, or the following formula (a"):

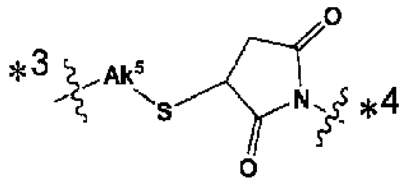

(a")

wherein, *3 represents a bond to the copolymer X and *4 represents a bond to $Ak^3$.

As a more preferable combination, the bond of the antibody to the copolymer X includes "the triazole-containing bond formed, for example, by the Huisgen reaction between the azide and the alkyne-containing functional group", and examples thereof can include the following formula (b):

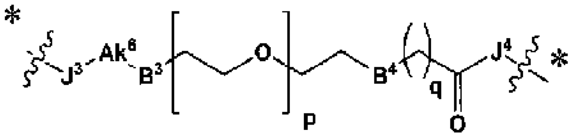

(b)

wherein, $J^3$ is a bond to the target recognition molecule, $Ak^6$ represents a single bond or a $C_{1-7}$ alkylene bond, $B^3$ represents a single bond, an amide bond or an ester bond, $J^4$ represents a triazole-containing bond formed by, for example, the Huisgen reaction between an azide and an alkyne-containing functional group, and p and q each independently represent an integer of 0 to 100.

In general formula (b), $J^3$ is a bond to the target recognition molecule, and represents -CO-, or the following formula (b'):

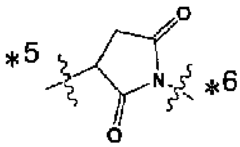

(b')

wherein, *5 represents a bond to the target recognition molecule and *6 represents a bond to $Ak^6$.

In general formula (b), $Ak^6$ represents a single bond or a $C_{1-7}$ alkylene bond.

In general formula (b), $B^3$ and $B^4$ each independently represent a single bond, an amide bond, or an ester bond.

In general formula (b), p and q each independently represent an integer of 0 to 100, preferably an integer of 1 to 50, and more preferably an integer of 2 to 25.

In general formula (b), $J^4$ is the bond to the copolymer X, and represents the triazole-containing bond formed by, for example, the Huisgen reaction between the azide and the functional group containing alkyne, and examples thereof can include the following formula (b"):

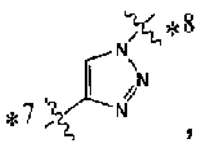
,
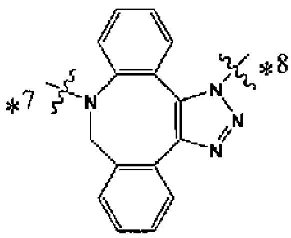
,
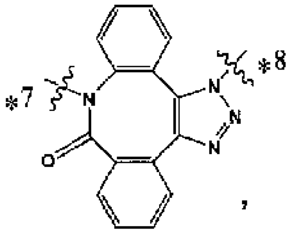
,
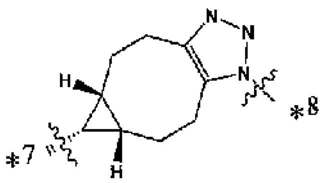

(b")

wherein, *7 represents a bond to -CO- of general formula (b) and *8 represents a bond to the copolymer X.

Moreover, examples of more preferable bonds of the target recognition molecule to the copolymer X can include a linker represented by the following formulas (6) to (13):

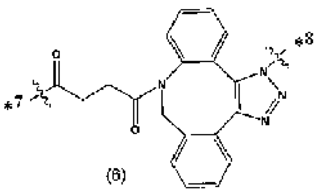
(6),

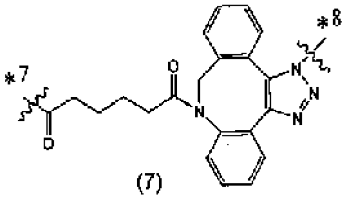
(7),

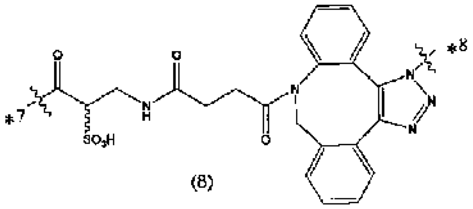
(8),

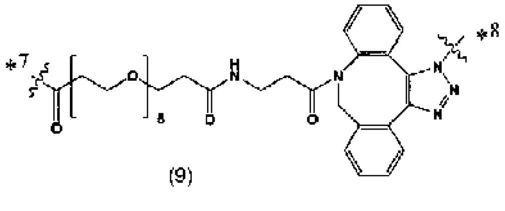
(9),

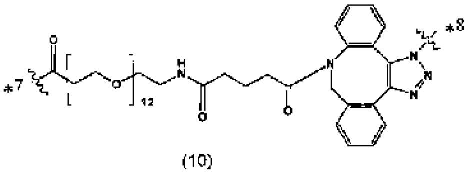
(10),

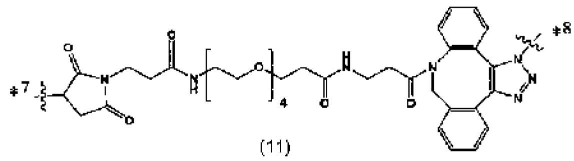
(11)

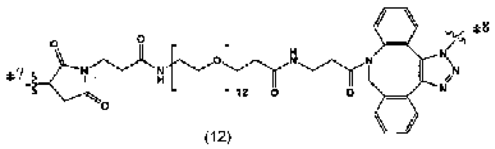
(12), or

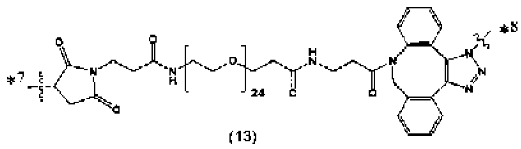
(13)

wherein, *7 represents a bond to the target recognition molecule and *8 represents a bond to the copolymer X.

Further, examples of a preferable combination of the bond of the drug to the copolymer X can include the following formula (c):

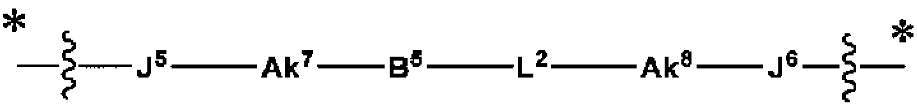

(c)

wherein, $J^5$ is a bond to the copolymer X, $J^6$ is a bond to the drug, $Ak^7$ and $Ak^8$ each independently represent a single bond or a $C_{1-7}$ alkylene bond, $B^5$ represents a single bond, an amide bond or an ester bond, and $L^2$ represents a single bond, phenylene, cyclohexylene, -CO-peptide-NH-, or -CO-peptide-NH-phenylene.

In general formula (c), $J^5$ is the bond to the copolymer X, and represents a single bond or the following formula (c'):

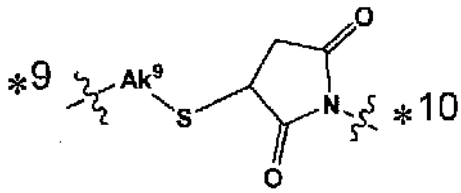

(c')

wherein, *9 represents a bond to the copolymer X and *10 represents a bond to $Ak^7$.

In general formula (c), $Ak^7$, $Ak^8$, $Ak^9$ and $Ak^{10}$ each independently represent a single bond or a $C_{1-7}$ alkylene bond.

In general formula (c), $B^5$ represents a single bond, an amide bond, or an ester bond.

In general formula (c), $L^2$ represents a single bond, phenylene, cyclohexylene, -CO-peptide-NH-, or -CO-peptide-NH-phenylene. The peptide contains 2 to 5 amino acid residues.

In general formula (c), $J^6$ is a bond to the drug, represents -CO-, -S-, -O-CO-, -*-$Ak^{10}$-CO-, or the following formula (c"), and preferably includes a drug-derived sulfur atom and a "disulfide bond",

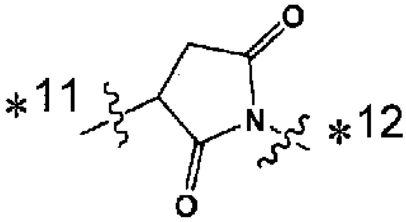

(c")

wherein, *11 represents a bond to the drug and *12 represents a bond to $Ak^8$.

Moreover, examples of a more preferable bond between the drug and the copolymer X include a linker represented by the following formulas (14) to (22):

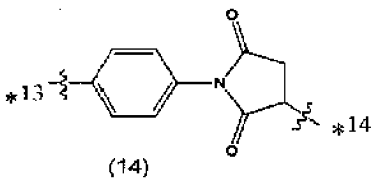

(14) ,

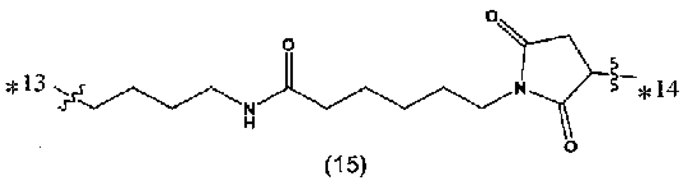

(15) ,

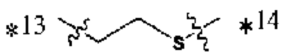

(16) ,

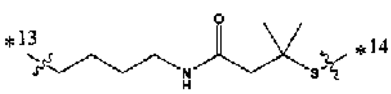

(17) ,

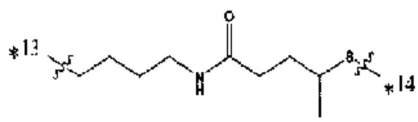

(18) ,

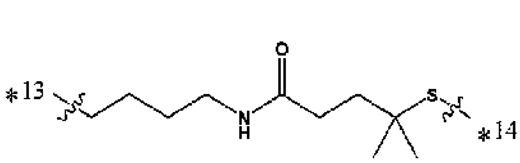

(19) ,

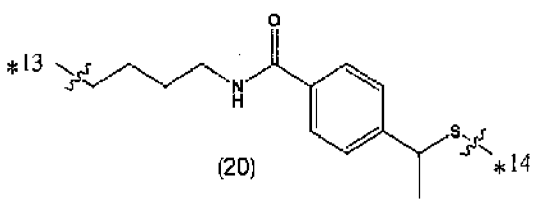

(20) ,

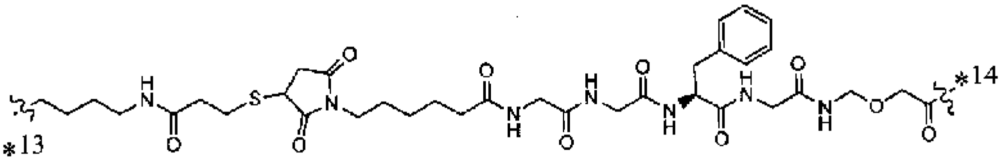

(21)

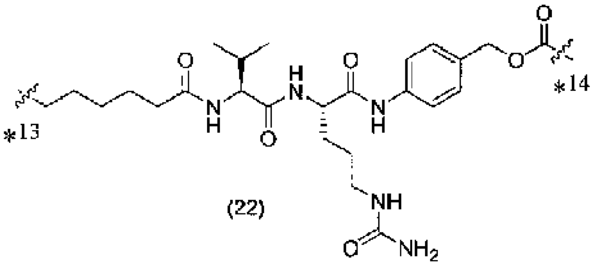

(22)

wherein, *13 represents a bond to the copolymer X and *14 represents a bond to the drug.

Where geometric isomers or optical isomers are present in the compounds of the present invention, mixtures or separations of those isomers are also included in the scope of the present invention. Separation of the isomers can be performed by a conventional method.

The target-recognizable copolymer of the present invention can be produced by various known methods. The production method is not particularly limited, and for example, the target-recognizable copolymer can be produced according to a synthesis method through the click reaction described below.

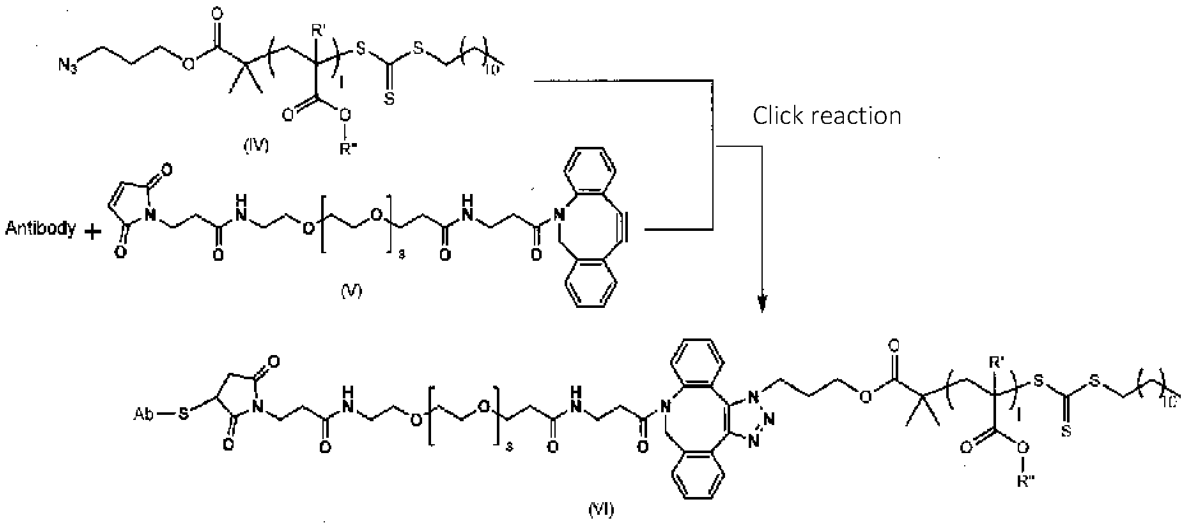


(IV) (V) (VI)

wherein R' represents a hydrogen atom or a $C_{1-3}$ alkyl group, R" represents a group represented by $R^4$, $R^5$ or $R^6$, and Ab represents the target recognition molecule.

An SH group of the target recognition molecule is obtained by reducing a disulfide bond between cysteine residues connecting chains. Examples of a reducing agent include tricarboxyethylphosphine (TCEP), 2-mercaptoethanol, 2-mercaptoethylamine, cysteine hydrochloride, dithiothreitol, and salts thereof (e.g., hydrochloride). In this method, a solution containing the target recognition molecule and a solution containing the reducing agent may be mixed. In addition to generating a partially reduced antibody, it is possible to make a reaction with a linker having a functional group that reacts with the SH group of the target recognition molecule to generate a linker-bonded (modified) target recognition molecule. A concentration of the target recognition molecule in this reaction is, for example, 1 mg/mL to 100 mg/mL. A concentration of the reducing agent is, for example, 1 mM to 100 mM, and can be mixed in an excessive amount with respect to the target recognition molecule. The reducing agent can be used in a range of 1 to 50 molar equivalents, for example, 2 to 30 molar equivalents, 5 to 20 molar equivalents, 7 to 13 molar equivalents, or 10 molar equivalents with respect to the target recognition molecule. Further, the reaction can be performed by heating to an extent that the protein is not denatured, for example, in a range of 1 to 37°C, and the reaction time can be adjusted according to an amount of the reducing agent. For

example, the reaction time is about several seconds to 5 minutes, about several seconds to 2 minutes, preferably about 1 minute to 5 minutes, and more preferably about 1 minute to 2 minutes.

For complexing the target recognition molecule with SCNP by the covalent bond, a functional group for a complexation reaction is introduced onto a surface of SCNP, and the complexation can be performed through a reaction with the SH group, the amino group, or the carboxyl group at ends or side chains of the target recognition molecules that can react with the functional group. It is also possible to introduce a spacer into the ends or the side chains of the target recognition molecule with an appropriate crosslinking reagent (crosslinker) to form a covalent bond to SCNP as a modified target recognition molecule. Examples of such bond formation include click chemistry between an azide group and an alkyne, a reaction between a sulfhydryl group and a maleimide group, and a reaction between an amino group and a succinimidyl group.

The "click chemistry" is classified to reactions similar to natural biochemical reactions, having the following attributes, and has the following characteristics. The click chemistry is a very efficient reaction that proceeds rapidly to obtain high yields, and is a very selective reaction that produces no (or little) by-products and allows polyfunctional groups. Moreover, the click chemistry proceeds under mild reaction conditions such as a low temperature (or ambient temperature) or in an aqueous solution. This reaction can be performed in water or in a solvent such as an alcohol such as methanol, ethanol, 1-propanol, or 2-propanol; an ether such as diethyl ether, tetrahydrofuran, or 1,4-dioxane; an aromatic hydrocarbon such as benzene, toluene, or xylene; a halogenated hydrocarbon such as dichloromethane, chloroform, or 1,2-dichloroethane; N,N-dimethylformamide; N,N-dimethylacetamide; N-methylpyrrolidone; acetonitrile; or ethyl acetate. It is preferable to use water or N,N-dimethylformamide as the solvent. In some aspects, the cyclooctyne is dibenzocyclooctyne (DBCO), difluorobenzocyclooctyne (DIFBO), biarylazacyclooctynone (BARAC), dibenzocyclooctyne (DIBO), difluorinated cyclooctyne (DIFO), monofluorinated cyclooctyne (MOFO), dimethoxyazacyclooctyne (DIMAC), or aryl-less octyne (ALO), with dibenzocyclooctyne (DBCO) being preferably used. In some aspects, the alkyne is aliphatic alkyne and the reacting step is performed in the presence of a copper (I) catalyst. In some aspects, the alkyne is cyclooctyne and the reacting step is performed under copper-free conditions. The reaction temperature is 0 to 300°C, preferably 0 to 150°C, and more preferably 1 to 100°C, and the reaction time is 1 minute to 48 hours, and preferably 5 minutes to 24 hours.

Reaction of the reaction formula is Huisgen cycloaddition which is a kind of click reaction, and is a 1,3-dipolar cycloaddition reaction in which 1,2,3-triazole is formed from the azide and the alkyne.

The produced polymer X, target-recognizable copolymer, and target-recognizable micelle drug complex of the present invention can be purified by a polymer isolation and purification method generally known in the field of polymer chemistry. Specific examples thereof include treatment operations such as extraction, recrystallization, salting out using, for example, ammonium sulfate or sodium sulfate, centrifugation, dialysis, ultrafiltration, adsorption chromatography, ion exchange chromatography, hydrophobic chromatography, normal phase chromatography, reverse phase chromatography, desalting column chromatography, gel filtration, gel permeation chromatography, affinity chromatography, electrophoresis, countercurrent distribution, and combinations thereof. In particular, in the hydrophobic chromatography, since retention time varies in accordance with the number of SCNP bonded to one target recognition molecule, it is possible to collect a fraction satisfying any DAR by fractionation.

The copolymer X and the target-recognizable copolymer of the present invention can be used as a carrier for transporting of various physiologically active substances (drugs). For example, a pharmaceutical composition in which a therapeutic agent for tumor is carried on (encapsulated in) the copolymer X, the target-recognizable copolymer, or the target-recognizable micelle drug complex of the present invention can be used as a prophylactic and/or therapeutic agent for various cancer diseases such as a colon cancer, a duodenal cancer, a gastric cancer, a pancreatic cancer, a liver cancer, a lung cancer, a uterine cancer, and an ovarian cancer, because the pharmaceutical composition suppresses growth of tumors, as confirmed in test examples described later. Further, with a high tumor accumulation ability, the pharmaceutical composition can be used as a diagnostic agent or a contrast agent for tumor.

When the copolymer and the target-recognizable copolymer of the present invention are used as a drug transport carrier, the dose and the number of doses may be appropriately selected in consideration of, for example, administration form, age and body weight of a patient, and nature or severity of a symptom to be treated, and the dose and the number of doses should not be limited. However, when a polymer encapsulating a drug or the target-recognizable micelle drug complex is intravenously injected by an injection, for example, for an adult (60 kg), a single dose is preferably administered in an amount of 0.12 mg to 12 000 000 mg, more preferably 1.2 mg to 1 200 000 mg, and particularly preferably 12 to 120 000 mg.

The pharmaceutical composition of the present invention can be produced by mixing the drug with the copolymer X, the target-recognizable copolymer, or the target-recognizable micelle drug complex of the present invention. Preferably, the copolymer, the target-recognizable copolymer, or the target-recognizable micelle drug complex of the present invention may be mixed with the drug to produce the single chain nanoparticle, or the single chain nanoparticle of the copolymer X, the target-recognizable copolymer, or the target-recognizable micelle drug complex of the present invention may be produced and then mixed with the drug. The single chain nanoparticle can be produced by a known method.

In the pharmaceutical composition of the present invention, the drug may be carried on the copolymer X, the

target-recognizable copolymer, or the target-recognizable micelle drug complex by the action such as electrostatic interaction, hydrogen bond, hydrophobic interaction, or covalent bond.

The drug is preferably an anticancer agent, more preferably an anticancer agent that acts on cancer cells to suppress proliferation of the cancer cells, and examples thereof include an antimetabolite, an alkylating agent, an anthracycline, an antibiotic, a mitotic inhibitor, a topoisomerase inhibitor, a proteasome inhibitor, and an anti-hormone agent.

Examples of the antimetabolite include azathioprine, 6-mercaptopurine, 6-thioguanine, fludarabine, pentostatin, cladribine, 5-fluorouracil (5FU), floxuridine (FUDR), cytosine arabinoside (cytarabine), methotrexate, trimethoprim, pyrimethamine, and pemetrexed. Examples of the alkylating agent include cyclophosphamide, mechlorethamine, uramustine, melphalan, chlorambucil, thiotepa/chlorambucil, ifosfamide, carmustine, lomustine, streptozocin, busulfan, dibromomannitol, cisplatin, carboplatin, nedaplatin, oxaliplatin, miriplatin, satraplatin, triplatin tetranitrate, procarbazine, altretamine, dacarbazine, mitozolomide, trabectedin, and temozolomide. Examples of the anthracycline include daunorubicin, doxorubicin, epirubicin, idarubicin, valrubicin, aclarubicin, amrubicin, and pirarubicin. Examples of the antibiotic include dactinomycin, bleomycin, mithramycin, anthramycin, streptozotocin, gramicidin D, staurosporine, mitomycins (e.g., mitomycin C), duocarmycins (e.g., CC-1065), and calicheamicins. Examples of the mitotic inhibitor include maytansinoids (e.g., DM0, mertansine (also known as DM1), DM2, DM3, DM4, or emtansine), auristatins (e.g., auristatin E, auristatin phenylalanine phenylenediamine (AFP), monomethyl auristatin E, monomethyl auristatin D, or monomethyl auristatin F), dolastatins, cryptophycins, vinca alkaloid (e.g., vincristine, vinblastine, vindesine, or vinorelbine), taxanes (e.g., paclitaxel or docetaxel), and colchicines. Examples of the topoisomerase inhibitor include irinotecan, topotecan, nogitecan, amsacrine, etoposide, teniposide, mizantrone, mitoxantrone, SN-38, exatecan, and deruxtecan.

Examples of the proteasome inhibitor can include a peptidyl boronic acid, carfilzomib, and bortezomib. Examples of the anti-hormone agent can include fulvestrant, tamoxifen, and toremifene. Where these drugs are prepared into the pharmaceutical composition of the present invention, one or more of the drugs can also be used in combination, and the drugs may be carried on the copolymer as a free form.

A route of administration of the pharmaceutical composition of the present invention is desirably the most effective one for treatment, and the pharmaceutical composition can be administered by a parenteral administration preparation such as an oral administration preparation, an injection, or a transdermal administration preparation. For example, parenteral administration such as intraarterial injection, intravenous injection, subcutaneous injection, intramuscular injection, or intraperitoneal injection is preferable, and intraarterial injection and intravenous injection are more preferable. The number of doses should not be limited, and examples thereof include one to several doses per week average.

Various preparations suitable for the route of administration can be produced by a conventional method by appropriately selecting preparation additives such as excipients, extenders, binders, wetting agents, disintegrants, lubricants, surfactants, dispersants, buffers, preservatives, solubilizing agents, antiseptics, flavoring agents, soothing agents, stabilizers, and isotonizing agents that are conventionally used in formulation.

The preparation additives that can be contained in the various preparations described above are not particularly limited as long as the preparation additives are pharmaceutically acceptable. Examples of such preparation additives include purified water, water for injection, distilled water for injection, pharmaceutically acceptable organic solvents, collagen, polyvinyl alcohol, polyvinylpyrrolidone, carboxyvinyl polymer, sodium alginate, water-soluble dextran, sodium carboxymethyl starch, pectin, xanthan gum, gum arabic, casein, gelatin, agar, glycerin, propylene glycol, polyethylene glycol, petrolatum, paraffin, stearyl alcohol, stearic acid, human serum albumin, mannitol, sorbitol, and lactose. Additives to be used are appropriately selected according to various preparations, and can be used alone or in combination.

The injection can also be prepared as a non-aqueous diluent (for example, polyethylene glycol, vegetable oils such as olive oil, and alcohols such as ethanol), a suspension, or an emulsion. Sterilization of the injection can be performed by filtration sterilization using a filter, and blending of, for example, a microbicide. In addition, the injection can be produced in a form of preparation before use. That is, the injection can be formed into a sterile solid composition by, for example, lyophilization, and can be dissolved in water for injection, distilled water for injection, or another solvent before use.

## Examples

Hereinafter, the present invention will be described more specifically with reference to examples. These examples are provided for purpose of exemplification and are not intended to limit embodiments of the invention.

### [Example 1] Production of poly[(benzyl acrylate)-co-(poly(ethylene glycol)methyl ether acrylate)-co-(1-ethoxyethyl acrylate)]

(1) Synthesis of 1-ethoxyethyl acrylate (EEA)

Ethyl vinyl ether (28.725 mL) was weighed under an argon atmosphere, and thereto was added a phosphoric acid (50 mg) under ice cooling. Thereto was added acrylic acid (17.15 mL), and the mixture was stirred at room temperature for 48 hours. Further thereto was added hydrotalcite (3 g), and the mixture was stirred for 2 hours, and the reaction was stopped. After celite filtration, unreacted ethyl vinyl ether was removed by evaporation. Thereto was added phenothiazine (up to 500 ppm) as a polymerization inhibitor, and the mixture was purified by distillation under a reduced pressure together with calcium hydride (distillation temperature of 28 to 32°C). The obtained 1-ethoxyethyl acrylate was dispensed into a glass vial and stored at -30°C.

$^{13}C$ NMR (400MHz, $CDCl_3$), δ, ppm: 15.29 ($-OCH_2CH_3$), 21.16 ($-COOCH(CH_3)$), 64.98 ($-OCH_2-$), 96.73 ($-COOCH(CH_3)$), 128.84 ($CH_2CH-$), 131.43 ($CH_2CH-$), 166.00 (-COO).

(2) Synthesis of poly[(benzyl acrylate)-co-(poly(ethylene glycol) methyl ether acrylate)-co-(1-ethoxyethyl acrylate)]

100 mg of 2-(dodecylthiocarbonothioylthio)-2-methylpropionic acid (DDMAT) was weighed and dissolved in 17.3 mL of toluene to prepare a DDMAT/toluene stock solution (5.78 mg/mL as a DDMAT concentration). Similarly, 22 mg of 2,2'-azobis(2-methylpropionitrile) (AIBN) was weighed and dissolved in 17.3 mL of toluene to prepare an AIBN/toluene stock solution (AIBN concentration: 1.27 mg/mL). Separately, 1.296 g of poly(ethylene glycol) methyl ether acrylate (mPEGA, the average value (n) of the numbers of repetitions of ethylene glycol is 9), 0.394 g of benzyl acrylate (BnA), 0.039 g of 1-ethoxyethyl acrylate, 1.73 mL of a DDMAT/toluene stock solution, and 1.73 mL of an AIBN/toluene stock solution were added, and polymerization was performed in an oil bath at 70°C. After a lapse of 90 minutes, the polymerization was stopped, and then the reaction solution was subjected to a reprecipitation method or dialyzed against methanol to recover the copolymer. Since the obtained copolymer was basically a viscous body, in the reprecipitation method, an operation of dropping the reaction solution into a centrifuge tube to which a poor solvent (hexane/ethyl acetate = 7/3 [v/v]) was added and recovering the solution by centrifugation (2 000 × g, 5 min) was repeated 3 times, and finally vacuum drying was performed to obtain 1.223 g of poly[(benzyl acrylate)-co-(poly(ethylene glycol) methyl ether acrylate)-co-(1-ethoxyethyl acrylate)].

As a result of analyzing the polymerization degree of each monomer and the number average molecular weight ($M_{n,NMR}$) from a $^1H$-NMR spectrum of the obtained copolymer measured using NMR, the polymerization degree of mPEGA (n = 9) was 102, the polymerization degree of BnA was 94, the polymerization degree of EEA was 9, and $M_{n,NMR}$ was 65 900. The molecular weight dispersion ($M_w/M_n$) of the obtained copolymer was measured using GPC, and as a result, it was found to be 1.53.

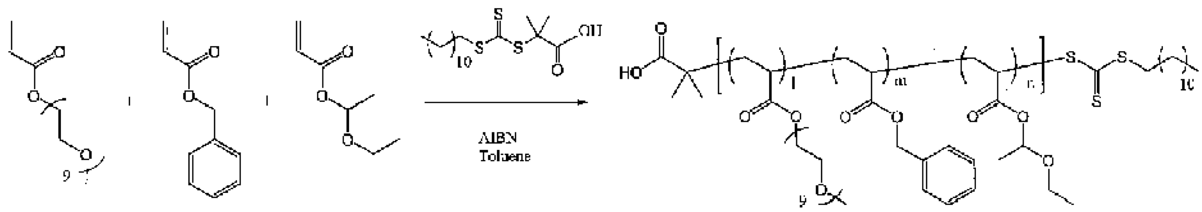


[Measurement apparatus and conditions]

(1) $^1H$-NMR measurement

Apparatus: JNM-ECX 400 (400 MHz)/JEOL Ltd.
Solvent: Dimethyl sulfoxide-$d_6$ containing 0.03% tetramethylsilane/KANTO CHEMICAL CO., INC.
Sample concentration: 20 mg/mL
Measurement temperature: 25°C
Number of integration times: 256 times
Result: Fig. 1

(2) GPC measurement

Apparatus: HPLC-Prominence system/SHIMADZU CORPORATION
Detector: RID-10A Refractive index detector/SHIMADZU CORPORATION

Column: TSKgel α-2500 column/Tosoh Corporation
(Column size: 7.8 mm × 300 mm, particle size: 7 μm, exclusion limit molecular weight: 5 × $10^3$)
TSKgel α-4000 column/Tosoh Corporation
(Column size: 7.8 mm × 300 mm, particle size: 10 μm, exclusion limit molecular weight: 4 × $10^5$)
TSKgel guardcolum/Tosoh Corporation
Mobile phase: N,N-dimethyformamide (DMF) containing 10 mmol/L lithium bromide
Temperature: 40°C
Flow rate: 0.5 mL/min
Sample concentration: 6 mg/mL
Standard substance: Poly(methyl methacrylate) standard ReadyCal set, $M_p$ 800 - 2 200 000 Da/SIGMA
Result: Fig. 2

[Table 1]

| Example | Composition ratio (molar ratio to chain transfer agent) | | | | | Solvent | Temperature (°C) | Polymerization time (min) | Polymerization degree | | | $M_{n,NMR}$ | $M_w/M_n$ |
|---|---|---|---|---|---|---|---|---|---|---|---|---|---|
| | Chain transfer agent | Initiator | Monomer | | | | | | | | | | |
| | DDMAT | AIBN | mPEGA n=9 | BnA | EEA | | | | mPEGA n=9 | BnA | EEA | | |
| 1 | 1 | 0.5 | 100 | 90 | 10 | Toluene | 70 | 90 | 102 | 94 | 9 | 65 900 | 1.53 |

[Examples 2 to 68]

Polymers having different composition ratios and average molecular weights shown in the following table were produced by appropriately changing the type, charged amount, reaction temperature, and polymerization time of the monomers (mPEGA, BnA, and EEA) used in Example 1, and using the same method as in Example 1.

[Table 2]

| Example | Composition ratio (molar ratio to chain transfer agent) | | | | | | | | | | | Solvent | Temperature (°C) | Polymerization time (min) |
|---|---|---|---|---|---|---|---|---|---|---|---|---|---|---|
| | Chain transfer agent | Initiator | | Monomer | | | | | | | | | | |
| | | | | mPEGA | | | | | | | | | | |
| | DDMAT | AIBN | ACHN | n=4 | n=9 | n=22 | BnA | EEA | 2-Hydroxy ethyl acrylate | 4-Hydroxy butyl acrylate | 2-Hydroxy-3-phenoxy propyl acrylate | | | |
| 2 | 1 | 2 | - | 255 | - | - | 15 | 30 | - | - | - | Toluene | 70 | 90 |
| 3 | 1 | 2 | - | 160 | - | - | 20 | 20 | - | - | - | Toluene | 70 | 60 |
| 4 | 1 | 2 | - | 240 | - | - | 30 | 30 | - | - | - | Toluene | 70 | 60 |
| 5 | 1 | 2 | - | 170 | - | - | 10 | 20 | - | - | - | Toluene | 70 | 60 |

| | | | | | | | | | | | | | | |
|---|---|---|---|---|---|---|---|---|---|---|---|---|---|---|
| 6 | 1 | 2 | - | 255 | - | - | 15 | 30 | - | - | - | Toluene | 70 | 60 |
| 7 | 1 | 0.5 | - | - | 16 | - | 16 | 8 | - | - | - | Toluene | 70 | 90 |
| 8 | 1 | 0.5 | - | - | 18 | - | 14 | 8 | - | - | - | Toluene | 70 | 90 |
| 9 | 1 | 0.5 | - | - | 20 | - | 12 | 8 | - | - | - | Toluene | 70 | 90 |
| 10 | 1 | 0.5 | - | - | 20 | - | 12 | 8 | - | - | - | Toluene | 70 | 90 |
| 11 | 1 | 0.5 | - | - | 20 | - | 12 | 8 | - | - | - | Toluene | 70 | 90 |
| 12 | 1 | 0.5 | - | - | 20 | - | 16 | 4 | - | - | - | Toluene | 70 | 10 |
| 13 | 1 | 0.5 | - | - | 20 | - | 16 | 4 | - | - | - | Toluene | 70 | 30 |
| 14 | 1 | 0.5 | - | - | 20 | - | 16 | 4 | - | - | - | Toluene | 70 | 50 |
| 15 | 1 | 0.5 | - | - | 20 | - | 16 | 4 | - | - | - | Toluene | 70 | 70 |
| 16 | 1 | 0.5 | - | - | 20 | - | 16 | 4 | - | - | - | Toluene | 70 | 90 |
| 17 | 1 | 0.5 | - | - | 22 | - | 10 | 8 | - | - | - | Toluene | 70 | 90 |
| 18 | 1 | 0.5 | - | - | 24 | - | 8 | 8 | - | - | - | Toluene | 70 | 90 |
| 19 | 1 | 0.5 | - | - | 24 | - | 8 | 8 | - | - | - | Toluene | 70 | 90 |
| 20 | 1 | 0.5 | - | - | 28 | - | 4 | 8 | - | - | - | Toluene | 70 | 90 |
| 21 | 1 | 0.5 | - | - | 10 | - | 25 | 15 | - | - | - | Toluene | 70 | 90 |
| 22 | 1 | 0.5 | - | - | 17 | - | 25 | 8 | - | - | - | Toluene | 70 | 90 |
| 23 | 1 | 0.5 | - | - | 22.5 | - | 12.5 | 15 | - | - | - | Toluene | 70 | 90 |
| 24 | 1 | 0.5 | - | - | 25 | - | 20 | 5 | - | - | - | Toluene | 70 | 90 |
| 25 | 1 | 0.5 | - | - | 29.5 | - | 12.5 | 8 | - | - | - | Toluene | 70 | 90 |
| 26 | 1 | 0.5 | - | - | 30 | - | 24 | 6 | - | - | - | Toluene | 70 | 90 |
| 27 | 1 | 0.5 | - | - | 35 | - | 28 | 7 | - | - | - | Toluene | 70 | 90 |
| 28 | 1 | 0.5 | - | - | 50 | - | 40 | 10 | - | - | - | Toluene | 70 | 90 |
| 29 | 1 | 0.5 | - | - | 10 | - | 10 | 180 | - | - | - | Toluene | 70 | 90 |
| 30 | 1 | 0.5 | - | - | 30 | - | 30 | 140 | - | - | - | Toluene | 70 | 90 |
| 31 | 1 | 0.5 | - | - | 30 | - | 70 | 100 | - | - | - | Toluene | 70 | 90 |
| 32 | 1 | 0.5 | - | - | 30 | - | 110 | 60 | - | - | - | Toluene | 70 | 90 |
| 33 | 1 | 0.5 | - | - | 50 | - | 10 | 140 | - | - | - | Toluene | 70 | 90 |
| 34 | 1 | 0.5 | - | - | 50 | - | 50 | 100 | - | - | - | Toluene | 70 | 90 |
| 35 | 1 | 0.5 | - | - | 50 | - | 90 | 60 | - | - | - | Toluene | 70 | 90 |
| 36 | 1 | 0.5 | - | - | 50 | - | 130 | 20 | - | - | - | Toluene | 70 | 90 |
| 37 | 1 | 0.5 | - | - | 70 | - | 30 | 100 | - | - | - | Toluene | 70 | 90 |
| 38 | 1 | 0.5 | - | - | 70 | - | 70 | 60 | - | - | - | Toluene | 70 | 90 |
| 39 | 1 | 0.5 | - | - | 70 | - | 110 | 20 | - | - | - | Toluene | 70 | 90 |
| 40 | 1 | 0.5 | - | - | 80 | - | 80 | 40 | - | - | - | Toluene | 70 | 90 |
| 41 | 1 | 0.5 | - | - | 80 | - | 100 | 20 | - | - | - | Toluene | 70 | 90 |
| 42 | 1 | 0.5 | - | - | 90 | - | 10 | 100 | - | - | - | Toluene | 70 | 90 |
| 43 | 1 | 0.5 | - | - | 90 | - | 50 | 60 | - | - | - | Toluene | 70 | 90 |
| 44 | 1 | 0.5 | - | - | 90 | - | 90 | 20 | - | - | - | Toluene | 70 | 90 |
| 45 | 1 | 0.5 | - | - | 100 | - | 20 | 80 | - | - | - | Toluene | 70 | 90 |
| 46 | 1 | 0.5 | - | - | 100 | - | 40 | 60 | - | - | - | Toluene | 70 | 90 |
| 47 | 1 | 0.5 | - | - | 100 | - | 50 | 50 | - | - | - | Toluene | 70 | 90 |
| 48 | 1 | 0.5 | - | - | 100 | - | 60 | 40 | - | - | - | Toluene | 70 | 90 |
| 49 | 1 | 0.5 | - | - | 100 | - | 70 | 30 | - | - | - | Toluene | 70 | 90 |
| 50 | 1 | 0.5 | - | - | 100 | - | 80 | 20 | - | - | - | Toluene | 70 | 90 |
| 51 | 1 | 0.5 | - | - | 110 | - | 30 | 60 | - | - | - | Toluene | 70 | 90 |

| | | | | | | | | | | | | | | |
|---|---|---|---|---|---|---|---|---|---|---|---|---|---|---|
| 52 | 1 | 0.5 | - | - | 110 | - | 70 | 20 | - | - | - | Toluene | 70 | 90 |
| 53 | 1 | 0.5 | - | - | 130 | - | 10 | 60 | - | - | - | Toluene | 70 | 90 |
| 54 | 1 | 0.5 | - | - | 130 | - | 50 | 20 | - | - | - | Toluene | 70 | 90 |
| 55 | 1 | 0.5 | - | - | 150 | - | 30 | 20 | - | - | - | Toluene | 70 | 90 |
| 56 | 1 | 0.5 | - | - | 170 | - | 10 | 20 | - | - | - | Toluene | 70 | 90 |
| 57 | 1 | 0.5 | - | - | 200 | - | 160 | 40 | - | - | - | Toluene | 70 | 90 |
| 58 | 1 | 0.5 | - | - | 300 | - | 240 | 60 | - | - | - | Toluene | 70 | 90 |
| 59 | 1 | 0.5 | - | - | 400 | - | 320 | 80 | - | - | - | Toluene | 70 | 90 |
| 60 | 1 | 2 | - | - | - | 105 | 155 | 40 | - | - | - | Toluene | 70 | 90 |
| 61 | 1 | 2 | - | - | - | 120 | 240 | 40 | - | - | - | Toluene | 70 | 90 |
| 62 | 1 | 2 | - | - | - | 140 | 210 | 50 | - | - | - | Toluene | 70 | 90 |
| 63 | 1 | 2 | - | - | - | 140 | 220 | 40 | - | - | - | Toluene | 70 | 90 |
| 64 | 1 | 0.1 | - | - | 100 | - | 80 | - | 20 | - | - | 1,4-dioxane | 70 | 90 |
| 65 | 1 | - | 0.5 | - | 100 | - | 50 | - | - | 50 | - | 1,4-dioxane | 70 | 180 |
| 66 | 1 | - | 0.5 | - | 100 | - | 80 | - | - | 20 | - | 1,4-dioxane | 70 | 180 |
| 67 | 1 | 0.1 | - | - | 130 | - | 65 | - | - | - | 65 | 1,4-dioxane | 70 | 90 |
| 68 | 1 | 0.1 | - | - | 130 | - | 104 | - | - | - | 26 | 1,4-dioxane | 70 | 90 |

[Table 3]

| Example | Polymerization degree | | | | | | | | $M_{n,NMR}$ | $M_w/M_n$ |
|---|---|---|---|---|---|---|---|---|---|---|
| | mPEGA | | | BnA | EEA | 2-Hydroxy ethyl acrylate | 4-Hydroxy butyl acrylate | 2-Hydroxy-3-phenoxy propyl acrylate | | |
| | n=4 | n=9 | n=22 | | | | | | | |
| 2 | 250 | - | - | 18 | 31 | - | - | - | 62 400 | 1.37 |
| 3 | 155 | - | - | 22 | 20 | - | - | - | 39 200 | 1.23 |
| 4 | 211 | - | - | 29 | 29 | - | - | - | 53 100 | 1.32 |
| 5 | 162 | - | - | 11 | 20 | - | - | - | 39 000 | 1.22 |
| 6 | 227 | - | - | 15 | 28 | - | - | - | 54 300 | 1.33 |
| 7 | - | 16 | - | 16 | 8 | - | - | - | 11 800 | 1.20 |
| 8 | - | 18 | - | 14 | 8 | - | - | - | 12 400 | 1.20 |
| 9 | - | 13 | - | 8 | 5 | - | - | - | 8 600 | 1.46 |
| 10 | - | 20 | - | 13 | 8 | - | - | - | 13 400 | 1.30 |
| 11 | - | 18 | - | 11 | 7 | - | - | - | 11 700 | 1.33 |
| 12 | - | 8 | - | 7 | 1 | - | - | - | 5 300 | 1.51 |
| 13 | - | 16 | - | 13 | 3 | - | - | - | 10 800 | 1.30 |
| 14 | - | 19 | - | 15 | 3 | - | - | - | 12 200 | 1.28 |
| 15 | - | 20 | - | 16 | 4 | - | - | - | 13 300 | 1.39 |
| 16 | - | 24 | - | 19 | 4 | - | - | - | 15 700 | 1.29 |
| 17 | - | 21 | - | 10 | 8 | - | - | - | 13 200 | 1.20 |
| 18 | - | 13 | - | 5 | 4 | - | - | - | 8 000 | 1.44 |

| | | | | | | | | | | |
|---|---|---|---|---|---|---|---|---|---|---|
| 19 | - | 24 | - | 8 | 8 | - | - | - | 14 300 | 1.21 |
| 20 | - | 26 | - | 4 | 8 | - | - | - | 14 600 | 1.20 |
| 21 | - | 7 | - | 17 | 9 | - | - | - | 8 100 | 1.41 |
| 22 | - | 11 | - | 17 | 5 | - | - | - | 9 200 | 1.39 |
| 23 | - | 15 | - | 9 | 10 | - | - | - | 10 700 | 1.30 |
| 24 | - | 26 | - | 21 | 5 | - | - | - | 16 900 | 1.31 |
| 25 | - | 20 | - | 10 | 6 | - | - | - | 12 400 | 1.33 |
| 26 | - | 33 | - | 27 | 6 | - | - | - | 21 500 | 1.34 |
| 27 | - | 39 | - | 30 | 7 | - | - | - | 25 000 | 1.36 |
| 28 | - | 39 | - | 4 | 8 | - | - | - | 20 200 | 1.25 |
| 29 | - | 10 | - | 10 | 159 | - | - | - | 29 700 | 1.19 |
| 30 | - | 27 | - | 27 | 122 | - | - | - | 35 600 | 1.27 |
| 31 | - | 30 | - | 66 | 91 | - | - | - | 38 600 | 1.17 |
| 32 | - | 27 | - | 91 | 52 | - | - | - | 35 800 | 1.20 |
| 33 | - | 45 | - | 10 | 123 | - | - | - | 41 000 | 1.31 |
| 34 | - | 44 | - | 45 | 86 | - | - | - | 41 100 | 1.20 |
| 35 | - | 45 | - | 79 | 54 | - | - | - | 42 700 | 1.24 |
| 36 | - | 43 | - | 111 | 19 | - | - | - | 42 000 | 1.28 |
| 37 | - | 60 | - | 28 | 87 | - | - | - | 46 500 | 1.24 |
| 38 | - | 55 | - | 57 | 49 | - | - | - | 43 000 | 1.27 |
| 39 | - | 56 | - | 89 | 18 | - | - | - | 44 300 | 1.30 |
| 40 | - | 79 | - | 80 | 36 | - | - | - | 56 400 | 1.43 |
| 41 | - | 81 | - | 106 | 17 | - | - | - | 58 900 | 1.51 |
| 42 | - | 81 | - | 12 | 93 | - | - | - | 54 600 | 1.27 |
| 43 | - | 71 | - | 42 | 51 | - | - | - | 48 600 | 1.30 |
| 44 | - | 70 | - | 73 | 18 | - | - | - | 48 400 | 1.32 |
| 45 | - | 84 | - | 19 | 72 | - | - | - | 54 200 | 1.33 |
| 46 | - | 80 | - | 35 | 52 | - | - | - | 51 900 | 1.35 |
| 47 | - | 84 | - | 45 | 44 | - | - | - | 54 200 | 1.33 |
| 48 | - | 82 | - | 52 | 35 | - | - | - | 53 100 | 1.36 |
| 49 | - | 94 | - | 68 | 26 | - | - | - | 60 100 | 1.50 |
| 50 | - | 104 | - | 83 | 20 | - | - | - | 66 700 | 1.52 |
| 51 | - | 93 | - | 30 | 55 | - | - | - | 57 700 | 1.33 |
| 52 | - | 84 | - | 56 | 18 | - | - | - | 52 400 | 1.35 |
| 53 | - | 105 | - | 10 | 52 | - | - | - | 60 000 | 1.35 |
| 54 | - | 102 | - | 42 | 18 | - | - | - | 58 900 | 1.37 |
| 55 | - | 111 | - | 25 | 18 | - | - | - | 60 100 | 1.40 |
| 56 | - | 131 | - | 10 | 18 | - | - | - | 67 400 | 1.43 |
| 57 | - | 129 | - | 108 | 31 | - | - | - | 84 100 | 1.51 |
| 58 | - | 170 | - | 144 | 40 | - | - | - | 111 200 | 1.61 |
| 59 | - | 221 | - | 191 | 50 | - | - | - | 144 700 | 1.68 |
| 60 | - | - | 58 | 102 | 78 | - | - | - | 91 200 | 1.71 |

| | | | | | | | | | | |
|---|---|---|---|---|---|---|---|---|---|---|
| 61 | - | - | 62 | 143 | 21 | - | - | - | 93 100 | 1.68 |
| 62 | - | - | 59 | 105 | 93 | - | - | - | 95 000 | 1.82 |
| 63 | - | - | 60 | 106 | 18 | - | - | - | 85 400 | 1.68 |
| 64 | - | 75 | - | 66 | - | 19 | - | - | 49 400 | 1.46 |
| 65 | - | 67 | - | 37 | - | - | 39 | - | 44 300 | 1.40 |
| 66 | - | 61 | - | 56 | - | - | 20 | - | 41 600 | 1.43 |
| 67 | - | 75 | - | 42 | - | - | - | 42 | 52 300 | 1.61 |
| 68 | - | 75 | - | 68 | - | - | - | 18 | 51 400 | 1.56 |

[Example 69]

Production of poly[(benzyl acrylate)-co-(poly(ethylene glycol)methyl ether acrylate)-co-(acrylic acid)]

By treating poly[(benzyl acrylate)-co-(poly(ethylene glycol)methyl ether acrylate)-co-(1-ethoxyethyl acrylate)] obtained in Example 1 with 0.5N HCl at room temperature, an ethoxyethyl group was eliminated to obtain a terpolymer having a carboxyl group (1.176 g). A Z-average particle diameter and a polydispersity index of the obtained terpolymer in water were measured by dynamic light scattering (DLS), and as a result, the Z-average particle diameter was 8.5 nm (polydispersity index: 0.14).

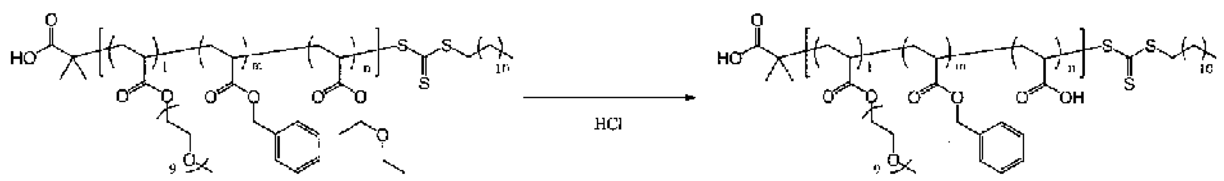


[Measurement apparatuses and conditions]

(1) DLS measurement

Apparatus: Zetasizer NanoZS/Malvern Instruments Ltd.
Measurement temperature: 25°C
Sample concentration: 10 mg/mL
Results: Fig. 3

[Example 70]

Method for producing (1,2-diaminocyclohexane)platinum(II)-encapsulating SCNP

65.28 mg of a Cl($H_2O$) form of (1,2-diaminocyclohexane)platinum(II) (hereinafter, abbreviated as DACHPt) (DACHPt.Cl.$H_2O$) was dissolved in 20 mL of purified water and stirred at 70°C for 2 hours. To 5 mL of this solution, 287.4 mg of the terpolymer obtained in Example 69 was added, and the mixture was stirred at 50°C overnight. After completion of stirring, the reaction solution was dialyzed and purified using purified water as an external solution to obtain 5 mL of a DACHPt-encapsulating SCNP. The Pt content of the DACHPt-encapsulating SCNP obtained after purification was measured by inductively coupled plasma-atomic emission spectrometry (ICP-AES) and found to be 720 μg/mL (1.14 mg/mL as DACHPt). Separately, 200 μL of the DACHPt-encapsulating SCNP was lyophilized, and the solid content concentration was calculated, then the ratio to the Pt content was taken, and the Pt binding amount per polymer was calculated. As a result, the Pt binding amount was 3.4 mol/mol. In addition, the Z-average particle diameter and the polydispersity index of the obtained DACHPt-encapsulating SCNP were measured by dynamic light scattering (DLS), and as a result, the Z-average particle diameter was found to be 8.7 nm (polydispersity index: 0.14). The particle diameter of the SCNP before and after encapsulation of DACHPt is shown in Fig. 3. The particle diameter of the SCNP hardly varied before and after encapsulation of DACHPt. The results are summarized in the following table.

[Measurement apparatuses and conditions]

(1) ICP-AES measurement

Apparatus: Sequential high-frequency plasma light-emitting apparatus ICPE-9000/SHIMADZU CORPORATION
Pretreatment apparatus: microwave sample pretreatment apparatus ETHOS EASY/Milestone General
Measurement wavelength: 214 nm
Standard solution: Platinum standard solution (Pt1000) for ICP analysis/FUJIFILM Wako Pure Chemical Corporation

2) DLS measurement

Apparatus: Zetasizer NanoZS/Malvern Instruments Ltd.
Measurement temperature: 25°C
Sample concentration: 10 mg/mL
Results: Fig. 3

[Table 4]

| Example | Yield (mL) | Pt content (μg/mL) | Pt binding amount per polymer (mol/mol) | Z-average particle diameter (nm) | Polydispersity index |
|---|---|---|---|---|---|
| 70 | 5 | 720 | 3.4 | 8.7 | 0.14 |

[Example 71]

Production of $N_3$-poly[ (benzyl acrylate)-co-(poly(ethylene glycol)methyl ether acrylate)-co-(acrylic acid)]

Synthesis was performed in a similar manner to Example 1 and Example 69 by using $N_3$-CTA in place of the chain transfer agent DDMAT used in Example 1. In toluene (17.3 mL), 2-(dodecylthiocarbonothioylthio)-2-methylpropionic acid 3-azido-1-propanol ester ($N_3$-CTA) (100 mg) was dissolved after being weighed to obtain an $N_3$-CTA/toluene stock solution ($N_3$-CTA concentration: 5.78 mg/mL). Similarly, 2,2'-azobis(2-methylpropionitrile) (AIBN) (10 mg) was weighed and dissolved in toluene (7.87 mL) to obtain an AIBN/toluene stock solution (AIBN concentration: 1.27 mg/mL). Separately, poly(ethylene glycol)methyl ether acrylate (mPEGA, average value (n) of the numbers of repetition times of ethylene glycol is 9) (2.592 g), benzyl acrylate (BnA) (0.684 g), 1-ethoxyethyl acrylate (0.172 g), the $N_3$-CTA/toluene stock solution (4.15 mL), and the AIBN/toluene stock solution (3.46 mL) were added, and polymerization was performed in an oil bath at 70°C. After a lapse of 90 minutes, the polymerization was stopped, and then a copolymer was recovered by subjecting the reaction solution to reprecipitation or dialysis against methanol. For the reprecipitation, an operation of dropping the reaction solution into a centrifuge tube to which the poor solvent (hexane/ethyl acetate = 7/3 [v/v]) was added and recovering the solution by centrifugation (2 000 × g, 5 min) was repeated three times, and finally the vacuum drying was performed. By treating obtained copolymer with 0.5N HCl at room temperature, an ethoxyethyl group was eliminated to obtain $N_3$-poly[ (benzyl acrylate)-co-(poly(ethylene glycol)methyl ether acrylate)-co-(acrylic acid)] (2.455 g) .

As a result of analyzing a polymerization degree of each of monomers and a number average molecular weight ($M_{n,NMR}$) of the obtained copolymer from a $^1$H-NMR spectrum measured by using NMR, the polymerization degree of mPEGA (n = 9) was 70, the polymerization degree of BnA was 56, the polymerization degree of EEA was 15, and $M_{n,NMR}$ was 44 000. Moreover, a molecular weight dispersion ($M_w/M_n$) of the obtained copolymer was measured by using the GPC and a result thereof was 1.32.

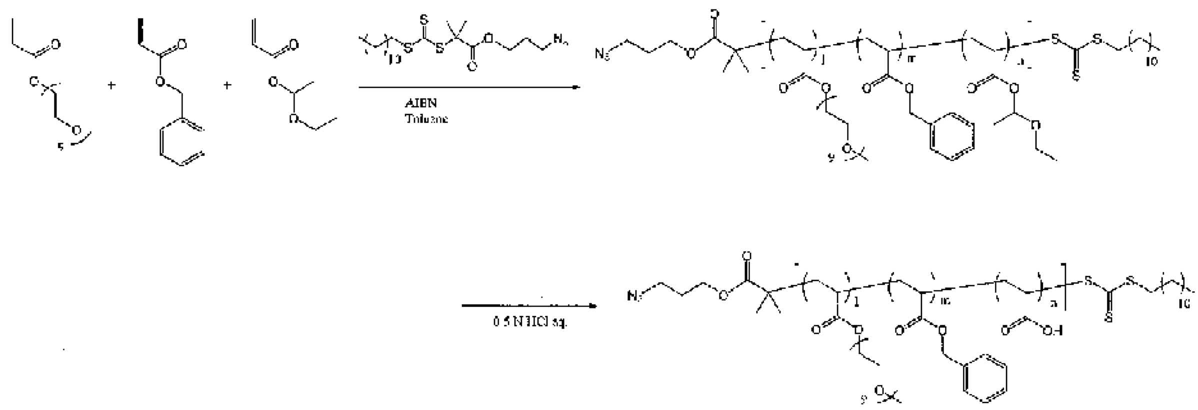


[Measurement apparatuses and conditions]

(1) $^1$H-NMR measurement

Apparatus: JNM-ECX400 (400 MHz)/JEOL Ltd.
Solvent: dimethyl sulfoxide-$d_6$ containing 0.03% tetramethylsilane/KANTO CHEMICAL CO., INC.
Sample concentration: 20 mg/mL
Measurement temperature: 25°C
Number of integration times: 256 times
Results: Fig. 4

(2) GPC measurement

Apparatus: HPLC-Prominence system/SHIMADZU CORPORATION
Detector: RID-10A Refractive index detector/SHIMADZU CORPORATION
Column: TSKgel α-2500 column/Tosoh Corporation
(Column size: 7.8 mm × 300 mm, particle size: 7 μm, and exclusion limit molecular weight: 5 × $10^3$)
TSKgel α-4000 column/Tosoh Corporation
(Column size: 7.8 mm × 300 mm, particle size: 10 μm, and exclusion limit molecular weight: 4 × $10^5$)
TSKgel guardcolum/Tosoh Corporation
Mobile phase: N,N-dimethylformamide (DMF) containing 10 mmol/L of lithium bromide
Temperature: 40°C
Flow rate: 0.5 mL/min
Sample concentration: 6 mg/mL
Standard substance: poly(methyl methacrylate) standard ReadyCal set, $M_p$ 800 - 2 200 000 Da/SIGMA
Results: Fig. 5

[Table 5]

| Example | Composition ratio (molar ratio to chain transfer agent) | | | | | Solvent | Temperature (°C) | Polymerization time (min) | Polymerization degree | | | $M_{n,NMR}$ | $M_w/M_n$ |
|---|---|---|---|---|---|---|---|---|---|---|---|---|---|
| | Chain transfer agent | Initiator | Monomer | | | | | | | | | | |
| | $N_3$-CTA | AIBN | mPEGA n=9 | BnA | EEA | | | | mPEGA n=9 | BnA | EEA | | |
| 71 | 1 | 0.5 | 100 | 80 | 20 | Toluene | 70 | 90 | 70 | 56 | 15 | 44 000 | 1.32 |

[Examples 72 to 85]

Polymers having different composition ratios and average molecular weights as shown in the following table were produced by appropriately changing charged amounts and polymerization times of the monomers (mPEGA, BnA, and EEA) used in Example 71, and using the same method as in Example 71.

[Table 6]

| Example | Composition ratio (molar ratio to chain transfer agent) | | | | | Solvent | Temperature (°C) | Polymerization time (min) |
|---|---|---|---|---|---|---|---|---|
| | Chain transfer agent | Initiator | Monomer | | | | | |
| | $N_3$-CTA | AIBN | mPEGA (n=9) | BnA | EEA | | | |
| 72 | 1 | 0.5 | 100 | 90 | 10 | Toluene | 70 | 90 |
| 73 | 1 | 0.5 | 100 | 80 | 20 | Toluene | 70 | 90 |
| 74 | 1 | 0.5 | 100 | 88 | 12 | Toluene | 70 | 90 |
| 75 | 1 | 0.5 | 100 | 88 | 12 | Toluene | 70 | 90 |
| 76 | 1 | 0.5 | 100 | 80 | 20 | Toluene | 70 | 90 |
| 77 | 1 | 0.5 | 100 | 70 | 30 | Toluene | 70 | 90 |
| 78 | 1 | 0.5 | 100 | 70 | 30 | Toluene | 70 | 90 |
| 79 | 1 | 0.5 | 100 | 70 | 30 | Toluene | 70 | 90 |
| 80 | 1 | 0.5 | 100 | 80 | 20 | Toluene | 70 | 90 |
| 81 | 1 | 0.5 | 100 | 80 | 20 | Toluene | 70 | 90 |
| 82 | 1 | 0.5 | 100 | 80 | 20 | Toluene | 70 | 90 |
| 83 | 1 | 0.5 | 100 | 78 | 22 | Toluene | 70 | 90 |
| 84 | 1 | 0.5 | 100 | 62 | 38 | Toluene | 70 | 90 |
| 85 | 1 | 0.5 | 100 | 38 | 62 | Toluene | 70 | 90 |

[Table 7]

| Example | Polymerization degree | | | $M_{n}$,NMR | *Mw*/*Mn* |
|---|---|---|---|---|---|
| | Monomer | | | | |
| | mPEGA (n=9) | BnA | EEA (Acrylic acid) | | |
| 72 | 72 | 69 | 9 | 46 900 | 1.47 |
| 73 | 79 | 65 | 18 | 49 800 | 1.38 |
| 74 | 77 | 75 | 12 | 50 500 | 1.34 |
| 75 | 73 | 68 | 12 | 47 200 | 1.34 |
| 76 | 77 | 66 | 20 | 49 600 | 1.34 |
| 77 | 74 | 55 | 26 | 46 700 | 1.34 |
| 78 | 75 | 55 | 26 | 47 100 | 1.28 |
| 79 | 85 | 61 | 26 | 52 900 | 1.33 |
| 80 | 82 | 69 | 16 | 52 300 | 1.32 |
| 81 | 82 | 67 | 16 | 51 600 | 1.32 |
| 82 | 77 | 64 | 15 | 48 600 | 1.34 |
| 83 | 65 | 52 | 16 | 41 300 | 1.31 |
| 84 | 70 | 45 | 30 | 43 300 | 1.35 |
| 85 | 75 | 31 | 50 | 45 100 | 1.29 |

[Examples 86 to 88]

Production of $N_3$($PEG_m$ ester)-poly[ (benzyl acrylate)-co-(poly(ethylene glycol)methyl ether acrylate)-co-(acrylic acid)]

In place of the chain transfer agent $N_3$-CTA used in Example 71, synthesis was performed by using 2-(dodecylthiocarbonothioylthio)-2-methylpropionic acid 2-(2-(2-azidoethoxy)ethoxy)ethyl ester ($N_3$-PEG2-Ester CTA), 2-(dodecylthiocarbonothioylthio)-2-methylpropionic acid 17-azido-3,6,9,12,15-pentaoxaheptadecan-1-ol ester ($N_3$-PEG5-Ester CTA), or 2-(dodecylthiocarbonothioylthio)-2-methylpropionic acid 23-azido-3,6,9,12,15,18,21-heptaoxatricosan-1-ol

ester ($N_3$-PEG7-Ester CTA) under similar (composition ratio) conditions as in Example 83.

[Table 8]

| Example | Composition (molar ratio to chain transfer agent) | | | | | Solvent | Temperature (°C) | Polymerization time (min) | Polymerization degree | | | $M_{n,NMR}$ | $M_w/M_n$ | Z-average particle diameter (nm) | Polydispersity index |
|---|---|---|---|---|---|---|---|---|---|---|---|---|---|---|---|
| | Chain transfer agent | Initiator | Monomer | | | | | | | | | | | | |
| | $N_3$-Ester CTA | AIBN | mPEGA n=9 | BnA | EEA | | | | mPEGA n=9 | BnA | EEA | | | | |
| 86 | 1 (PEG2) | 0.5 | 100 | 78 | 22 | Toluene | 70 | 90 | 83 | 68 | 19 | 52 500 | 1.32 | 10 | 0.27 |
| 87 | 1 (PEG5) | 0.5 | 100 | 78 | 22 | Toluene | 70 | 90 | 72 | 60 | 19 | 46 100 | 1.33 | 9 | 0.22 |
| 88 | 1 (PEG7) | 0.5 | 100 | 78 | 22 | Toluene | 70 | 90 | 75 | 63 | 20 | 48 200 | 1.33 | 10 | 0.25 |

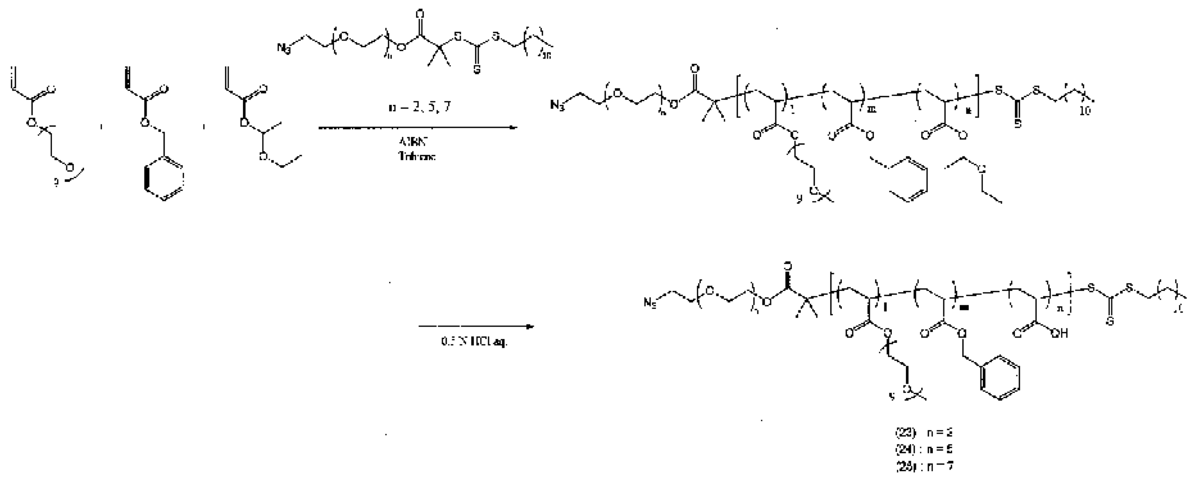


(23) n = 2
(24) : n = 5
(25) : n = 7

[Examples 89 to 91]

Production of $N_3$($PEG_m$ amide)-poly[ (benzyl acrylate)-co-(poly(ethylene glycol)methyl ether acrylate)-co-(acrylic acid) ]

In place of the chain transfer agent $N_3$-CTA used in Example 71, synthesis was performed by using N-(8-azido-3,6-dioxaoctan-1-yl)-2-(dodecylthiocarbonothioylthio)-2-methylpropanamide ($N_3$-PEG2-Amide CTA), N-(17-azido-3,6,9,12,15-pentaoxaheptadecan-1-yl)-2-(dodecylthiocarbonothioylthio)-2-methylpropanamide ($N_3$-PEG5-Amide CTA), or N-(23-azido-3,6,9,12,15,18,21-heptaoxatricosan-1-yl)-2-(dodecylthiocarbonothioylthio)-2-methylpropanamide ($N_3$-PEG7-Amide CTA) under the same conditions (composition ratio) as in Example 83.

[Table 9]

| Examp | Composition ratio (molar ratio to chain transfer agent) | | | Solvent | Tempe | Polymerizatio | Polymerization degree | $M_{n,NMR}$ | $M_w/M_n$ | Z-average | Polydis |
|---|---|---|---|---|---|---|---|---|---|---|---|
| | Chain transfer | Initiator | Monomer | | | | | | | | |

| | agent | | | | | | | | | | | | | particle diameter (nm) | |
|---|---|---|---|---|---|---|---|---|---|---|---|---|---|---|---|
| | $N_3$-Amide CTA | AIBN | mPEGA n=9 | BnA | EEA | | | | mPEGA n=9 | BnA | EEA | | | | |
| 89 | 1 (PEG2) | 0.5 | 100 | 78 | 22 | Toluene | 70 | 90 | 72 | 60 | 19 | 45 900 | 1.46 | 10 | 0.24 |
| 90 | 1(PEG5) | 0.5 | 100 | 78 | 22 | Toluene | 70 | 90 | 68 | 58 | 19 | 44 000 | 1.47 | 9 | 0.23 |
| 91 | 1(PEG7) | 0.5 | 100 | 78 | 22 | Toluene | 70 | 90 | 70 | 59 | 20 | 45 200 | 1.49 | 9 | 0.23 |

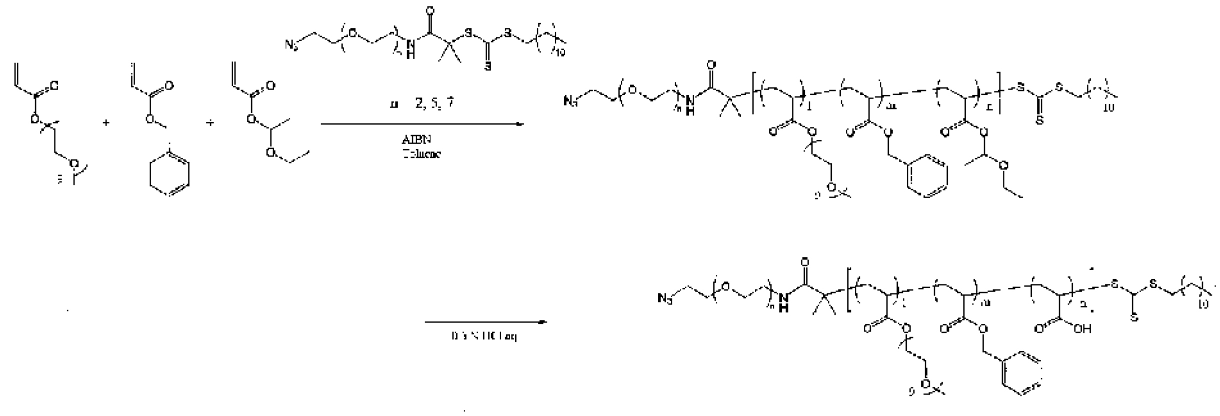


(26) : n = 2
(27) : n = 5
(28) : n = 7

[Example 92]

Production of $N_3$-poly[(benzyl acrylate)-co-(poly(ethylene glycol)methyl ether acrylate)-co-(acrylic acid)]-isobutyronitrile

In toluene (32 mL), the copolymer obtained in Example 71 (2.40 g) was dissolved after being weighed. To this solution, the AIBN (170 mg) and lauroyl peroxide (62 mg) were added, and the mixture was stirred in an oil bath at 80°C for 20 hours. After the reaction was stopped by ice cooling, the copolymer was recovered by subjecting the reaction solution to reprecipitation or dialysis against methanol. Since the obtained copolymer was basically a viscous form, in the reprecipitation, an operation of dropping the reaction solution into a centrifuge tube to which the poor solvent (hexane/ethyl acetate = 7/3 [v/v]) was added and recovering the solution by centrifugation (2 000 × g, 5 min) was repeated 3 times, and finally the vacuum drying was performed to obtain $N_3$-poly[ (benzyl acrylate)-co-(poly(ethylene glycol) methyl ether acrylate)-co-(acrylic acid)]-isobutyronitrile (2.21 g) having a converted end structure. A residual ratio of the end structure of the obtained copolymer was evaluated from a UV spectrum measured with an ultraviolet-visible spectrophotometer, and the result thereof was 0.0%.

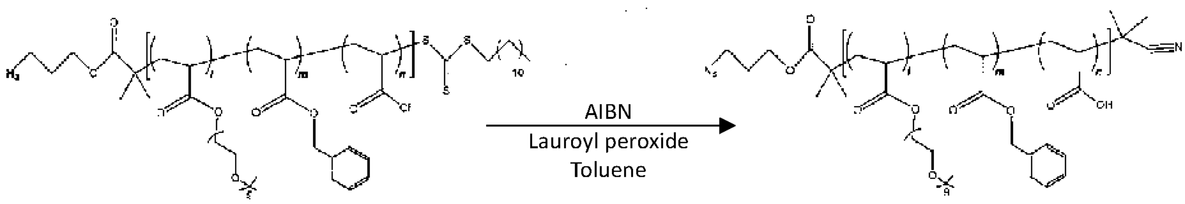


(29)

[Measurement apparatuses and conditions]

(1) UV spectrum measurement

Apparatus: Hitachi spectrophotometer U-9300/Hitachi, Ltd.
Solvent: purified water
Sample concentration: 4 mg/mL
Measurement wavelength: 250 to 500 nm
Results: Fig. 6

[Table 10]

| Example | Copolymer used | Azo compound used in reaction | Residual ratio of end structure (%) |
|---|---|---|---|
| 92 | Example 71 | AIBN | 0.0 |

[Examples 93 to 95]

Copolymers having different end structures as shown in the following table were synthesized by appropriately modifying a type and a charge amount of the azo compound for the copolymers obtained in Examples 84 and 85 and using the same method as in Example 92.

[Table 11]

| Example | Copolymer used | Azo compound used in reaction | Residual ratio of end structure (%) |
|---|---|---|---|
| 93 | Example 84 | AIBN | 0.0 |
| 94 | Example 84 | MAIB | 0.0 |
| 95 | Example 85 | AIBN | 0.0 |

[Example 96]

Production of DM1-cysteamine-bonded copolymer

To a solution of the copolymer obtained in Example 92 (200 mg) in DMF (2 mL), were added (1-cyano-2-ethoxy-2-oxoethylideneaminooxy)dimethylaminomorpholinocarbenium hexafluorophosphate (COMU) (59 mg) and 2,2,6,6-tetramethylpiperidine (TMP) (23.2 μL), and the mixture was stirred at 30°C for 2 hours. Separately, to a solution of mertansine (DM1) (101 mg) and S-(2-pyridylthio)cysteamine hydrochloride (18 mg) in THF (3 mL) was added DIPEA (35.9 μL) and the mixture was stirred at 30°C for 2 hours. The respective reaction liquids were mixed, and the obtained reaction solution was stirred at 30°C for 24 hours. The reaction solution was dialyzed and purified (dialysis membrane: Spectra/Por Regenerated Cellulose Membrane 6, molecular cut off: 3.5 kDa, and external liquid: methanol), then the solvent was removed by distillation under reduced pressure and the vacuum drying, and the copolymer was recovered to obtain the DM1-cysteamine-bonded copolymer (222 mg). Further, a Z-average particle diameter and a polydispersity index of the obtained copolymer in water were measured by dynamic light scattering (DLS), and a result of the Z-average particle diameter was 11 nm (polydispersity index: 0.32).

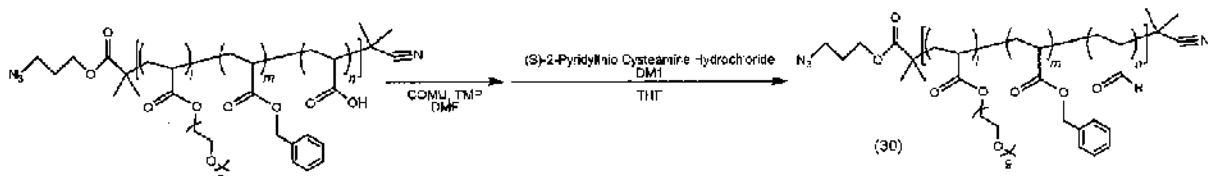


(30)

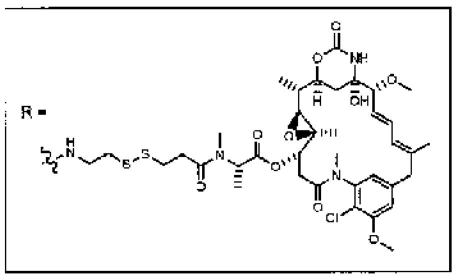

For the DM1-cysteamine-bonded copolymer, the number of DM1 introduced to one molecule of the copolymer was analyzed from a $^1$H-NMR spectrum measured by using NMR, and a result thereof was 13 mol/mol.

[Measurement apparatuses and conditions]

(1) $^1$H-NMR measurement

Apparatus: JNM-ECX400 (400 MHz)/JEOL Ltd.
Solvent: dimethyl sulfoxide-$d_6$ containing 0.03% tetramethylsilane/KANTO CHEMICAL CO., INC.
Sample concentration: 20 mg/mL
Measurement temperature: 25°C
Number of integration times: 256 times
Results: Fig. 7

[Table 12]

| Example | Copolymer used | Number of DM1 introduced to one molecule of copolymer (mol/mol) |
|---|---|---|
| 96 | Example 92 | 13 |

[Examples 97 to 107]

For the copolymers obtained in Examples 79, 85 to 91, and 93 to 95, copolymers having different numbers of DM1 introduced to one molecule of each of the copolymer as shown in the following table were synthesized by appropriately modifying charge amounts of the linker and DM1 and using the same method as in Example 96.

[Table 13]

| Example | Copolymer used | Number of DM1 introduced to one molecule of copolymer (mol/mol) |
|---|---|---|
| 97 | Example 93 | 20 |
| 98 | Example 94 | 26 |
| 99 | Example 95 | 20 |
| 100 | Example 85 | 26 |
| 101 | Example 79 | 19 |
| 102 | Example 86 | 13 |
| 103 | Example 87 | 13 |
| 104 | Example 88 | 12 |
| 105 | Example 89 | 13 |
| 106 | Example 90 | 12 |
| 107 | Example 91 | 15 |

[Example 108]

Production of DM1-N-4-APM-bonded copolymer

To a solution of the copolymer obtained in Example 72 (509 mg) in DMF (10 mL), were added COMU (84 mg) and TMP (33 μL), and the mixture was stirred at room temperature for 3 hours. Thereto was added N-(4-aminophenyl) maleimide (N-4-APM) (55 mg), and the mixture was stirred at 30°C for 3 days. The reaction solution was dialyzed and purified (dialysis membrane: Spectra/Por Regenerated Cellulose Membrane 6, molecular cut off: 3.5 kDa, and external liquid: methanol), and then the solvent was removed by the distillation under reduced pressure and the vacuum drying to

obtain an N-4-APM-bonded copolymer (475 mg). In DMF (10 mL), the obtained N-4-APM-bonded copolymer (475 mg) was dissolved, DM1 (102 mg) was added, and the mixture was stirred at room temperature for 24 hours. The reaction solution was dialyzed and purified (dialysis membrane: Spectra/Por Regenerated Cellulose Membrane 6, molecular cut off: 3.5 kDa, and external liquid: methanol), and then the solvent was removed by the distillation under reduced pressure and the vacuum drying to obtain the DM1-N-4-APM-bonded copolymer (458 mg). A Z-average particle diameter and a polydispersity index of the obtained copolymer in water were measured by dynamic light scattering (DLS), and a result of the Z-average particle diameter was 11 nm (polydispersity index: 0.19).

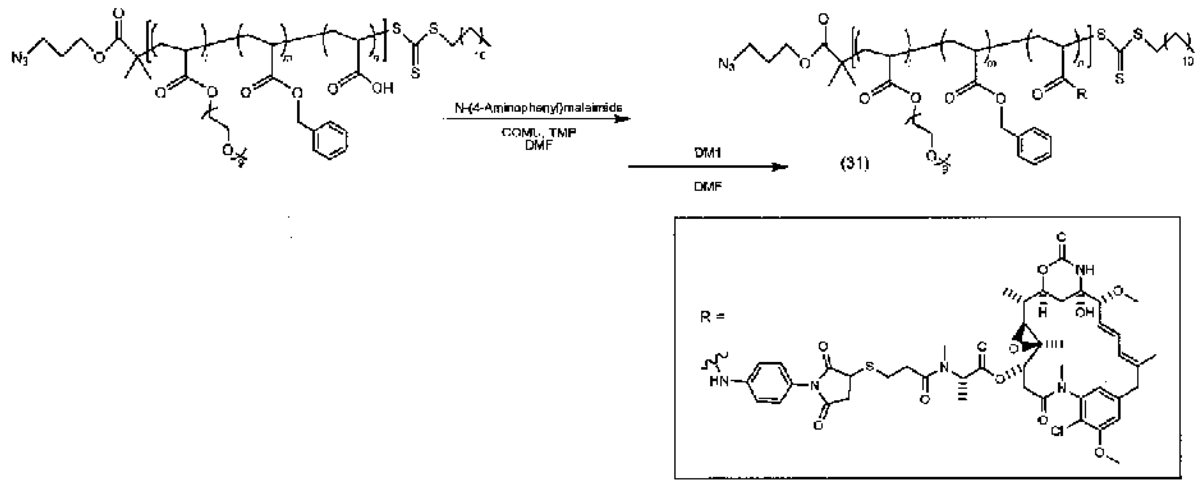

(31)

R =

For the DM1-N-4-APM-bonded copolymer, the number of DM1 introduced to one molecule of the copolymer was analyzed from a $^1$H-NMR spectrum measured by using the NMR, and a result thereof was 7 mol/mol.

[Measurement apparatuses and conditions]

(1) $^1$H-NMR measurement

Apparatus: JNM-ECX400 (400 MHz)/JEOL Ltd.
Solvent: dimethyl sulfoxide-$d_6$ containing 0.03% tetramethylsilane/KANTO CHEMICAL CO., INC.
Sample concentration: 20 mg/mL
Measurement temperature: 25°C
Number of integration times: 256 times
Results: Fig. 8

[Table 14]

| Example | Copolymer used | Number of DM1 introduced to one molecule of copolymer (mol/mol) |
|---|---|---|
| 108 | Example 72 | 7 |

[Example 109]

Synthesis of a 1,4-diaminobutane-bonded copolymer

To a solution of the copolymer obtained in Example 74 (810 mg) in DMF (16 mL), were added COMU (164.9 mg) and TMP (78 μL), and the mixture was stirred at room temperature for 3 hours. Thereto was added N-(tert-butoxycarbonyl)-1,4-diaminobutane (735 μL), and the mixture was stirred at 30°C for 3 days. The reaction solution was dialyzed and purified (dialysis membrane: Spectra/Por Regenerated Cellulose Membrane 6, molecular cut off: 3.5 kDa, and external liquid: methanol), and then the solvent was removed by the distillation under reduced pressure and the vacuum drying to recover the copolymer. An obtained N-Boc-1,4-diaminobutane copolymer was dissolved in a solution mixture of DCM and TFA [DCM/TFA = 5/3 (v/v), 32 mL] and the mixture was stirred at room temperature overnight to perform deprotection, and

then the solvent was removed by the distillation under reduced pressure and the vacuum drying to obtain the 1,4-diaminobutane-bonded copolymer (643 mg).

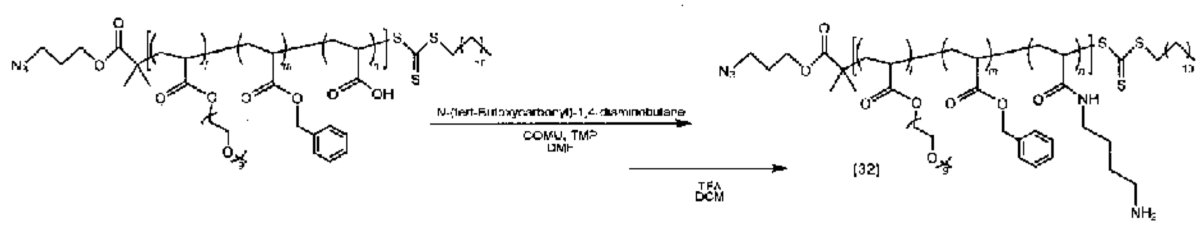


For the 1,4-diaminobutane-bonded copolymer, the number of 1,4-diaminobutane introduced to one molecule of the copolymer was analyzed from a $^{1}$H-NMR spectrum measured by using the NMR, and a result thereof was 12 mol/mol.

[Measurement apparatuses and conditions]

(1) $^{1}$H-NMR measurement

Apparatus: JNM-ECX400 (400 MHz)/JEOL Ltd.
Solvent: dimethyl sulfoxide-$d_6$ containing 0.03% tetramethylsilane/KANTO CHEMICAL CO., INC.
Sample concentration: 20 mg/mL
Measurement temperature: 25°C
Number of integration times: 256 times
Results: Fig. 9

[Table 15]

| Example | Copolymer used | Number of 1,4-diaminobutane introduced to one molecule of copolymer (mol/mol) |
|---|---|---|
| 109 | Example 74 | 12 |

[Examples 110 to 116]

For the copolymers obtained in Examples 73, 75 to 78 and 80 to 81, copolymers having different numbers of 1,4-diaminobutane introduced to one molecule of each of the copolymer as shown in the following table were synthesized by appropriately modifying a charge amount of N-(tert-butoxycarbonyl)-1,4-diaminobutane, and using the same method as in Example 109.

[Table 16]

| Example | Copolymer used | Number of 1,4-diaminobutane introduced to one molecule of copolymer (mol/mol) |
|---|---|---|
| 110 | Example 73 | 18 |
| 111 | Example 75 | 12 |
| 112 | Example 76 | 20 |
| 113 | Example 77 | 26 |
| 114 | Example 78 | 26 |
| 115 | Example 80 | 16 |
| 116 | Example 81 | 16 |

[Example 117]

Production of DM1-MHA-1,4-diaminobutane-bonded copolymer

In DMF (6 mL), the copolymer obtained in Example 109 (295 mg) was dissolved, and thereto were added COMU (59.3 mg) and TMP (28 μL), and the mixture was stirred at room temperature for 3 hours. Thereto was added 6-maleimidohexanoic acid (MHA) (293 mg), and the mixture was stirred at 30°C for 3 days. The reaction solution was dialyzed and purified (dialysis membrane: Spectra/Por Regenerated Cellulose Membrane 6, molecular cut off: 3.5 kDa, and external liquid: methanol), then the solvent was removed by the distillation under reduced pressure and the vacuum drying to obtain an MHA-1,4-diaminobutane-bonded copolymer (313 mg). The obtained MHA-1,4-diaminobutane-bonded copolymer was dissolved in DMF (10 mL), DM1 (80 mg) was added, and the mixture was stirred at 30°C for 24 hours. The reaction solution was dialyzed and purified (dialysis membrane: Spectra/Por Regenerated Cellulose Membrane 6, molecular cut off: 3.5 kDa, and external liquid: methanol), then the solvent was removed by the distillation under reduced pressure and the vacuum drying to obtain the DM1-MHA-1,4-diaminobutane-bonded copolymer (321 mg). A Z-average particle diameter and a polydispersity index of the obtained copolymer in water were measured by dynamic light scattering (DLS), and a result of the Z-average particle diameter was 10 nm (polydispersity index: 0.21).

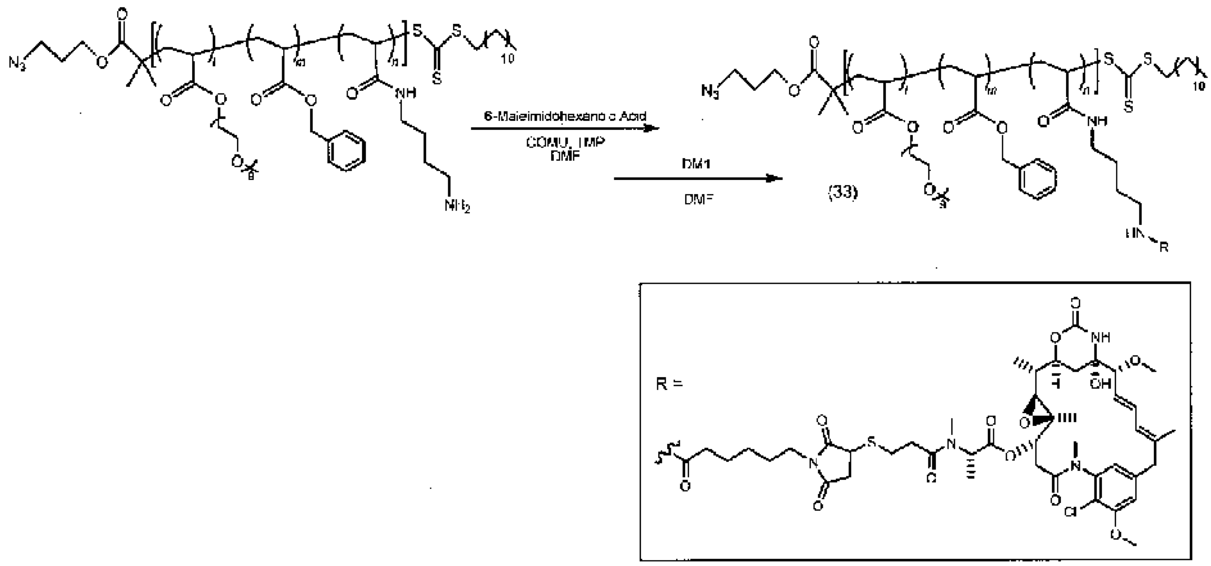


For the DM1-MHA-1,4-diaminobutane-bonded copolymer, the number of DM1 introduced to one molecule of the copolymer was analyzed from a $^1$H-NMR spectrum measured by using the NMR, and a result thereof was 12 mol/mol.

[Measurement apparatuses and conditions]

(1) $^1$H-NMR measurement

Apparatus: JNM-ECX400 (400 MHz)/JEOL Ltd.
Solvent: dimethyl sulfoxide-$d_6$ containing 0.03% tetramethylsilane/KANTO CHEMICAL CO., INC.
Sample concentration: 20 mg/mL
Measurement temperature: 25°C
Number of integration times: 256 times
Results: Fig. 10

[Table 17]

| Example | Copolymer used | Number of DM1 introduced to one molecule of copolymer (mol/mol) |
|---|---|---|
| 117 | Example 109 | 12 |

[Example 118]

Production of DM1-SPDP-1,4-diaminobutane-bonded copolymer

To a solution of DM1 (81 mg) and 2,5-dioxopyrrolidin-1-yl 3-(pyridin-2-yldisulfanyl)propanoate (SPDP) (29 mg) in DCM (1 mL) was added N,N-diisopropylethylamine (DIPEA) (16 μL), and the mixture was stirred at 30°C for 3 hours. To the reaction solution was added a solution of the copolymer obtained in Example 110 (200 mg) in DCM (4 mL), and the obtained reaction liquid was stirred at 30°C for 24 hours. The reaction solution was dialyzed and purified (dialysis membrane: Spectra/Por Regenerated Cellulose Membrane 6, molecular cut off: 3.5 kDa, and external liquid: methanol), then the solvent was removed by the distillation under reduced pressure and the vacuum drying, and the copolymer was recovered to obtain the DM1-SPDP-1,4-diaminobutane-bonded copolymer (189 mg). A Z-average particle diameter and a polydispersity index of the obtained copolymer in water were measured by dynamic light scattering (DLS), and a result of the Z-average particle diameter was 13 nm (polydispersity index: 0.23).

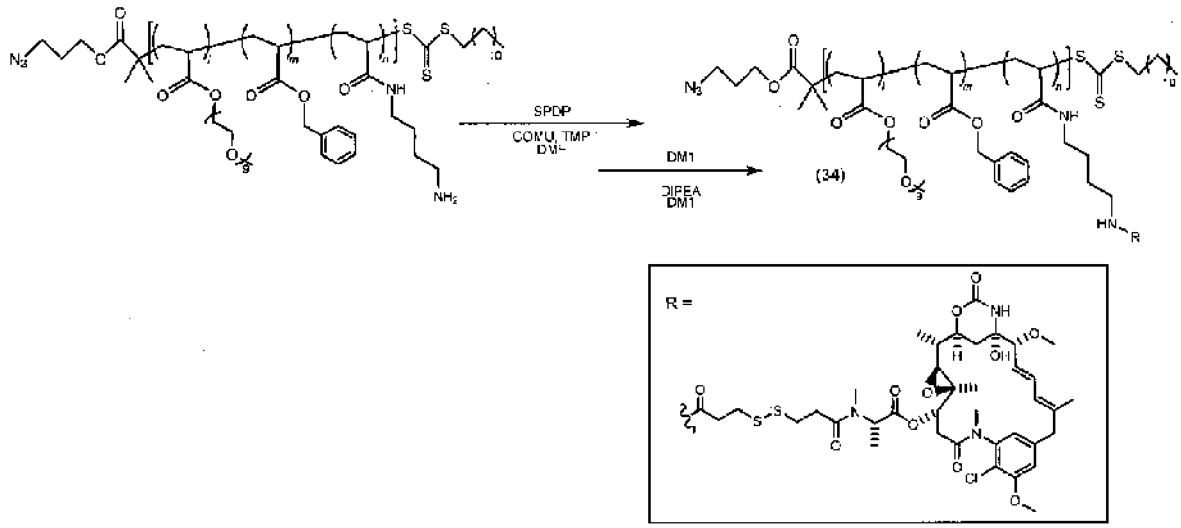


For the DM1-SPDP-1,4-diaminobutane-bonded copolymer, the number of DM1 introduced to one molecule of the copolymer was analyzed from a $^{1}$H-NMR spectrum measured by using the NMR, and a result thereof was 18 mol/mol.

[Measurement apparatuses and conditions]

(1) $^{1}$H-NMR measurement

Apparatus: JNM-ECX400 (400 MHz)/JEOL Ltd.
Solvent: dimethyl sulfoxide-$d_6$ containing 0.03% tetramethylsilane/KANTO CHEMICAL CO., INC.
Sample concentration: 20 mg/mL
Measurement temperature: 25°C
Number of integration times: 256 times
Results: Fig. 11

[Table 18]

| Example | Copolymer used | Number of DM1 introduced to one molecule of copolymer (mol/mol) |
|---|---|---|
| 118 | Example 110 | 18 |

[Examples 119 to 121]

For the copolymers obtained in Examples 112 to 114, copolymers having different numbers of DM1 introduced to one molecule of each of the copolymer as shown in the following table were synthesized by appropriately modifying the charge amount of DM1 and using the same method as in Example 118.

[Table 19]

| Example | Copolymer used | Number of DM1 introduced to one molecule of copolymer (mol/mol) |
|---|---|---|
| 119 | Example 112 | 16 |
| 120 | Example 113 | 20 |
| 121 | Example 114 | 26 |

[Example 122]

Production of DM1-SMCC-1,4-diaminobutane-bonded copolymer

To a solution of DM1 (39 mg) and N-succinimidyl 4-(N-Maleimidomethyl)cyclohexanecarboxylate (SMCC) (15 mg) in DCM (1 mL), was added the DIPEA (7.8 μL), and the mixture was stirred at 30°C for 3 hours. A solution of the copolymer obtained in Example 110 (96 mg) in DCM (4 mL), was added to the reaction solution, and the obtained reaction liquid was stirred at 30°C for 48 hours. The reaction solution was dialyzed and purified (dialysis membrane: Spectra/Por Regenerated Cellulose Membrane 6, molecular cut off: 3.5 kDa, and external liquid: methanol), then the solvent was removed by the distillation under reduced pressure and the vacuum drying, and the copolymer was recovered to obtain the DM1-SMCC-1,4-diaminobutane-bonded copolymer (122 mg). A Z-average particle diameter and a polydispersity index of the obtained copolymer in water were measured by dynamic light scattering (DLS), and a result of the Z-average particle diameter was 12 nm (polydispersity index: 0.29).

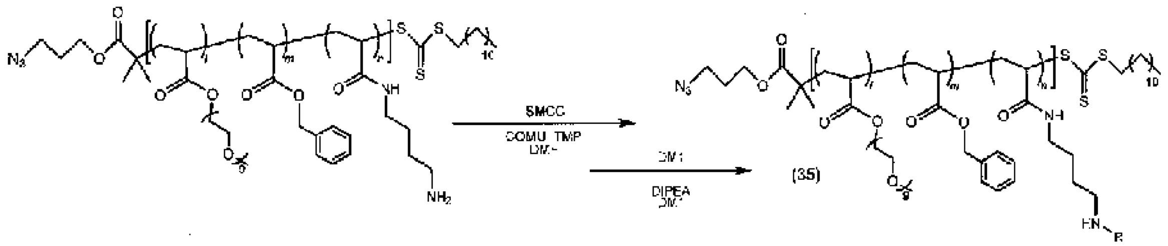


(35)

R =
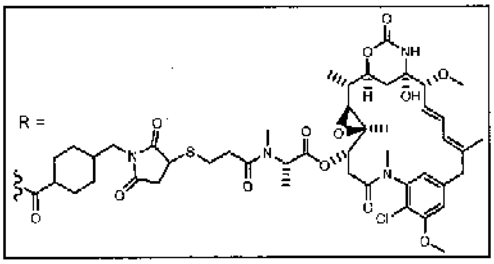

For the DM1-SMCC-1,4-diaminobutane-bonded copolymer, the number of DM1 introduced to one molecule of the copolymer was analyzed from a $^1$H-NMR spectrum measured by using the NMR, and a result thereof was 18 mol/mol.

[Measurement apparatuses and conditions]

(1) $^1$H-NMR measurement

Apparatus: JNM-ECX400 (400 MHz)/JEOL Ltd.
Solvent: dimethyl sulfoxide-$d_6$ containing 0.03% tetramethylsilane/KANTO CHEMICAL CO., INC.
Sample concentration: 20 mg/mL
Measurement temperature: 25°C
Number of integration times: 256 times
Results: Fig. 12

[Table 20]

| Example | Copolymer used | Number of DM1 introduced to one molecule of copolymer (mol/mol) |
|---|---|---|
| 122 | Example 110 | 18 |

[Example 123]

For the copolymer obtained in Example 111, copolymers having different numbers of DM1 introduced to one molecule of the copolymer as shown in the following table were synthesized by appropriately modifying the charge amount of DM1 and using the same method as in Example 122.

[Table 21]

| Example | Copolymer used | Number of DM1 introduced to one molecule of copolymer (mol/mol) |
|---|---|---|
| 123 | Example 111 | 7 |

[Example 124]

Production of DM1-CL-031-1,4-diaminobutane-bonded copolymer

To a solution of DM1 (81 mg) and 2,5-dioxopyrrolidin-1-yl 4-(pyridin-2-yldisulfanyl)pentanoate (CL-031) (32 mg) in DCM (1 mL), were added the DIPEA (16 μL), and the mixture was stirred at 30°C for 3 hours. A solution of the copolymer obtained in Example 110 (200 mg) in DCM (4 mL), was added to the reaction solution, and the obtained reaction liquid was stirred at 30°C for 24 hours. The reaction solution was dialyzed and purified (dialysis membrane: Spectra/Por Regenerated Cellulose Membrane 6, molecular cut off: 3.5 kDa, and external liquid: methanol), then the solvent was removed by the distillation under reduced pressure and the vacuum drying, and the copolymer was recovered to obtain the DM1-CL-031-1,4-diaminobutane-bonded copolymer (222 mg). A Z-average particle diameter and a polydispersity index of the obtained copolymer in water were measured by dynamic light scattering (DLS), and a result of the Z-average particle diameter was 13 nm (polydispersity index: 0.34).

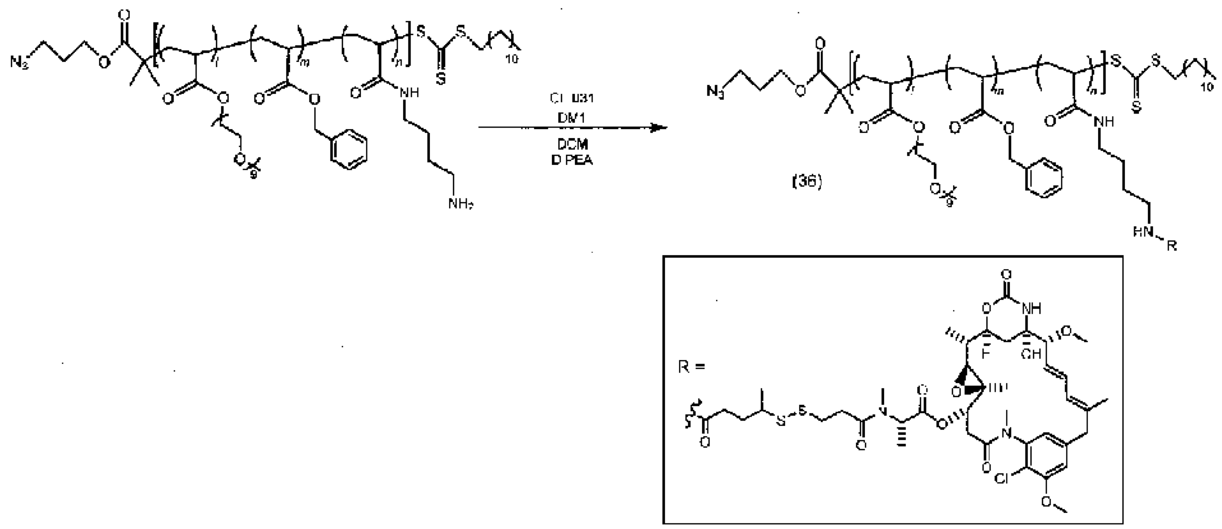

(36)

For the DM1-CL-031-1,4-diaminobutane-bonded copolymer, the number of DM1 introduced to one molecule of the copolymer was analyzed from a $^1$H-NMR spectrum measured by using the NMR, and a result thereof was 17 mol/mol.

[Measurement apparatuses and conditions]

(1) $^1$H-NMR measurement

Apparatus: JNM-ECX400 (400 MHz)/JEOL Ltd.
Solvent: dimethyl sulfoxide-$d_6$ containing 0.03% tetramethylsilane/KANTO CHEMICAL CO., INC.
Sample concentration: 20 mg/mL
Measurement temperature: 25°C
Number of integration times: 256 times
Results: Fig. 13

[Table 22]

| Example | Copolymer used | Number of DM1 introduced to one molecule of copolymer (mol/mol) |
|---|---|---|
| 124 | Example 110 | 17 |

[Example 125]

Production of DM1-CL-018-1,4-diaminobutane-bonded copolymer

To a solution of DM1 (114 mg) and 2,5-dioxopyrrolidin-1-yl 3-methyl-3-(pyridin-2-yldisulfanyl)butanoate (CL-018) (43 mg) in N,N-dimethylacetamide (DMAC) (1 mL), was added 4-dimethylaminopyridine (DMAP) (8.4 mg), and the mixture was stirred at 50°C for 2 hours. A solution of the copolymer obtained in Example 115 (150 mg) in DMAC (4 mL) was added to the reaction solution, and the obtained reaction liquid was stirred at 50°C for 24 hours. The reaction solution was dialyzed and purified (dialysis membrane: Spectra/Por Regenerated Cellulose Membrane 6, molecular cut off: 3.5 kDa, and external liquid: methanol), then the solvent was removed by the distillation under reduced pressure and the vacuum drying, and the copolymer was recovered to obtain the DM1-CL-018-1,4-diaminobutane-bonded copolymer (156 mg). A Z-average particle diameter and a polydispersity index of the obtained copolymer in water were measured by dynamic light scattering (DLS), and a result of the Z-average particle diameter was 13 nm (polydispersity index: 0.23).

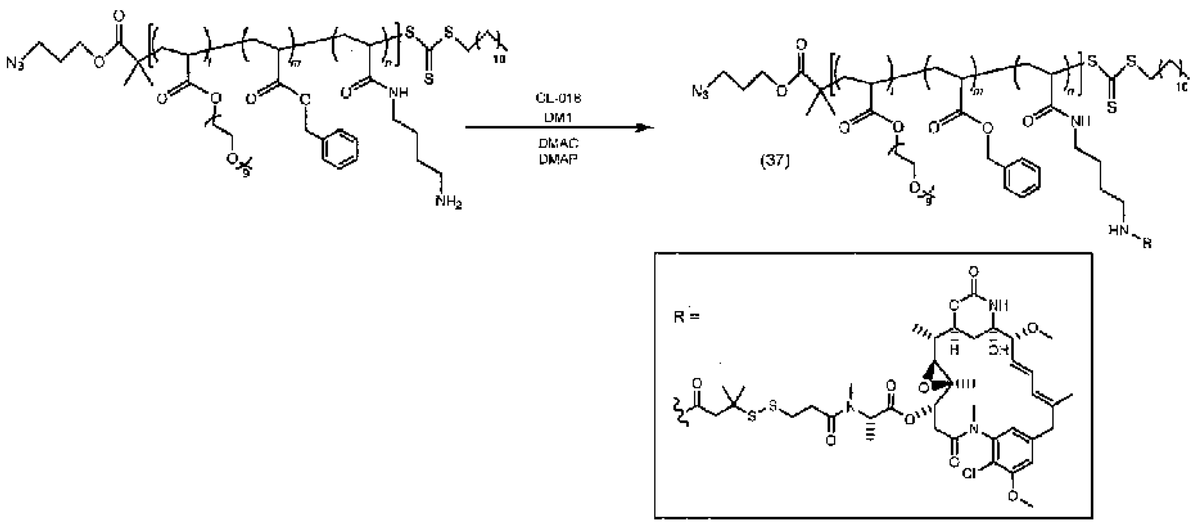

(37)

For the DM1-CL-018-1,4-diaminobutane-bonded copolymer, the number of DM1 introduced to one molecule of the copolymer was analyzed from a $^1$H-NMR spectrum measured by using the NMR, and a result thereof was 16 mol/mol.

[Measurement apparatuses and conditions]

(1) $^1$H-NMR measurement

Apparatus: JNM-ECX400 (400 MHz)/JEOL Ltd.
Solvent: dimethyl sulfoxide-$d_6$ containing 0.03% tetramethylsilane/KANTO CHEMICAL CO., INC.
Sample concentration: 20 mg/mL

Measurement temperature: 25°C
Number of integration times: 256 times
Results: Fig. 14

[Table 23]

| Example | Copolymer used | Number of DM1 introduced to one molecule of copolymer (mol/mol) |
|---|---|---|
| 125 | Example 115 | 16 |

[Example 126]

Production of DM1-CL-038-1,4-diaminobutane-bonded copolymer

To a solution of DM1 (76 mg) and 2,5-dioxopyrrolidin-1-yl 4-methyl-4-(pyridin-2-yldisulfanyl)pentanoate (CL-038) (29 mg) in DMAC (1 mL), were added DMAP (6 mg), and the mixture was stirred at 50°C for 2 hours. To the reaction solution was added a solution of the copolymer obtained in Example 115 (100 mg) in DMAC (4 mL), and the mixture was stirred at 50°C for 24 hours. The reaction solution was dialyzed and purified (dialysis membrane: Spectra/Por Regenerated Cellulose Membrane 6, molecular cut off: 3.5 kDa, and external liquid: methanol), then the solvent was removed by the distillation under reduced pressure and the vacuum drying, and the copolymer was recovered to obtain the DM1-CL-038-1,4-diaminobutane-bonded copolymer (56 mg). A Z-average particle diameter and a polydispersity index of the obtained copolymer in water were measured by dynamic light scattering (DLS), and a result of the Z-average particle diameter was 11 nm (polydispersity index: 0.32).

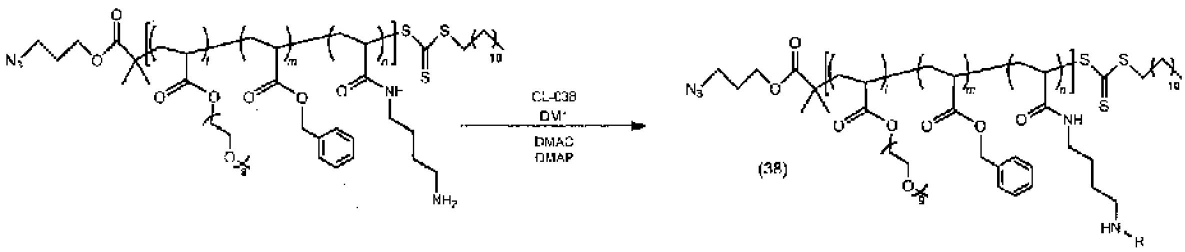


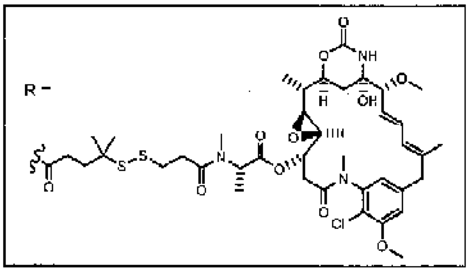

For the DM1-CL-038-1,4-diaminobutane-bonded copolymer, the number of DM1 introduced to one molecule of the copolymer was analyzed from a $^1$H-NMR spectrum measured by using the NMR, and a result thereof was 8 mol/mol.

[Measurement apparatuses and conditions]

(1) $^1$H-NMR measurement

Apparatus: JNM-ECX400 (400 MHz)/JEOL Ltd.
Solvent: dimethyl sulfoxide-$d_6$ containing 0.03% tetramethylsilane/KANTO CHEMICAL CO., INC.
Sample concentration: 20 mg/mL
Measurement temperature: 25°C
Number of integration times: 256 times
Results: Fig. 15

[Table 24]

| Example | Copolymer used | Number of DM1 introduced to one molecule of copolymer (mol/mol) |
|---|---|---|
| 126 | Example 115 | 8 |

[Example 127]

Production of DM1-CL-047-1,4-diaminobutane-bonded copolymer

To a solution of 4-succinimidyloxycarbonyl-α-methyl-α-(2-pyridyldithio)toluene (CL-047) (21.2 mg) in DMF (3 mL), were added the copolymer obtained in Example 113 (100 mg) and DIPEA (10 μL), and the mixture was stirred at 30°C for 24 hours. To the reaction liquid, DM1 (40 mg) was added, and the mixture was stirred at 30°C for 24 hours. The reaction solution was dialyzed and purified (dialysis membrane: Spectra/Por Regenerated Cellulose Membrane 6, molecular cut off: 3.5 kDa, and external liquid: methanol), then the solvent was removed by the distillation under reduced pressure and the vacuum drying, and the copolymer was recovered to obtain the DM1-CL-047-1,4-diaminobutane-bonded copolymer (153 mg). A Z-average particle diameter and a polydispersity index of the obtained copolymer in water were measured by dynamic light scattering (DLS), and a result of the Z-average particle diameter was 12 nm (polydispersity index: 0.18).

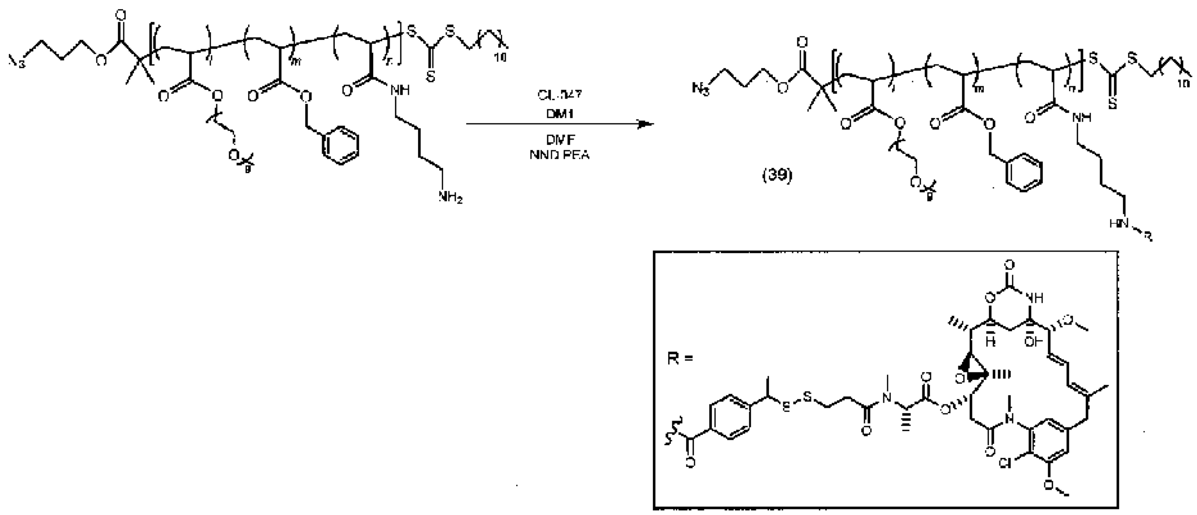

(39)

For the DM1-CL-047-1,4-diaminobutane-bonded copolymer, the number of DM1 introduced to one molecule of the copolymer was analyzed from a $^{1}$H-NMR spectrum measured by using the NMR, and a result thereof was 20 mol/mol.

[Measurement apparatuses and conditions]

(1) $^{1}$H-NMR measurement

Apparatus: JNM-ECX400 (400 MHz)/JEOL Ltd.
Solvent: dimethyl sulfoxide-$d_6$ containing 0.03% tetramethylsilane/KANTO CHEMICAL CO., INC.
Sample concentration: 20 mg/mL
Measurement temperature: 25°C
Number of integration times: 256 times
Results: Fig. 16

[Table 25]

| Example | Copolymer used | Number of DM1 introduced to one molecule of copolymer (mol/mol) |
|---|---|---|
| 127 | Example 113 | 20 |

[Example 128]

Production of SN-38-CO-1,4-diaminobutane-bonded copolymer

In a solution of SN-38 (0.5 g) in DCM (50 mL), were added di-tert-butyl dicarbonate (353 mg) and pyridine (3 mL), and the mixture was stirred overnight at room temperature. The reaction liquid was transferred to a separatory funnel and washed 3 times with a 0.5N aqueous HCl solution (150 mL) followed by once with a saturated aqueous $NaHCO_3$ solution. The organic layer was recovered, DCM was distilled under reduced pressure with an evaporator, and then vacuum-dried to obtain Boc-SN-38 (0.586 g).

Next, the copolymer obtained in Example 115 (200 mg) was dissolved in benzene and lyophilized. Under an argon atmosphere, dissolution occurred in THF, the Boc-SN-38 (27.6 mg), 1,1'-carbonyldiimidazole (12 mg), and DMAP (15 mg) were added, and the mixture was stirred at room temperature for 3 hours. The reaction solution was dialyzed and purified (dialysis membrane: Spectra/Por Regenerated Cellulose Membrane 6, molecular cut off: 3.5 kDa, and external liquid: methanol), then the solvent was removed by the distillation under reduced pressure and the vacuum drying to recover a Boc-SN-38-CO-1,4-diaminobutane-bonded copolymer. The obtained Boc-SN-38-CO-1,4-diaminobutane-bonded copolymer was dissolved in the solution mixture of DCM and TFA [DCM/TFA = 5/3 (v/v)] (32 mL) and the mixture was stirred at room temperature overnight to perform deprotection, and then the solvent was removed by the distillation under reduced pressure and the vacuum drying to obtain the SN-38-CO-1,4-diaminobutane-bonded copolymer (220 mg). A Z-average particle diameter and a polydispersity index of the obtained copolymer in water were measured by dynamic light scattering (DLS), and a result of the Z-average particle diameter was 8 nm (polydispersity index: 0.23).

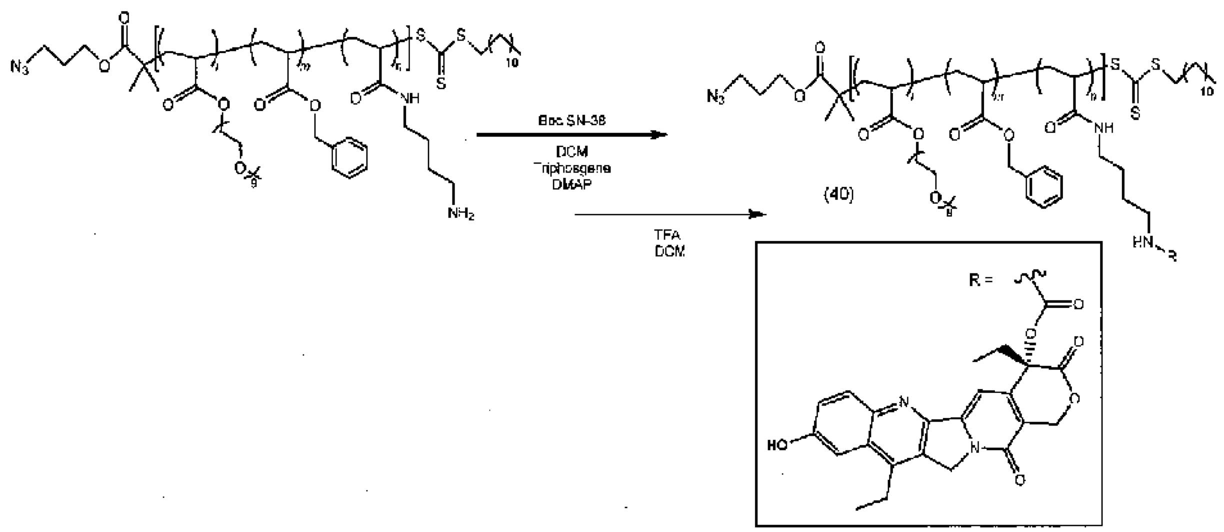


For the SN-38-CO-1,4-diaminobutane-bonded copolymer, the number of the SN-38 introduced to one molecule of the copolymer was analyzed from a $^1$H-NMR spectrum measured by using the NMR, and a result thereof was 13 mol/mol.

[Measurement apparatuses and conditions]

(1) $^1$H-NMR measurement

Apparatus: JNM-ECX400 (400 MHz)/JEOL Ltd.
Solvent: dimethyl sulfoxide-$d_6$ containing 0.03% tetramethylsilane/KANTO CHEMICAL CO., INC.
Sample concentration: 20 mg/mL
Measurement temperature: 25°C
Number of integration times: 256 times
Results: Fig. 17

[Table 26]

| Example | Copolymer used | Number of SN-38 introduced to one molecule of copolymer (mol/mol) |
|---|---|---|
| 128 | Example 115 | 13 |

[Example 129]

Production of deruxtecan-SPDP-1,4-diaminobutane-bonded copolymer

In DMF (4 mL), the copolymer obtained in Example 116 (200 mg), 2,5-dioxopyrrolidin-1-yl 3-(pyridin-2-yldisulfanyl)propanoate (SPDP) (14.2 mg), and deruxtecan (51.4 mg) were dissolved, and the mixture was stirred at 30°C for 2 hours. Then, an aqueous tris(2-carboxyethyl)phosphine (TCEP) solution (1 mL) was added dropwise into the reaction liquid while being stirred, and the mixture was stirred overnight at 30°C. The obtained reaction liquid was stirred at 30°C for 24 hours. The reaction solution was dialyzed and purified (dialysis membrane: Spectra/Por Regenerated Cellulose Membrane 6, molecular cut off: 3.5 kDa, and external liquid: methanol), then the solvent was removed by the distillation under reduced pressure and the vacuum drying, and the copolymer was recovered to obtain the deruxtecan-SPDP-1,4-diaminobutane-bonded copolymer (thioether bond) (205 mg). A Z-average particle diameter and a polydispersity index of the obtained copolymer in water were measured by dynamic light scattering (DLS), and a result of the Z-average particle diameter was 12 nm (polydispersity index: 0.23).

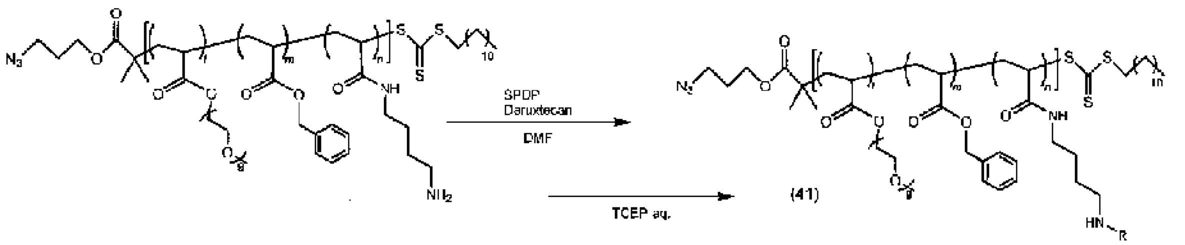


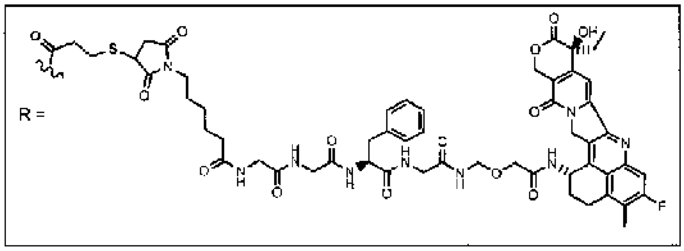

For the deruxtecan-SPDP-1,4-diaminobutane-bonded copolymer, the number of the deruxtecan introduced to one molecule of the copolymer was analyzed from a $^1$H-NMR spectrum measured by using the NMR, and a result thereof was 16 mol/mol.

[Measurement apparatuses and conditions]

(1) $^1$H-NMR measurement

Apparatus: JNM-ECX400 (400 MHz)/JEOL Ltd.
Solvent: dimethyl sulfoxide-$d_6$ containing 0.03% tetramethylsilane/KANTO CHEMICAL CO., INC.
Sample concentration: 20 mg/mL
Measurement temperature: 25°C
Number of integration times: 256 times
Results: Fig. 18

[Table 27]

| Example | Copolymer used | Number of deruxtecan introduced to one molecule of copolymer (mol/mol) |
|---|---|---|
| 129 | Example 116 | 16 |

[Example 130]

Production of 4-hydroxybutylamine-bonded copolymer

In DMF (4 mL), the copolymer obtained in Example 82 (200 mg) was dissolved after being weighed, COMU (64.4 mg) and TMP (31 μL) were added, and the mixture was stirred at room temperature for 3 hours. Then, 4-amino-1-butanol (73 μL) was added, and the mixture was stirred overnight at 30°C. The reaction solution was dialyzed and purified (dialysis membrane: Spectra/Por Regenerated Cellulose Membrane 6, molecular cut off: 3.5 kDa, and external liquid: methanol), then the solvent was removed by the distillation under reduced pressure and the vacuum drying to obtain the 4-hydroxybutylamine-bonded copolymer (205 mg).

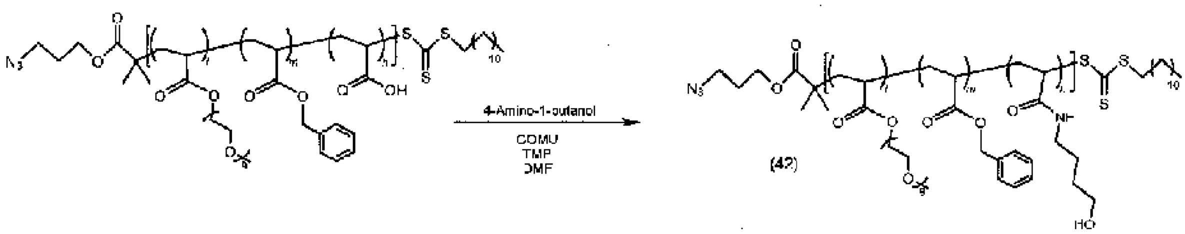


For the 4-hydroxybutylamine-bonded copolymer, the number of 4-amino-1-butanol introduced to one molecule of the copolymer was analyzed from a $^1$H-NMR spectrum measured by using the NMR, and a result thereof was 15 mol/mol.

[Measurement apparatuses and conditions]

(1) $^1$H-NMR measurement

Apparatus: JNM-ECX400 (400 MHz)/JEOL Ltd.
Solvent: dimethyl sulfoxide-$d_6$ containing 0.03% tetramethylsilane/KANTO CHEMICAL CO., INC.
Sample concentration: 20 mg/mL
Measurement temperature: 25°C
Number of integration times: 256 times
Results: Fig. 19

[Table 28]

| Example | Copolymer used | Number of 4-amino-1-butanol introduced to one molecule of copolymer (mol/mol) |
|---|---|---|
| 130 | Example 82 | 15 |

[Example 131]

Synthesis of a staurosporine (STS)-bonded copolymer

In THF, the copolymer obtained in Example 130 (200 mg) was dissolved, triphosgene (7.2 mg) and DMAP (22.1 mg) were added, and the mixture was stirred at 10°C for 30 minutes. Then, staurosporine (28.2 mg) was added, and the mixture was stirred at room temperature for 3 hours. The reaction solution was dialyzed and purified (dialysis membrane: Spectra/Por Regenerated Cellulose Membrane 6, molecular cut off: 3.5 kDa, and external liquid: methanol), and then the

solvent was removed by the distillation under reduced pressure and the vacuum drying to obtain the staurosporine-bonded copolymer (233 mg). A Z-average particle diameter and a polydispersity index of the obtained copolymer in water were measured by dynamic light scattering (DLS), and a result of the Z-average particle diameter was 10 nm (polydispersity index: 0.21).

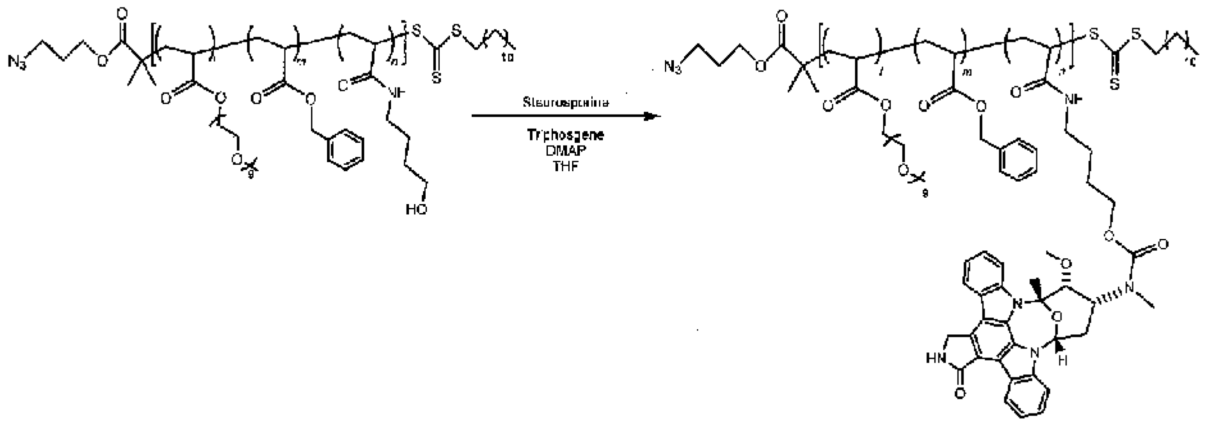


For the STS-bonded copolymer, the number of the STS introduced to one molecule of the copolymer was analyzed from a $^1$H-NMR spectrum measured by using the NMR, and a result thereof was 13 mol/mol.

[Measurement apparatuses and conditions]

(1) $^1$H-NMR measurement

Apparatus: JNM-ECX400 (400 MHz)/JEOL Ltd.
Solvent: dimethyl sulfoxide-$d_6$ containing 0.03% tetramethylsilane/KANTO CHEMICAL CO., INC.
Sample concentration: 20 mg/mL
Measurement temperature: 25°C
Number of integration times: 256 times
Results: Fig. 20

[Table 29]

| Example | Copolymer used | Number of STS introduced to one molecule of copolymer (mol/mol) |
|---|---|---|
| 131 | Example 130 | 13 |

[Example 132]

Production of cetuximab-maleimide-PEG4-DBCO-bonded micelle drug complex

To a glass vial into which a cetuximab (Erbitux, Merck Biopharma) antibody stock solution (10 mg/mL, 3 mL) and PBS (9 mL) were dispensed, 10 mM tris(2-carboxyethyl)phosphine (TCEP) (42 μL) dissolved in PBS was added, and the mixture was stirred at 37°C for 15 minutes. Then, 10 mM DBCO-PEG4-maleimide (99 μL) dissolved in DMSO was added, stirred at 37°C for 15 minutes, and reacted with a cysteine residue of the antibody. To that, an aqueous solution (10 mg/mL, 20 μL) obtained by dissolving L-cysteine (168149, Sigma-Aldrich) to PBS was added, and the mixture was stirred at 37°C for 1 minute to stop the reaction. The reaction liquid was recovered, dialyzed against purified water overnight (Slide-A-Lyzer™ Dialysis Cassette, molecular cut off: 20 K, 7736, Thermo Fisher Scientific), and then subjected to ultrafiltration (Vivaspin Turbo 15, molecular cut off: 100 K) to remove an unreacted modifying reagent. Dilution was performed in a measuring flask to exactly 5 mL with PBS, and the solution (1 mL) was dispensed into glass vials. Next, the copolymer obtained in Example 99 (10 mg) was added and left to stand in BICELL™ at -20°C for 1 day. Then, the solution obtained by thawing at 4°C was recovered. After a copolymer-bonded antibody and an unbonded antibody were separated by

hydrophobic chromatography due to the difference in retention time, target fractions were each recovered, and washed by the ultrafiltration (Vivaspin Turbo 15, molecular cut off: 100 K) with PBS to obtain the cetuximab-maleimide-PEG4-DBCO-bonded micelle drug complex as an object.

A Z-average particle diameter and a polydispersity index of the cetuximab-maleimide-PEG4-DBCO-bonded micelle drug complex were measured by dynamic light scattering (DLS). Further, the number average molecular weight (Mn, MALS) of the complex in water was measured by size exclusion chromatography (SEC) equipped with a multi angle light scattering (MALS) detector to calculate the absolute molecular weight, and the number of the copolymers bonded to one antibody and an antibody drug binding ratio (DAR) were calculated from a molecular weight of the copolymer in combination with the antibody molecular weight.

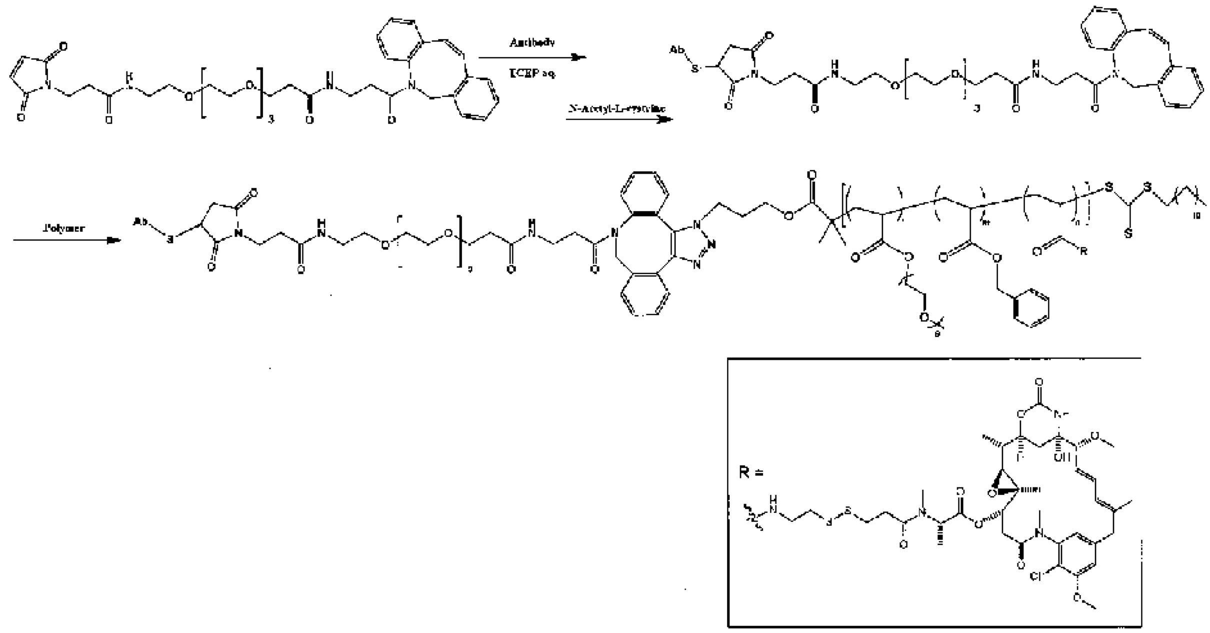


[Measurement apparatuses and conditions]

(1) SEC-MALS measurement

Apparatus: Waters Alliance/Waters
Detector: 2998 PDA detector/Waters 2414 Refractive index detector/Waters DAWN HELEOSII 8+/WYATT
Column: TSKgel G-3000PWXL/Tosoh Corporation
(Column size: 7.8 mm × 300 mm, particle size: 7 μm, and exclusion limit molecular weight: 2 × $10^5$)
TSKgel guardcolum/Tosoh Corporation
Mobile phase: 100 mmol/ L sodium chloride aqueous solution
Temperature: 25°C
Flow rate: 0.8 mL/min
Sample concentration: 10 mg/mL

[Table 30]

| Example | Copolymer used | Antibody | Drug | Fraction | Number of drugs bonded to one molecule of copolymer (mol/mol) | Number of copolymers bonded to one antibody (mol/mol) | DAR | Z-average particle diameter (nm) | Polydispersity index |
|---|---|---|---|---|---|---|---|---|---|
| 132 | Example 99 | Cetuximab | DM1 | 1 | 20 | 1.2 | 24 | 14 | 0.13 |
| | | | | 2 | | 2.0 | 40 | 18 | 0.11 |

[Examples 133 to 134]

In place of DBCO-PEG4-maleimide used in Example 132, synthesis was performed by using the DBCO-PEG12-maleimide in Example 133, or the DBCO-PEG24-maleimide in Example 134 by the same preparation method as in Example 132.

[Table 31]

| Example | Copolymer used | Antibody | Drug | Fraction | Number of drugs bonded to one molecule of copolymer (mol/mol) | Number of copolymers bonded to one antibody (mol/mol) | DAR | Z-average particle diameter (nm) | Polydispersity index |
|---|---|---|---|---|---|---|---|---|---|
| 133 | Example 99 | Cetuximab | DM1 | - | 20 | 1.9 | 38 | 19 | 0.20 |
| 134 | Example 99 | Cetuximab | DM1 | - | 20 | 2.1 | 42 | 20 | 0.22 |

[Examples 135 to 144]

For the copolymers obtained in Examples 83, 96, 100, 101, 120, 121, 122, 124, 125 and 128, synthesis was performed by appropriately modifying the charge amounts and using the same method as in Example 132.

[Table 32]

| Example | Copolymer used | Antibody | Drug | Fraction | Number of drugs bonded to one molecule of copolymer (mol/mol) | Number of copolymers bonded to one antibody (mol/mol) | DAR | Z-average particle diameter (nm) | Polydispersity index |
|---|---|---|---|---|---|---|---|---|---|
| 135 | Example 100 | Cetuximab | DM1 | 1 | 26 | 1.0 | 26 | 14 | 0.17 |
| | | | | 2 | | 1.6 | 42 | 15 | 0.19 |
| 136 | Example 125 | | DM1 | 1 | 16 | 1.2 | 18 | 17 | 0.08 |
| | | | | 2 | | 1.6 | 24 | 18 | 0.08 |
| 137 | Example 124 | | DM1 | 1 | 17 | 1.6 | 27 | 21 | 0.30 |
| | | | | 2 | | 1.8 | 31 | 17 | 0.15 |
| 138 | Example 120 | | DM1 | - | 20 | 1.9 | 38 | 18 | 0.11 |
| 139 | Example 121 | | DM1 | 1 | 26 | 1.2 | 31 | 17 | 0.32 |
| | | | | 2 | | 2.0 | 52 | 18 | 0.25 |
| | | | | 3 | | 3.3 | 85 | 25 | 0.25 |
| 140 | Example 101 | | DM1 | - | 19 | 1.9 | 36 | 16 | 0.10 |
| 141 | Example 128 | | SN-38 | - | 13 | 1.8 | 23 | 14 | 0.13 |
| 142 | Example 125 | | STS | - | 13 | 2.1 | 27 | 14 | 0.09 |
| 143 | Example 83 | | SCNP | - | 0 | 1.9 | 0 | 15 | 0.07 |
| 144 | Example 122 | | DM1 | 1 | 18 | 1.4 | 25 | 17 | 0.08 |
| | | | | 2 | | 2.1 | 38 | 17 | 0.07 |

[Example 145]

Production of cetuximab-NHS-DBCO-bonded micelle drug complex

The copolymer obtained in Example 118 and cetuximab were complexed. To a glass vial into which a PBS solution of the cetuximab (2.07 mg/mL, 14.5 mL) was dispensed, Sulfo DBCO-NHS Ester (0.5 mL) dissolved in PBS was added at 1.22 mg/mL. The mixture was stirred for 3 hours, with which a lysine residue of the antibody was reacted. Then, the unreacted modifying reagent was removed by the dialysis purification (dialysis membrane: Spectra/Por Regenerated Cellulose Membrane 6, molecular cut off: 3.5 kDa, and external liquid: purified water) and the ultrafiltration (Vivaspin Turbo 15, molecular cut off: 100 K). Dilution was performed in a measuring flask to exactly 5 mL with PBS. Into a glass vial, the solution (1 mL) was dispensed (antibody: 6 mg/vial) and recovered by lyophilization. Thereafter, the cetuximab-NHS-DBCO-bonded micelle drug complex, which is an object, was recovered by the same operation as in Example 132.

A Z-average particle diameter and a polydispersity index of the cetuximab-NHS-DBCO-bonded micelle drug complex were measured by dynamic light scattering (DLS), and a result of the Z-average particle diameter was 32 nm (polydispersity index: 0.47). Further, the absolute molecular weight was calculated by MALS measurement, and the number of the copolymers bonded to one antibody calculated from the molecular weight of the copolymer in combination with the antibody molecular weight was 1.9, and the antibody drug binding ratio (DAR) was 34.

[Table 33]

| Example | Copolymer used | Antibody | Drug | Fraction | Number of drugs bonded to one molecule of copolymer (mol/mol) | Number of copolymers bonded to one antibody (mol/mol) | DAR | Z-average particle diameter (nm) | Polydispersity index |
|---|---|---|---|---|---|---|---|---|---|
| 145 | Example 118 | Cetuximab | DM1 | - | 18 | 1.9 | 34 | 32 | 0.47 |

[Examples 146 to 148]

In place of the Sulfo DBCO-NHS ester used in Example 145, synthesis was performed by using the DBCO-NHS ester used in Example 146, the DBCO-PEG5-NHS ester used in Example 147, or the DBCO-PEG13-NHS ester used in Example 148 by the same preparation method as in Example 145.

[Table 34]

| Example | Copolymer used | Antibody | Drug | Fraction | Number of drugs bonded to one molecule of copolymer (mol/mol) | Number of copolymers bonded to one antibody (mol/mol) | DAR | Z-average particle diameter (nm) | Polydispersity index |
|---|---|---|---|---|---|---|---|---|---|
| 146 | Example 118 | Cetuximab | DM1 | - | 18 | 1.5 | 27 | 26 | 0.34 |
| 147 | Example 118 | Cetuximab | DM1 | - | 18 | 1.8 | 32 | 27 | 0.21 |
| 148 | Example 118 | Cetuximab | DM1 | - | 18 | 1.9 | 34 | 27 | 0.22 |

[Examples 149 to 152]

Production of Trastuzumab-bonded micelle drug complex

The same procedure set forth in Example 132 was carried out, where Trastuzumab (Herceptin, Chugai Pharmaceutical CO., LTD.) was used as an antibody.

[Table 35]

| Example | Copolymer used | Antibody | Drug | Fraction | Number of drugs bonded to one molecule of copolymer (mol/mol) | Number of copolymers bonded to one antibody (mol/mol) | DAR | Z-average particle diameter (nm) | Polydispersity index |
|---|---|---|---|---|---|---|---|---|---|
| 149 | Example 119 | Trastuzumab | DM1 | 1 | 16 | 1.1 | 18 | 17 | 0.21 |
| | | | | 2 | | 1.6 | 26 | 26 | 0.23 |
| | | | | 3 | | 2.4 | 38 | 28 | 0.19 |
| 150 | Example 123 | | DM1 | - | 7 | 3.3 | 23 | 23 | 0.25 |
| 151 | Example 120 | | DM1 | - | 20 | 2.0 | 40 | 26 | 0.23 |
| 152 | Example 129 | | Deruxtecan | - | 16 | 1.6 | 26 | 19 | 0.26 |

[Example 153]

Production of Rituximab-S-bonded micelle drug complex

The same procedure set forth in Example 132 was carried out, where Rituximab (Rituxan, Zenyaku Kogyo Co., Ltd.) was used as an antibody.

[Table 36]

| Example | Copolymer used | Antibody | Drug | Fraction | Number of drugs bonded to one molecule of copolymer (mol/mol) | Number of copolymers bonded to one antibody (mol/mol) | DAR | Z-average particle diameter (nm) | Polydispersit y index |
|---|---|---|---|---|---|---|---|---|---|
| 153 | Example 119 | Rituximab | DM1 | 1 | 16 | 1.7 | 27 | 21 | 0.22 |
| | | | | 2 | | 2.6 | 42 | 16 | 0.20 |

[Example 154]

Production of Rituximab-NH-bonded micelle drug complex

The same procedure set forth in Example 146 was carried out, where Rituximab (Rituxan, Zenyaku Kogyo Co., Ltd.) was used as an antibody.

[Table 37]

| Example | Copolymer used | Antibody | Drug | Fraction | Number of drugs bonded to one molecule of copolymer (mol/mol) | Number of copolymers bonded to one antibody (mol/mol) | DAR | Z-average particle diameter (nm) | Polydispersity index |
|---|---|---|---|---|---|---|---|---|---|
| 154 | Example 119 | Rituximab | DM1 | 1 | 16 | 1.5 | 24 | 20 | 0.20 |
| | | | | 2 | | 2.5 | 40 | 18 | 0.24 |

[Example 155]

Production of Panitumumab-bonded micelle drug complex

The same procedure set forth in Example 132 was carried out, where Panitumumab (Vectibix™, Takeda Pharmaceutical Company Limited) was used as an antibody.

[Table 38]

| Example | Copolymer used | Antibody | Drug | Fraction | Number of drugs bonded to one molecule of copolymer (mol/mol) | Number of copolymers bonded to one antibody (mol/mol) | DAR | Z-average particle diameter (nm) | Polydispersity index |
|---|---|---|---|---|---|---|---|---|---|
| 155 | Example 121 | Panitumumab | DM1 | 1 | 26 | 0.8 | 21 | 13 | 0.11 |
| | | | | 2 | | 1.5 | 39 | 18 | 0.22 |
| | | | | 3 | | 2.6 | 69 | 17 | 0.22 |

[Example 156]

Production of IgG-bonded micelle drug complex

The same procedure set forth in Example 132 was carried out, where IgG (normal human IgG, whole molecule, purified product, 143-09501, FUJIFILM Wako Pure Chemical Corporation) was used as an antibody.

[Table 39]

| Example | Copolymer used | Antibody | Drug | Fraction | Number of drugs bonded to one molecule of copolymer (mol/mol) | Number of copolymers bonded to one antibody (mol/mol) | DAR | Z-average particle diameter (nm) | Polydispersity index |
|---|---|---|---|---|---|---|---|---|---|
| 156 | Example 121 | IgG | DM1 | - | 26 | 1.8 | 47 | 17 | 0.20 |

[Example 157]

Production of cetuximab-Fab-bonded micelle drug complex

A fragmented antibody (Fab) of cetuximab was prepared by using a commercially available purification kit (Fab Preparation Kit, Pierce, #44985), and the same procedure as Example 145 was performed.

[Table 40]

| Example | Copolymer used | Antibody | Drug | Fraction | Number of drugs bonded to one molecule of copolymer (mol/mol) | Number of copolymers bonded to one antibody (mol/mol) | DAR | Z-average particle diameter (nm) | Polydispersity index |
|---|---|---|---|---|---|---|---|---|---|
| 157 | Example 121 | Cetuximab-Fab | DM1 | 1 | 26 | 1.0 | 26 | 15 | 0.13 |
| | | | | 2 | | 1.4 | 35 | 14 | 0.12 |

[Example 158]

Production of exatecan-PAB-Cit-Val-Ahx-bonded copolymer

Step 1: synthesis of Boc-Ahx-Val-Cit-PAB-PNP

To a solution of Boc-Ahx-Val-Cit-PAB-OH (compound HDP 30.1267 described in WO 2016/142049 A) (239 mg) and bis(4-nitrophenyl)carbonate (362 mg) in DMF (1.5 mL), was added N, N-diisopropylethylamine (207 $\mu$L), and the mixture was stirred overnight at room temperature. The reaction liquid was distilled under reduced pressure with the evaporator, and then purified by silica gel column chromatography ($CHCl_3$/MeOH = 100/0 to 85/15) to obtain the Boc-Ahx-Val-Cit-PAB-PNP (241 mg) .

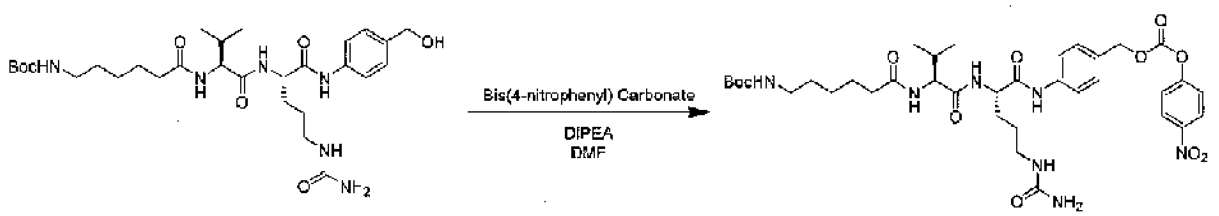


$^1$H NMR(400 MHz,DMSO-$d_6$)$\delta$:10.06(s,1H),8.33-8.29(m,2H),8.11(d,J=7.6Hz,1H),7.80(d,J=8.5Hz,1H),7.65(d,J=9.0 Hz,2H),7.57(dt,J=10.0,2.7Hz,2H),7.41(d,J=8.8Hz,2H),6.75(t,J =5.6Hz,1H),5.97(t,J=5.8Hz,1H),5.42(s,2H),5.24(s,2H),4.41-4.34(m,1H),4.20(dd,J=8.8,7.6Hz,1H),3.07-2.92(m,2-H),2.90-2.85(m,2H),2.23-2.08(m,2H),2.01-1.91(m,1H),1.75-1.32(m,17H),1.25-1.17(m,2H),0.87(d,J=6.8Hz,3-H),0.83(d,J=6.8Hz,3H).MS(ESI)m/z :758.2[M+H]$^+$

Step 2: synthesis of Boc-Ahx-Val-Cit-PAB-exatecan

To a solution of Boc-Ahx-Val-Cit-PAB-PNP (240 mg) and exatecan mesylate (168 mg) in DMSO (6.33 mL), was added triethylamine (88.2 $\mu$L), and the mixture was stirred overnight at room temperature. Water was added to the reaction liquid, and then a generated precipitate was collected by filtration with suction. The filtered product was purified by the silica gel column chromatography ($CHCl_3$/MeOH = 100/0 to 85/15) to obtain the Boc-Ahx-Val-Cit-PAB-exatecan (202 mg).

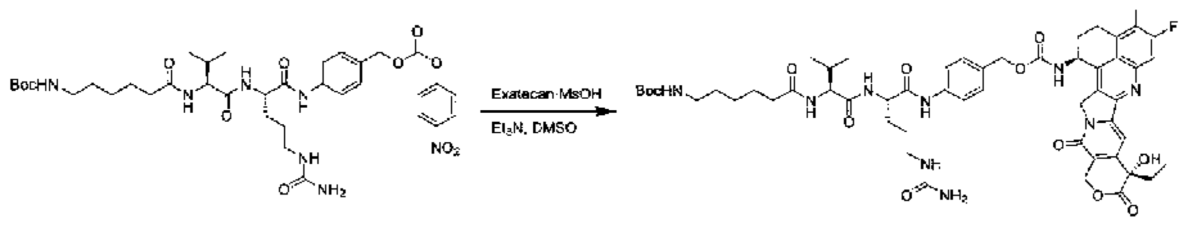


$^1$H NMR(400 MHz,DMSO-$d_6$) 6:9.98(s,1H),8.09-8.04(m,2H),7.81-7.76(m,2H),7.61(d,J=8.5Hz,2H),7.36(d,J=8.5Hz,2H),7.31(s,1H) ,6.74(t,J=5.6Hz,1-H),6.52(s,1H),5.97(t,J=5.8Hz,1H),5.45(s,2H ),5.41(s,2H),5.34-5.23 (m,3H),5.08 (s,2H),4.40-4.33 (m,1H),4.19 (dd,J = 8.5,6.7 Hz,1H),3.30-3.06 (m,2H),3.05-2.84 (m,4H),2.37 (s,3H),2.24-2.08 (m,4H),2.03-1.80 (m,3H),1.74-1.31 (m,17H),1.21-1.18 (m,2H),0.89-0.82 (m,9H).MS(ESI)m/z:1054.5[M+H]$^+$

Step 3: synthesis of H-Ahx-Val-Cit-PAB-exatecan trifluoroacetate

To a solution of Boc-Ahx-Val-Cit-PAB-exatecan (100 mg) in DCM (3 mL), trifluoroacetic acid (0.3 mL) was added, and the mixture was stirred at room temperature for 4 hours. The reaction liquid was distilled under reduced pressure with the evaporator, and then purified by reverse phase chromatography (0.1% TFA aqueous solution/MeCN = 100/0 to 60/40) with an ODS column to obtain the H-Ahx-Val-Cit-PAB-exatecan trifluoroacetate (63.7 mg).

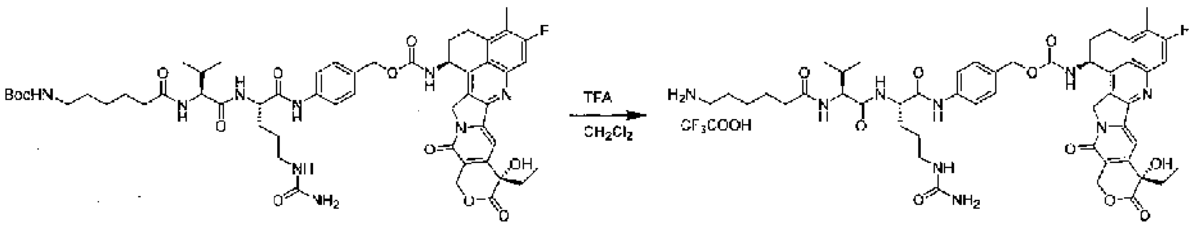


MS(ESI)m/z: 954.5 [M+H]+

Step 4: synthesis of an exatecan-PAB-Cit-Val-Ahx-bonded copolymer

In DMF (1 mL), a copolymer obtained by the same preparation method as in Example 72 (100 mg) was dissolved, H-Ahx-Val-Cit-PAB-exatecan trifluoroacetate (25.4 mg), N,N-diisopropylethylamine (15.5 μL), and HATU (11.3 mg) were added, and the mixture was stirred overnight at room temperature. The reaction liquid was dialyzed and purified (dialysis membrane: Spectra/Por Regenerated Cellulose Membrane 6, molecular cut off: 3.5 kDa, and external liquid: methanol), then the solvent was removed by the distillation under reduced pressure and the vacuum drying, and the copolymer was recovered to obtain the exatecan-PAB-Cit-Val-Ahx-bonded copolymer (106 mg). A Z-average particle diameter and a polydispersity index of the obtained copolymer in water were measured by dynamic light scattering (DLS), and a result of the Z-average particle diameter was 8.5 nm (polydispersity index: 0.129).

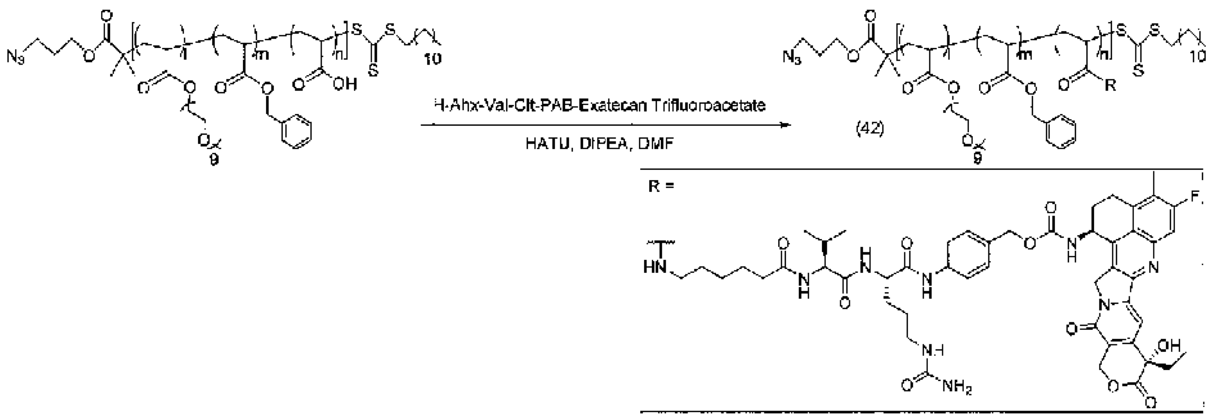


For the exatecan-PAB-Cit-Val-Ahx-bonded copolymer, the number of the exatecan introduced to one molecule of the copolymer was analyzed from a $^1$H-NMR spectrum measured by using the NMR, and a result thereof was 9.4 mol/mol.

[Measurement apparatuses and conditions]

(1) $^1$H-NMR measurement

Apparatus: JNM-ECX400 (400 MHz)/JEOL Ltd.
Solvent: dimethyl sulfoxide-$d_6$ containing 0.03% tetramethylsilane/KANTO CHEMICAL CO., INC.
Sample concentration: 20 mg/mL
Measurement temperature: 25°C
Number of integration times: 399 times
Results: Fig. 21

[Examples 159 to 163]

For the copolymer obtained in Example 158, synthesis was performed by appropriately modifying the charge amount and using the same method as in Example 132. Further, for the copolymers obtained in a similar manner to Examples 72, 79, 82 and 84, synthesis was performed by sequentially performing the same preparation method as in Examples 94, 96 and 132 while appropriately modifying the charge amounts.

[Table 41]

| Example | Copolymer used | Antibody | Drug | Fraction | Number of drugs bonded to one molecule of copolymer (mol/mol) | Number of copolymers bonded to one antibody (mol/mol) | DAR | Z-average particle diameter (nm) | Polydispersity index |
|---|---|---|---|---|---|---|---|---|---|
| 159 | Example 158 | Cetuximab | Exatecan | - | 9.4 | 2.3 | 21 | 13 | 0.13 |
| 160 | Example 82 | Cetuximab | DM1 | - | 12 | 2.1 | 25 | 27 | - |
| 161 | Example 84 | Cetuximab | DM1 | - | 24 | 1.7 | 41 | 27 | - |
| 162 | Example 72 | Cetuximab | DM1 | - | 10 | 2.0 | 20 | 27 | - |
| 163 | Example 79 | Cetuximab | DM1 | - | 24 | 1.8 | 43 | 27 | - |

[Comparative Example 1]

Preparation of oxaliplatin solution

To 5.58 mL of a 5.9 (w/v)% glucose solution was added 1 mL of ELPLAT I.V. infusion solution 50 mg (Yakult Honsha Co., Ltd.) to prepare a 5 (w/v)% glucose solution containing 760 μg of oxaliplatin.

[Comparative Example 2]

Preparation of a cetuximab solution

Cetuximab (Erbitux™ Injection 100 mg, Merck Biopharma Co., Ltd.) was diluted with physiological saline (Otsuka physiological saline solution) to prepare a cetuximab solution at 2 mg/mL, which was used for intravenous administration (dose at 10 mg/kg body). Further, by similarly diluting with RPMI1640 (hereinafter, RPMI1640 medium) containing 10% fetal bovine serum (Merck KGaA), cytotoxic effects were evaluated at final concentrations of cetuximab of 66 nmol/L, 20 nmol/L, 6.6 nmol/L, 2.0 nmol/L, 0.66 nmol/L, 0.20 nmol/L, 0.066 nmol/L, or 0.020 nmol/L.

[Comparative Example 3]

Preparation of a trastuzumab solution

In water for injection (3.0 mL) in accordance with the Japanese Pharmacopoeia, trastuzumab (Herceptin™ for Intravenous Infusion 60, Chugai Pharmaceutical CO., LTD.) (60 mg) was dissolved to prepare a solution at 20 mg/mL. Next, by diluting this solution with the RPMI1640 medium, cytotoxic effects were evaluated at final concentrations of trastuzumab of 20 nmol/L, 6.8 nmol/L, 2.0 nmol/L, 0.68 nmol/L, 0.20 nmol/L, 0.068 nmol/L, 0.020 nmol/L, 0.0068 nmol/L, or 0.0020 nmol/L.

[Comparative Example 4]

Preparation of a rituximab solution

By diluting rituximab (Rituxan™ Intravenous Infusion 100 mg, Zenyaku Kogyo Co., Ltd.) with the RPMI1640 medium, cytotoxic effects were evaluated at final concentration of rituximab of 69 nmol/L, 21 nmol/L, 6.9 nmol/L, 2.1 nmol/L, 0.69 nmol/L, 0.21 nmol/L, 0.069 nmol/L, or 0.021 nmol/L.

[Test example 1] Drug efficacy test

A tumor-bearing model obtained by subcutaneously transplanting a mouse colon cancer cell line C26 (American Type Culture Collection) into a female nude mouse (BALB/c-nu/nu, 7 weeks old; Charles River Laboratories Japan, Inc.) was used for a drug efficacy test.

A mouse colon cancer cell line C26 subcultured in a $CO_2$ incubator was suspended in a liquid medium (Dulbecco's Modified Eagle's Medium-high glucose, Sigma-Aldrich Co. LLC), and injected subcutaneously in the back of nude mice so that the number of cells per mouse was $1 \times 10^6$/100 μL. Thereafter, the nude mice were raised for about 1 week, and administration of a drug was started when the average value of the tumor volume had grown to about 30 $mm^3$. A DACHPt-encapsulating SCNP (DACHPt-encapsulating SCNP prepared using the copolymer of Example 70) was administered into the tail vein (3 times every other day), and the antitumor effect was evaluated based on the tumor volume (4 to 5 mice per 1 group). As a comparison, an oxaliplatin solution (Comparative Example 1) was used, and it was administered in the same manner. The dose of each preparation was 8 mg/kg (3.9 mg/kg in terms of Pt) as the maximum administrable dose for the oxaliplatin solution, and 3 mg/kg in terms of Pt for the DACHPt-encapsulating SCNP.

A change in the tumor volume over time is shown in Fig. 22. For the DACHPt-encapsulating SCNP, T/C was 0.4 after 14 days from administration [T/C: ratio of the tumor volume of the drug administration group (T) to that of the control group (C)]. For the oxaliplatin solution (Comparative Example 1), T/C was 1.1 after 14 days from administration. In addition, after 14 days from administration, it was confirmed that the DACHPt-encapsulating SCNP significantly suppressed the growth of a tumor as compared with the control (Student's t-test). The above results indicate that the DACHPt-encapsulating SCNP has an excellent antitumor effect as compared with the oxaliplatin solution.

[Test Example 2] Cytotoxic test

An EGFR antigen-positive human breast cancer cell line MDA-MB-468 (American Type Culture Collection) and an EGFR antigen-negative human breast cancer cell line MDA-MB-453 (RIKEN BRC CELL BANK) were adjusted to a cell concentration of 2.0 × $10^4$ cells/mL and 4.0 × $10^4$ cells/mL with the RPMI1640 medium, respectively, and 100 μL each was added to a 96-well microplate for cell culture and cultured overnight. On the next day, 100 μL of the evaluation specimen diluted with the RPMI medium was separately added to the microplate, and the cells were cultured for 4 days at 37°C under 5% $CO_2$. After the culturing, CellTiter-Glo™ Luminescent Cell Viability Assay (Promega Corporation) was added to the microplate, and the mixture was stirred and left to stand at room temperature for 10 minutes. The light emission amount was then measured with a plate reader (Molecular Devices, LLC.). The cell viability was calculated by the following equation.

$$\text{Cell viability (\%)} = a/b \times 100$$

In the equation, a represents an average value (n = 3) of light emission amounts of sample wells, and b represents an average value (n = 3) of light emission amounts of sample non-added wells.
An $IC_{50}$ value, which is an agent concentration at which the cell viability is 50%, was calculated with SAS™ 9.4 Software (SAS Institute Japan Ltd.).

[Table 42]

| Example | Fraction | High expression of EGFR | EGFR negative |
|---|---|---|---|
| | | $IC_{50}$(nmol/L) | |
| | | MDA-MB-468 | MDA-MB-453 |
| Comparative example 2 | - | >20 | >66 |
| 134 | 1 | 0.034 | 0.225 |
| | 2 | 0.028 | 0.081 |
| 135 | 1 | 0.147 | 22.1 |
| | 2 | 0.112 | 2.38 |
| 132 | 1 | 0.042 | 4.09 |
| 133 | 1 | 0.047 | 6.52 |
| 136 | - | 0.017 | 4.14 |
| 137 | 1 | 0.014 | 6.56 |
| | 2 | 0.006 | 1.52 |
| | 3 | 0.005 | 0.48 |
| 139 | - | 0.015 | >20 |
| 150 | 1 | 0.014 | 12.4 |
| | 2 | 0.010 | 4.06 |
| | 3 | 0.006 | 3.41 |
| 152 | 1 | 0.070 | 7.16 |
| 159 | - | 0.121 | >20 |

[Test Example 3] Cytotoxic test

A HER2 antigen-positive human gastric cancer cell line NCI-N87 (American Type Culture Collection) or a HER2 antigen-negative human breast cancer cell line MDA-MB-468 was adjusted to a cell concentration of 2.0 × $10^4$ cells/mL with the RPMI1640 medium, and 100 μL each was added to the 96-well microplate for cell culture and cultured overnight. On the next day, 100 μL of the evaluation specimen diluted with the RPMI medium was separately added to the microplate,

and the cells were cultured for 4 days at 37°C under 5% $CO_2$. After the culturing, CellTiter-Glo™ Luminescent Cell Viability Assay (Promega Corporation) was added to the microplate and the mixture was stirred and left to stand at room temperature for 10 minutes. The light emission amount was then measured with a plate reader (Molecular Devices, LLC.). The cell viability and $IC_{50}$ value were calculated.

[Table 43]

| Example | Fraction | High expression of HER2 | HER2 negative |
|---|---|---|---|
| | | $IC_{50}$(nmol/L) | |
| | | NCI-N87 | MDA-MB-468 |
| Comparative example 3 | - | >20 | >20 |
| 144 | 3 | 0.10 | 3.8 |
| 145 | - | 4.60 | - |
| 146 | - | 0.38 | 2.3 |
| 147 | - | 2.30 | >20 |

[Test Example 4] Cytotoxic test

CD20 antigen-positive human B-cell lymphoma-derived Raji cells or CD20 antigen-negative human T-lymphoblastic leukemia-derived MOLT-4 cells were adjusted to a cell concentration of $5.0 \times 10^4$ cells/mL with the RPMI1640 medium, and 50 μL each was added to the 96-well microplate for cell culture, then cultured overnight. On the next day, 50 μL of the evaluation specimen diluted with the RPMI medium was separately added to the microplate, and the cells were cultured for 4 days at 37°C under 5% $CO_2$. After the culturing, CellTiter-Glo™ Luminescent Cell Viability Assay (Promega Corporation) was added to the microplate, and the mixture was stirred and left to stand at room temperature for 10 minutes. The light emission amount was then measured with a plate reader (Molecular Devices, LLC.). The cell viability and $IC_{50}$ value were calculated.

[Table 44]

| Example | Fraction | High expression of CD20 | CD20 negative |
|---|---|---|---|
| | | $IC_{50}$(nmol/L) | |
| | | Raji | MOLT-4 |
| Comparative example 4 | - | >69 | >69 |
| 148 | 1 | 1.0 | 13 |
| | 2 | 0.50 | 6.9 |
| 149 | 1 | 0.60 | 20 |
| | 2 | 0.41 | 9.9 |

[Test Example 5] Test for confirming antitumor effects

An EGFR-positive human colon cancer cell line HT-29 was suspended in PBS and adjusted to a concentration of $2.5 \times 10^7$ cells/mL. Then, $5 \times 10^6$ cells (0.2 mL) were transplanted subcutaneously to the right ventral portions of nude mice (female, 6 weeks old) after being quarantined, and the mice were randomly divided into groups (12 mice per group) 8 days after the cell subcutaneous transplantation. The cetuximab-bonded micelle drug complex (Example 138, DAR = 38) was administered through the tail veins of the mice at doses of 1.8 and 5.5 mg/kg (dose to the antibody moiety: 1.0 and 3.0 mg Antibody/kg, and the dose to the DM1 moiety: 0.18 and 0.55 mg DM1/kg) or the DM1-bonded micelle (Example 120, unbonded antibody) was administered through the tail veins of the mice at a dose of 2.5 mg/kg (amount of the DM1 moiety: 0.55 mg DM1/kg), respectively. A physiological saline administration group was set as a control group. Results are shown in Fig. 23. The evaluation specimen showed antitumor effects depending on the doses. Further, no weight loss of the mice by the administration was confirmed.

[Test Example 6] Test for confirming antitumor effects

A HER2-positive human gastric cancer cell line NCI-N87 was suspended in PBS to prepare a concentration of 5 $\times$ $10^7$ cells/mL, and 1 $\times$ $10^7$ cells (0.2 mL) were transplanted subcutaneously to the right ventral portions of nude mice (female, 6 weeks old; Charles River Laboratories Japan, Inc.) .

7 days after the cell subcutaneous transplantation, the mice were randomly divided into groups (12 mice per group). The trastuzumab-bonded micelle drug complex (Example 151, DAR = 40) was administered through the tail veins of the mice at doses of 1.9 and 5.7 mg/kg (dose to the antibody moiety: 1.0 and 3.0 mg Antibody/kg, and the dose to the DM1 moiety: 0.20 and 0.60 mg DM1/kg) or the DM1-bonded micelle (Example 120, unbonded antibody) was administered through the tail veins of the mice at a dose of 0.70 mg/kg (dose to the DM1 moiety: 0.20 mg DM1/kg), respectively. A physiological saline administration group was set as a control group. Results are shown in Fig. 24. The evaluation specimen showed antitumor effects depending on the doses. Further, no weight loss of the mice by the administration was confirmed.

[Test Example 7] Test for confirming antitumor effects

An EGFR-positive human breast cancer cell line MDA-MB-468 was suspended in PBS and adjusted to a concentration of 5 $\times$ $10^7$ cells/mL, and 1 $\times$ $10^7$ cells (0.2 mL) were subcutaneously transplanted to right ventral portions of nude mice (female, 6 weeks old; Jackson Laboratory Japan, Inc.).

27 days after the cell subcutaneous transplantation, the mice were randomly divided into groups (10 mice per group). The cetuximab-bonded micelle drug complex (Example 160, DAR = 25) was administered through the tail veins of the mice at a dose of 3.0 mg/kg (dose to the antibody moiety: 1.6 mg Antibody/kg, and the dose to the DM1 moiety: 0.20 mg DM1/kg) or the cetuximab-bonded micelle drug complex (Example 161, DAR = 41) was administered through the tail veins of the mice at a dose of 1.8 mg/kg (dose to the antibody moiety: 1.0 mg Antibody/kg, and the dose to the DM1 moiety: 0.20 mg DM1/kg), respectively. A physiological saline administration group was set as a control group. Results are shown in Fig. 25. The evaluation specimen showed antitumor effects. Further, no weight loss of the mice by the administration was confirmed.

[Test Example 8] Test for confirming antitumor effects

An EGFR-positive human colon cancer cell line HCT-116 having the KRAS mutation (G13D) was suspended in PBS to prepare a concentration of 5 $\times$ $10^7$ cells/mL, and 1 $\times$ $10^7$ cells (0.2 mL) were transplanted subcutaneously to the right ventral portions of nude mice (female, 6 weeks old; Jackson Laboratory Japan, Inc.).

7 days after the cell subcutaneous transplantation, the mice were randomly divided into groups (10 mice per group). The cetuximab-bonded micelle drug complex (Example 162, DAR = 20) was administered through the tail veins the mice at a dose of 5.5 mg/kg (dose to the antibody moiety: 3.1 mg Antibody/kg, and the dose to the DM1 moiety: 0.30 mg DM1/kg) or the cetuximab-bonded micelle drug complex (Example 163, DAR = 43) was administered through the tail veins of the mice at a dose of 2.6 mg/kg (dose to the antibody moiety: 1.4 mg Antibody/kg, and the dose to the DM1 moiety: 0.30 mg DM1/kg), respectively. A physiological saline administration group was set as a control group. Results are shown in Fig. 26. The evaluation specimen showed antitumor effects. Further, no weight loss of the mice by the administration was confirmed.

Long diameters and short diameters of the tumors were measured twice a week with a digital caliper (product number: 19975, Shinwa Rules Co., Ltd.), and the tumor volumes were calculated by the following equation.

$$\text{Tumor volume (mm}^3\text{)} = a \times b^2/2$$

In the equation, a represents the long diameter (mm), and b represents the short diameter (mm).

## Claims

1. A copolymer in which a target recognition molecule is bonded to a copolymer X comprising structural units represented by the following formulas (A), (B), and (C),

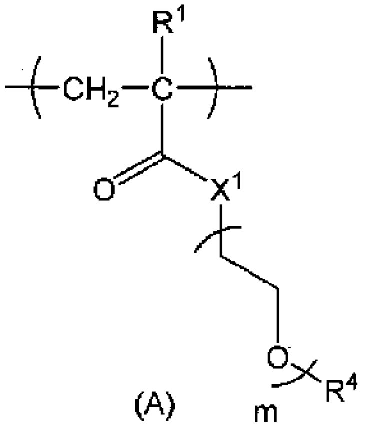

(A) ,

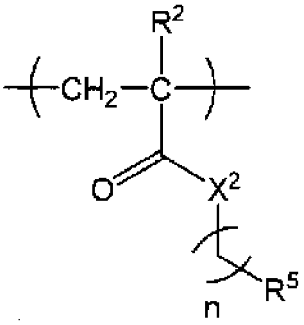

(B) ,

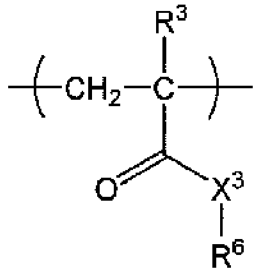

(C)

wherein, $R^1$, $R^2$, and $R^3$ are the same or different and represent a hydrogen atom or a $C_{1-3}$ alkyl group, $R^4$ represents a $C_{1-3}$ alkyl group, $R^5$ represents a hydrogen atom, a $C_{1-18}$ alkyl group, a 3- to 8- membered cycloalkyl group optionally having a substituent, an adamantyl group, a $C_{6-18}$ aryl group optionally having a substituent, or a 5-to 10- membered heteroaryl group optionally having a substituent, $X^1$, $X^2$, and $X^3$ are the same or different and represent an oxygen atom, a sulfur atom, or N-$R^7$, $R^6$ represents a hydrogen atom, a leaving group, or a linker, $R^7$ represents a hydrogen atom or a $C_{1-3}$ alkyl group, m represents an integer of 1 to 100, and n represents an integer of 0 to 3.

2. The copolymer according to claim 1, wherein the polymer X is a copolymer formed by polymerization of three types of monomers represented by the following general formulas (1) to (3),

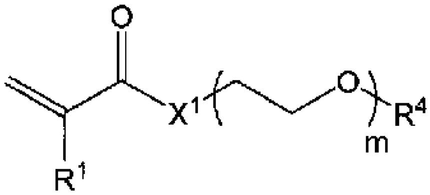

(1) ,

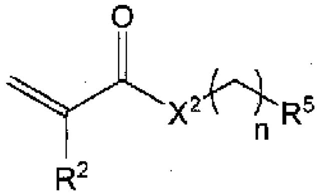

(2) ,

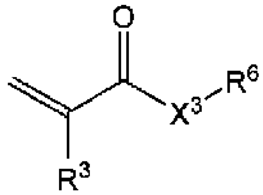

(3)

wherein, $R^1$, $R^2$, and $R^3$ are the same or different and represent a hydrogen atom or a $C_{1-3}$ alkyl group, $R^4$ represents a $C_{1-3}$ alkyl group, $R^5$ represents a hydrogen atom, a $C_{1-18}$ alkyl group, a 3- to 8-membered cycloalkyl group optionally having a substituent, an adamantyl group, a $C_{6-18}$ aryl group optionally having a substituent, or a 5-to 10-membered heteroaryl group optionally having a substituent, $X^1$, $X^2$, and $X^3$ are the same or different and represent an oxygen atom, a sulfur atom, or N-$R^7$, $R^6$ represents a hydrogen atom, a leaving group, or a linker, $R^7$ represents a hydrogen atom or a $C_{1-3}$ alkyl group, m represents an integer of 1 to 100, and n represents an integer of 0 to 3.

3. The copolymer according to claim 1 or 2, wherein $R^1$ is a hydrogen atom.

4. The copolymer according to claim 1 or 2, wherein $R^2$ is a hydrogen atom.

5. The copolymer according to claim 1 or 2, wherein $R^3$ is a hydrogen atom.

6. The copolymer according to claim 1 or 2, wherein $R^4$ is a methyl group.

7. The copolymer according to claim 1 or 2, wherein $R^5$ is a $C_{6-18}$ aryl group optionally having a substituent.

8. The copolymer according to claim 1 or 2, wherein $R^5$ is a phenyl group.

9. The copolymer according to claim 1 or 2, wherein $R^6$ is a hydrogen atom.

10. The copolymer according to claim 1 or 2, wherein the leaving group of $R^6$ is a group represented by the following formula (4).

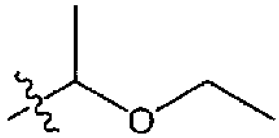

(4)

11. The copolymer according to claim 1 or 2, wherein the linker of $R^6$ is a group represented by the following formula (5):

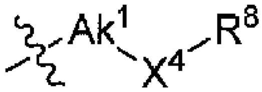

(5)

wherein, $R^8$ represents a hydrogen atom or a drug, $Ak^1$ represents a $C_{1-7}$ alkylene bond, and $X^4$ represents an oxygen atom, a sulfur atom, or $-N(R^7)-$, in which $R^7$ represents a hydrogen atom or a $C_{1-3}$ alkyl group.

12. The copolymer according to claim 1 or 2, wherein $X^1$ is an oxygen atom.

13. The copolymer according to claim 1 or 2, wherein $X^2$ is an oxygen atom.

14. The copolymer according to claim 1 or 2, wherein $X^3$ is an oxygen atom or NH.

15. The copolymer according to claim 1 or 2, wherein m is an integer of 4 to 22.

16. The copolymer according to claim 1 or 2, wherein n is 1.

17. The copolymer according to claim 1 or 2, wherein a ratio of structural units (A), (B), and (C) is composed of 0.01 to 100 parts by mass of (B) and 0.1 to 100 parts by mass of (C) with respect to 1 part by mass of (A).

18. The copolymer according to claim 2, wherein 0.01 to 100 parts by mass of monomer (2) and 0.1 to 100 parts by mass of monomer (3) are polymerized with respect to 1 part by mass of monomer (1).

19. The copolymer according to claim 1 or 2, wherein the copolymer has a number average molecular weight of 5 000 to 150 000.

20. The copolymer according to claim 1 or 2, wherein the target recognition molecule is an antibody.

21. The copolymer according to claim 20, wherein the antibody is an anti-EGFR antibody, an anti-Her2 antibody, an anti-CD20 antibody, an anti-CD276 antibody, an anti-MUC1 antibody, an anti-PD-L1 antibody, or an anti-TROP-2 antibody.

22. The copolymer according to claim 20, wherein the antibody is cetuximab, panitumumab, necitumumab, amivantamab, panitumumab, trastuzumab, pertuzumab, margetuximab, rituximab, ibritumomab, tositumomab, ofatumumab, obinutuzumab, clivatuzumab, gatipotuzumab, ifinatamab, mirzotamab, vobramitamab, atezolizumab, avelumab, durvalumab, sacituzumab, or functional fragments thereof.

23. A drug complex comprising the copolymer according to claim 1 or 2, and a drug.

24. The drug complex according to claim 23, wherein the drug is an antimetabolite, an alkylating agent, an anthracycline, an antibiotic, a mitotic inhibitor, a topoisomerase inhibitor, a proteasome inhibitor, or an anti-hormone agent.

25. The drug complex according to claim 23, wherein the drug is DM0, DM1, DM2, DM3, DM4, emtansine, auristatin E, auristatin phenylalanine phenylenediamine (AFP), monomethyl auristatin E, monomethyl auristatin D, monomethyl auristatin F, paclitaxel, docetaxel, irinotecan, topotecan, nogitecan, amsacrine, etoposide, teniposide, mizantrone, SN-38, exatecan, or deruxtecan.

**26.** The drug complex according to any one of claims 23 to 25, wherein the bond of the target recognition molecule or the drug to the copolymer X is a covalent bond or a noncovalent bond.

**27.** The drug complex according to any one of claims 23 to 26, wherein the bond of the target recognition molecule or the drug to the copolymer X is represented by the following formula (a):

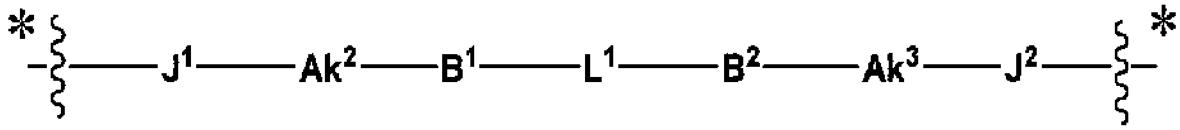

(a)

wherein, $J^1$ is a bond to the target recognition molecule or the drug, $J^2$ is a bond to the copolymer X, $Ak^2$ and $Ak^3$ each independently represent a single bond or a $C_{1-7}$ alkylene bond, $B^1$ and $B^2$ each independently represent a single bond, an amide bond, or an ester bond, $L^1$ represents a single bond, $-(CH_2CH_2O)_oCH_2CH_2-$, phenylene, cyclohexylene, -NH-peptide-CO-, or phenylene-NH-peptide-CO-, and o represents an integer of 0 to 100.

**28.** A single chain nanoparticle comprising the copolymer according to claim 1 or 2.

**29.** A single chain nanoparticle comprising the drug complex according to claim 23 or 24.

**30.** A pharmaceutical composition comprising the copolymer according to claim 1 or 2.

**31.** A pharmaceutical composition comprising the drug complex according to claim 23 or 24.

FIG. 1

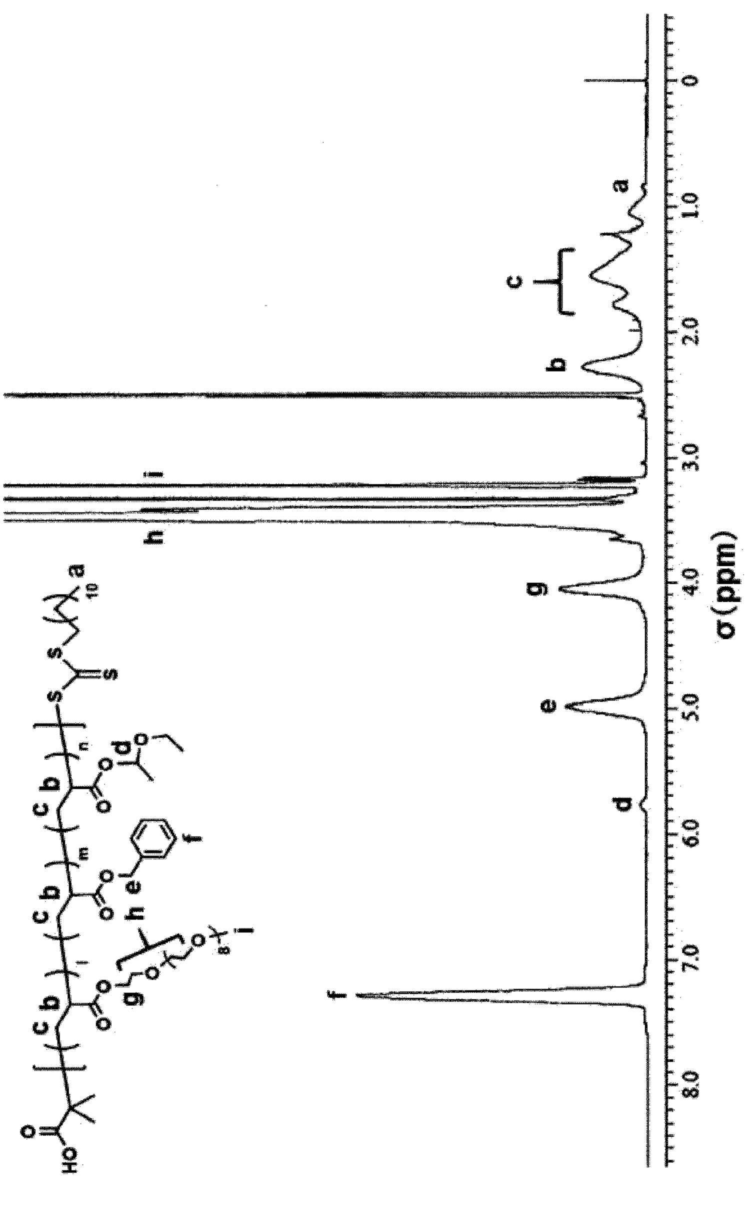

FIG. 2

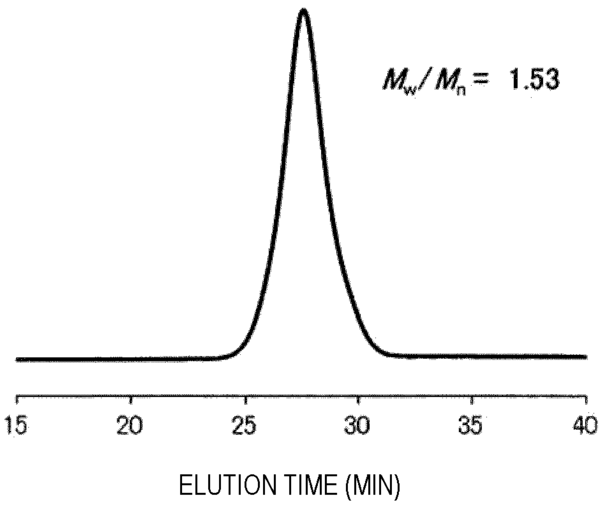
$M_w/M_n$ = 1.53
15
20
25
30
35
40
ELUTION TIME (MIN)

FIG. 3

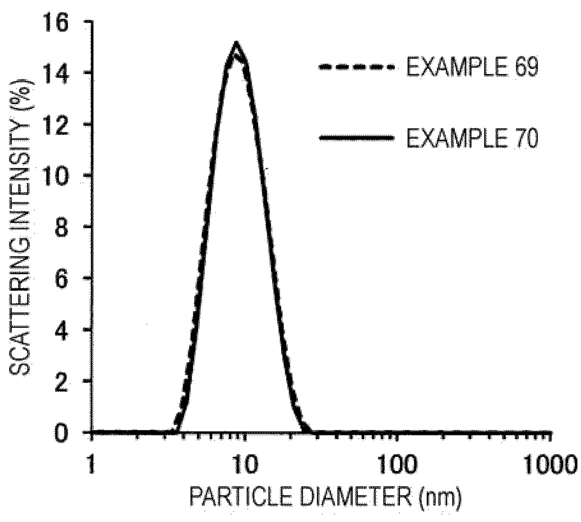
16
14
12
10
8
6
4
2
0
SCATTERING INTENSITY (%)
EXAMPLE 69
EXAMPLE 70
1
10
100
1000
PARTICLE DIAMETER (nm)

FIG. 4

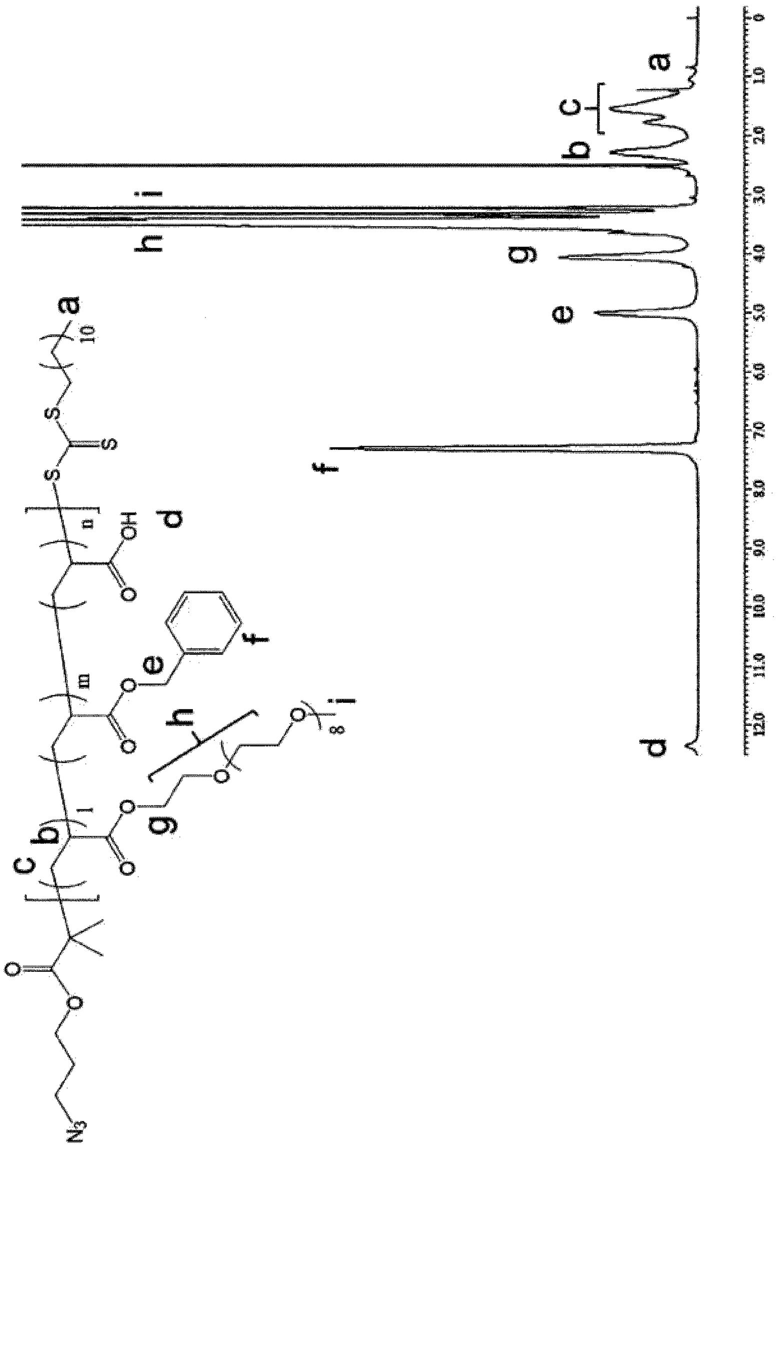

FIG. 5

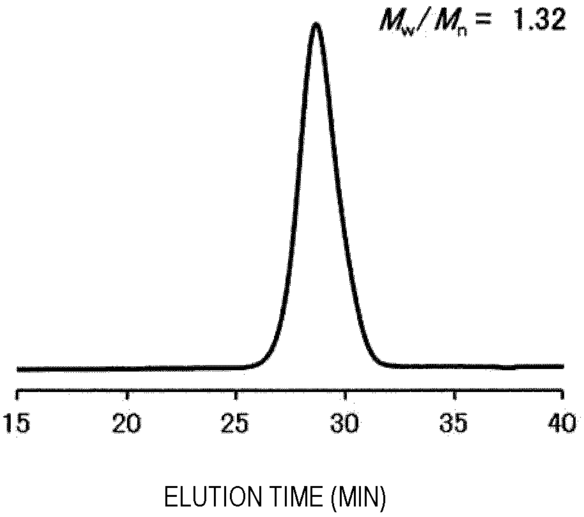
$M_w/M_n$ = 1.32
15
20
25
30
35
40
ELUTION TIME (MIN)

FIG. 6

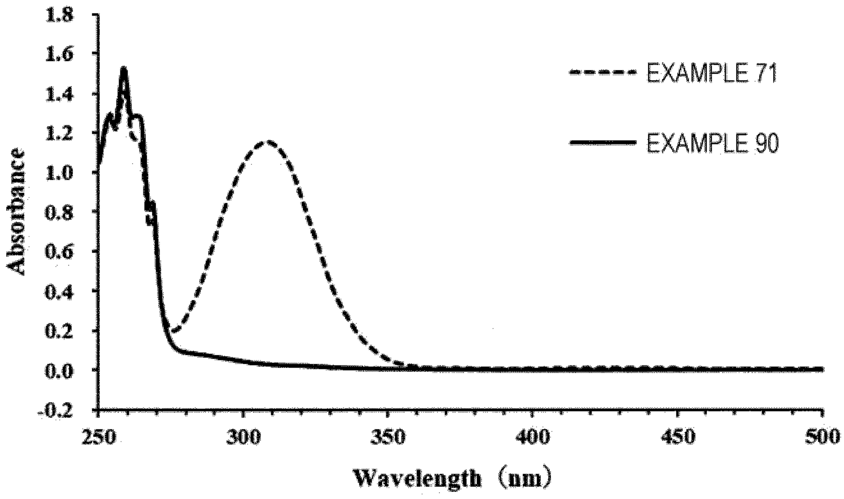
1.8
1.6
1.4
1.2
1.0
0.8
0.6
0.4
0.2
0.0
-0.2
Absorbance
EXAMPLE 71
EXAMPLE 90
250
300
350
400
450
500
Wavelength (nm)

FIG. 7

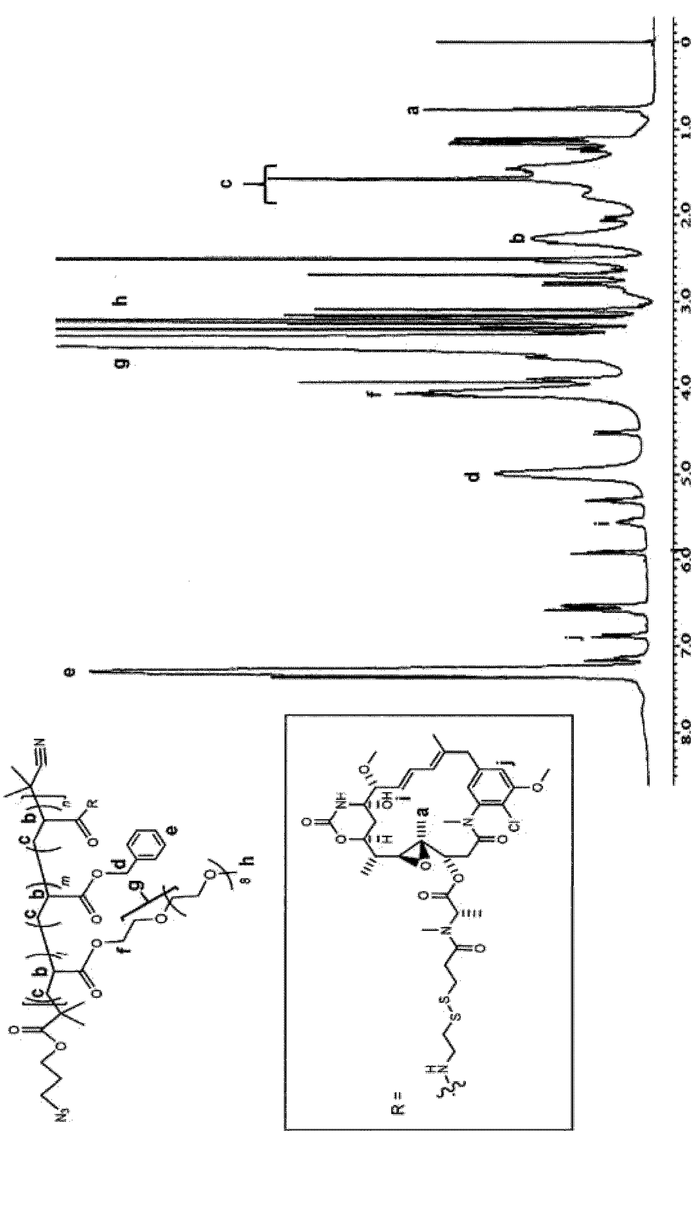

*FIG. 8*

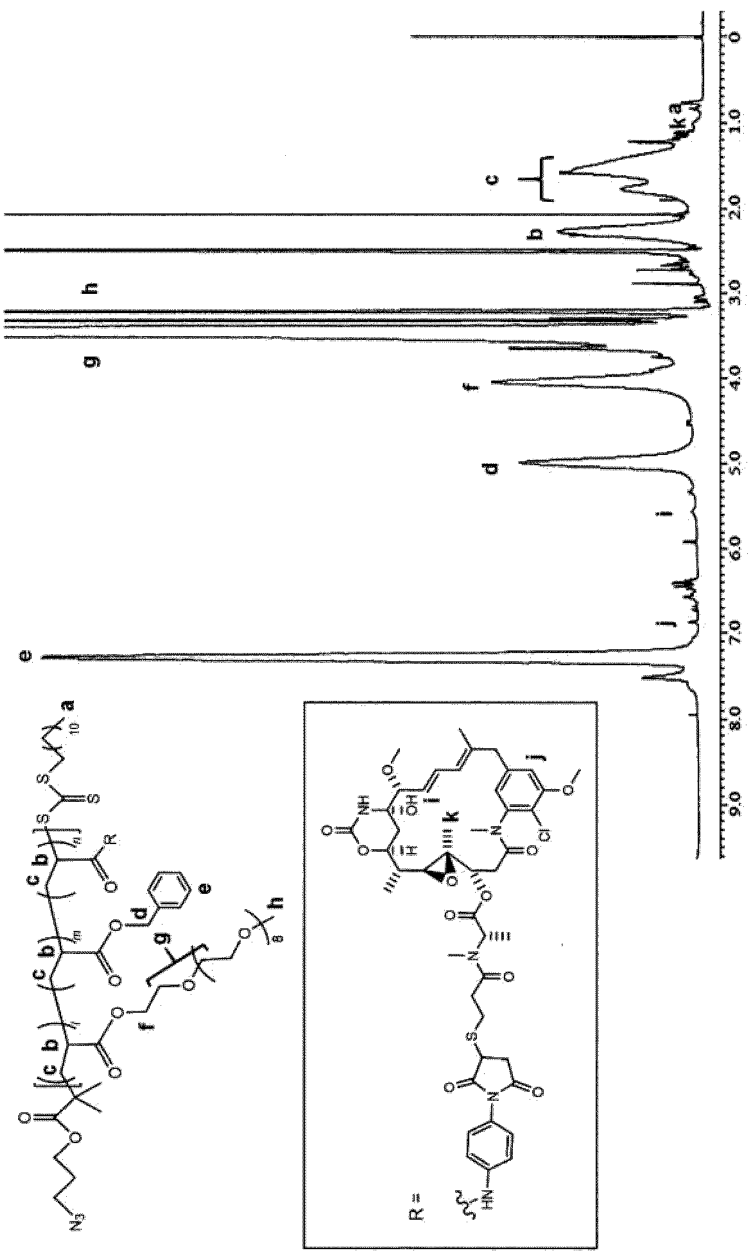

FIG. 9

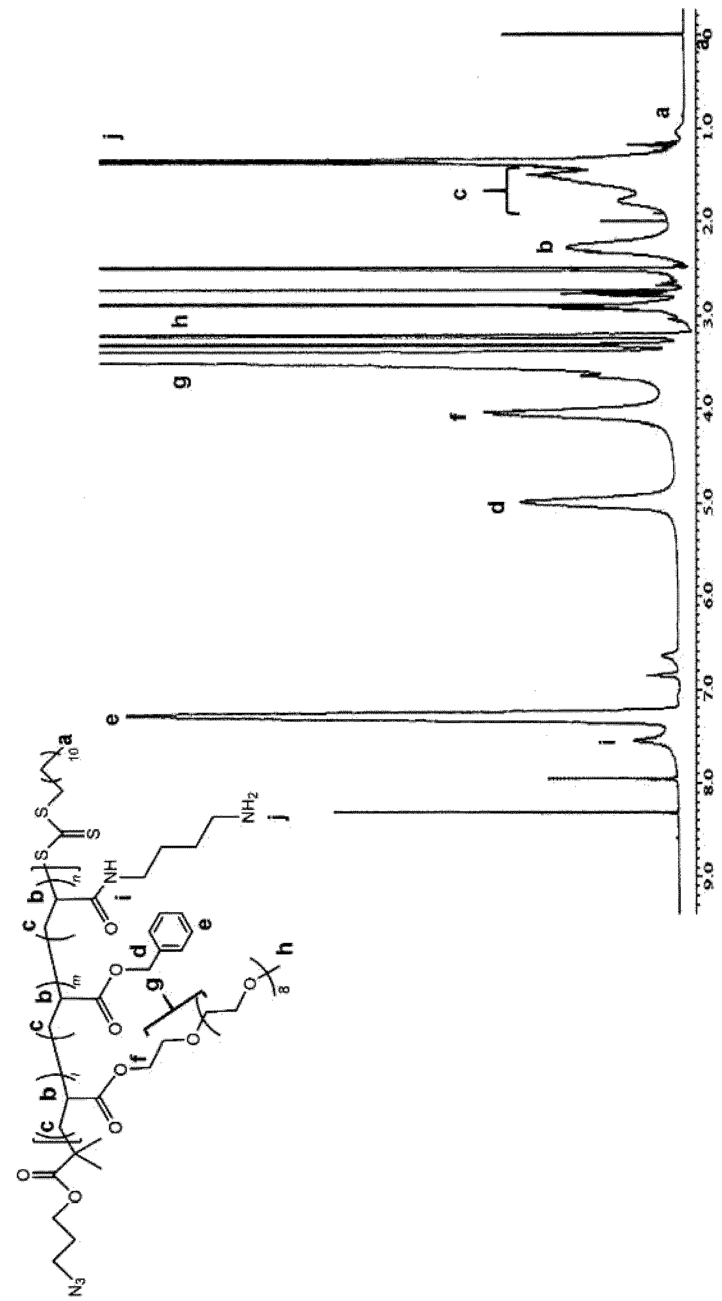

FIG. 10

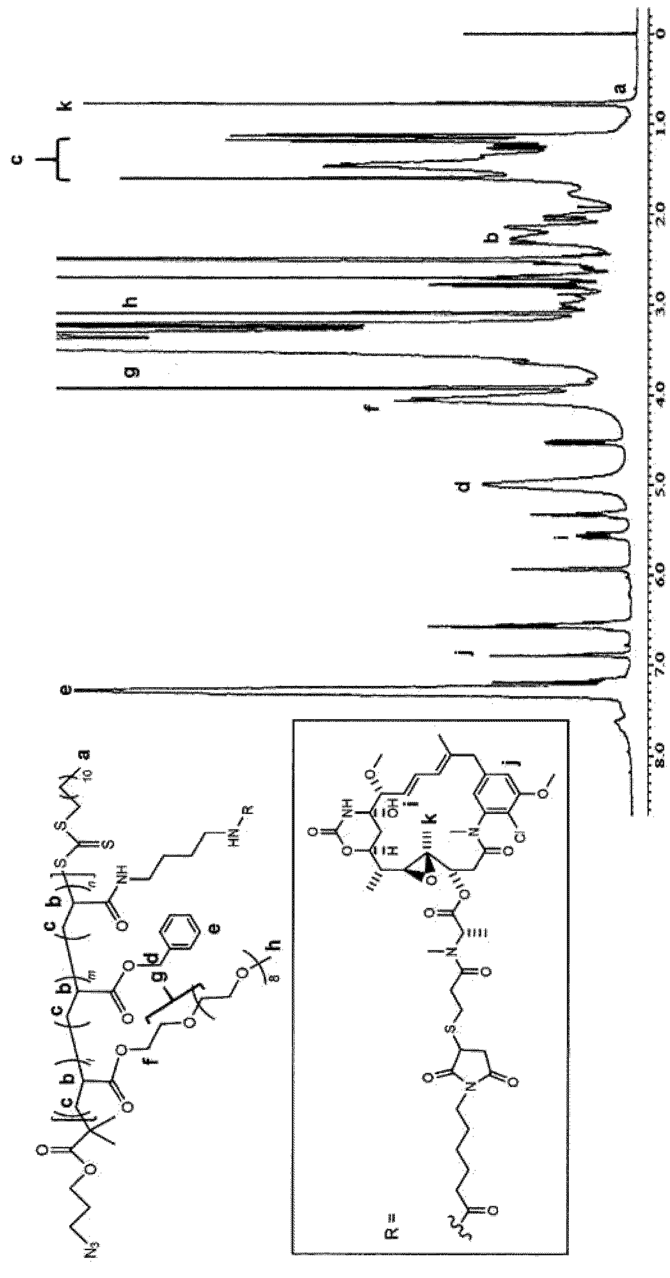

FIG. 11

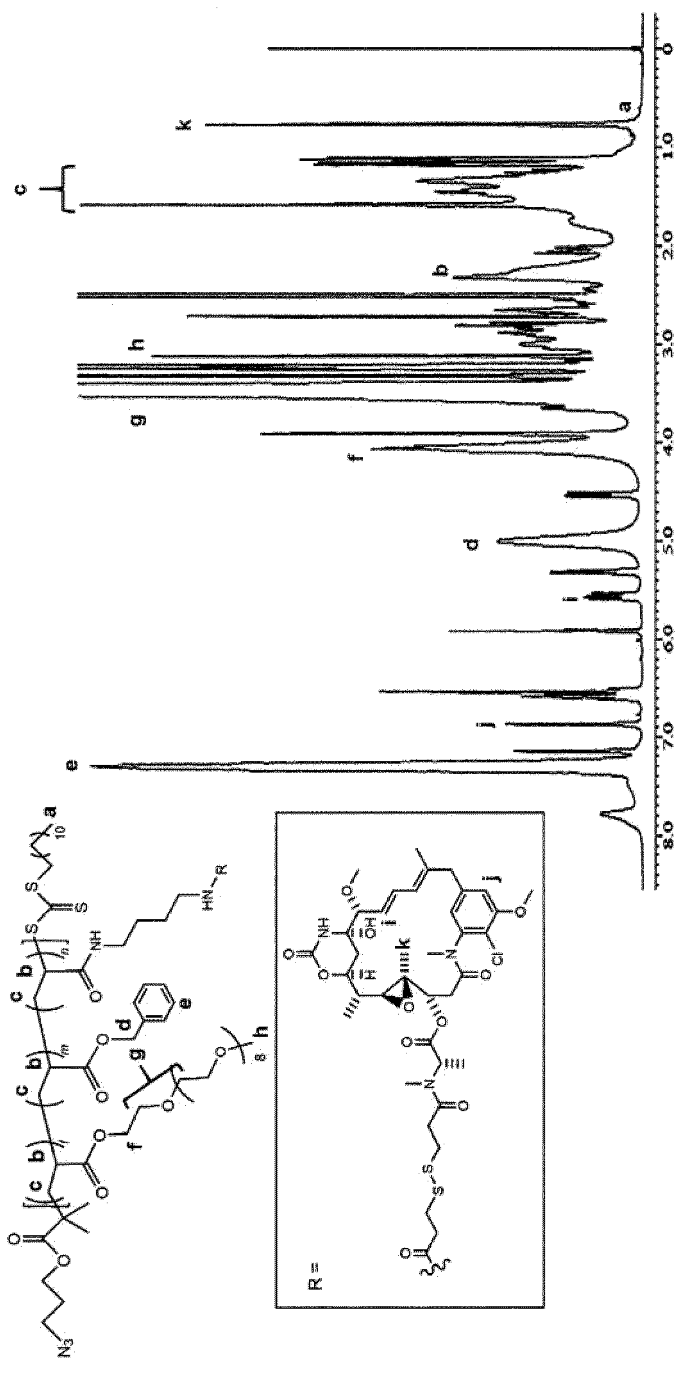

FIG. 12

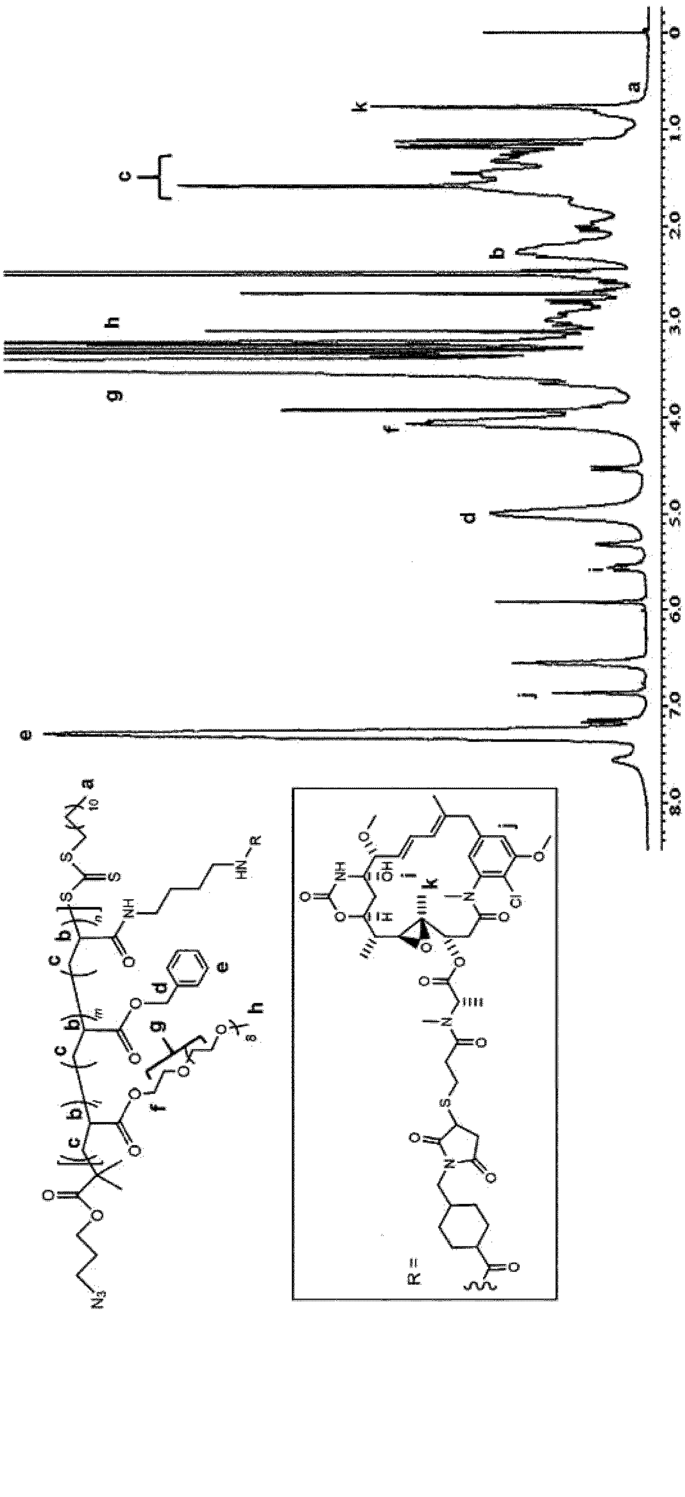

FIG. 13

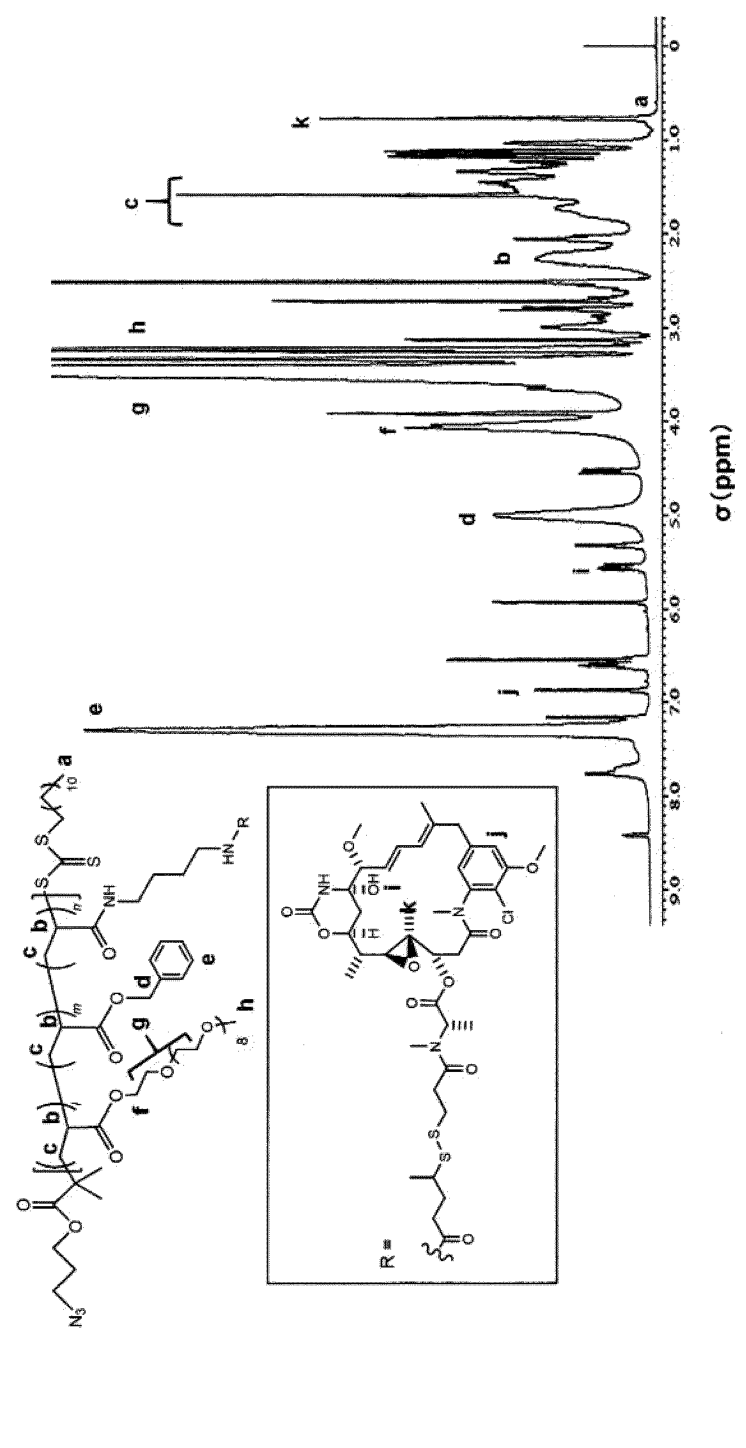

FIG. 14

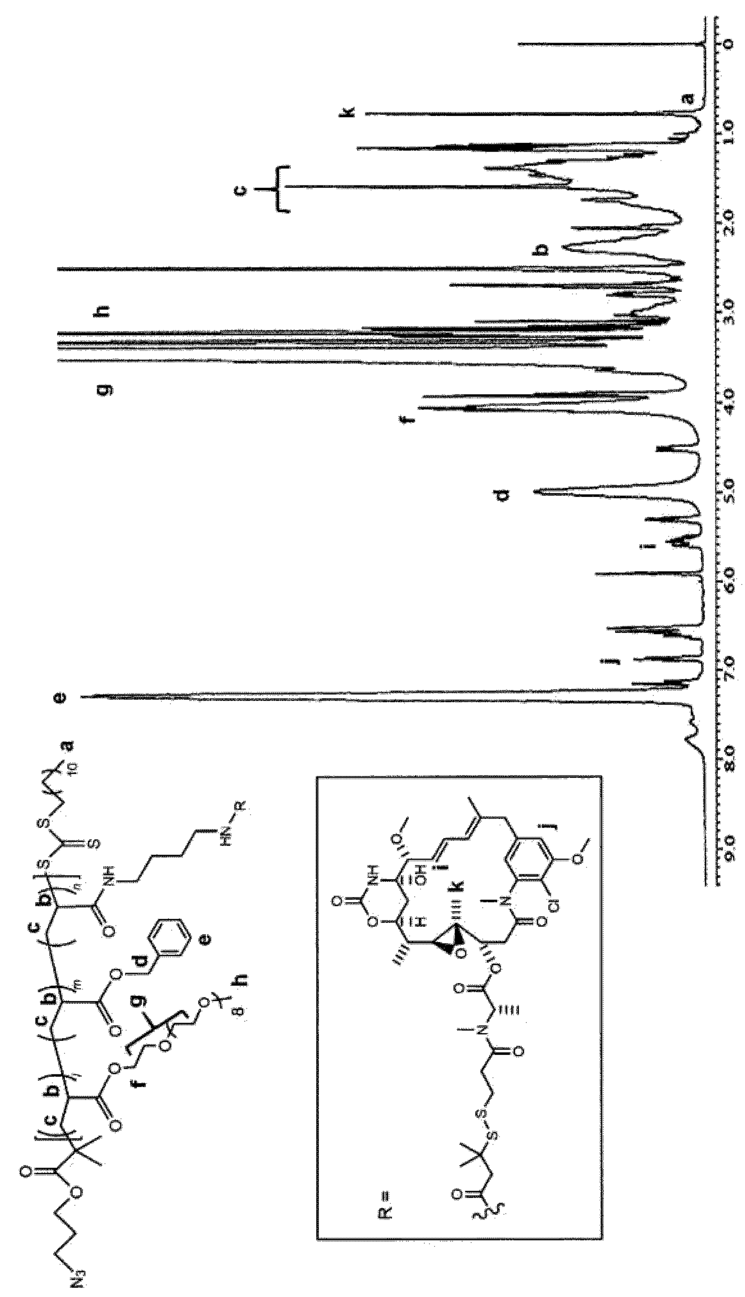

FIG. 15

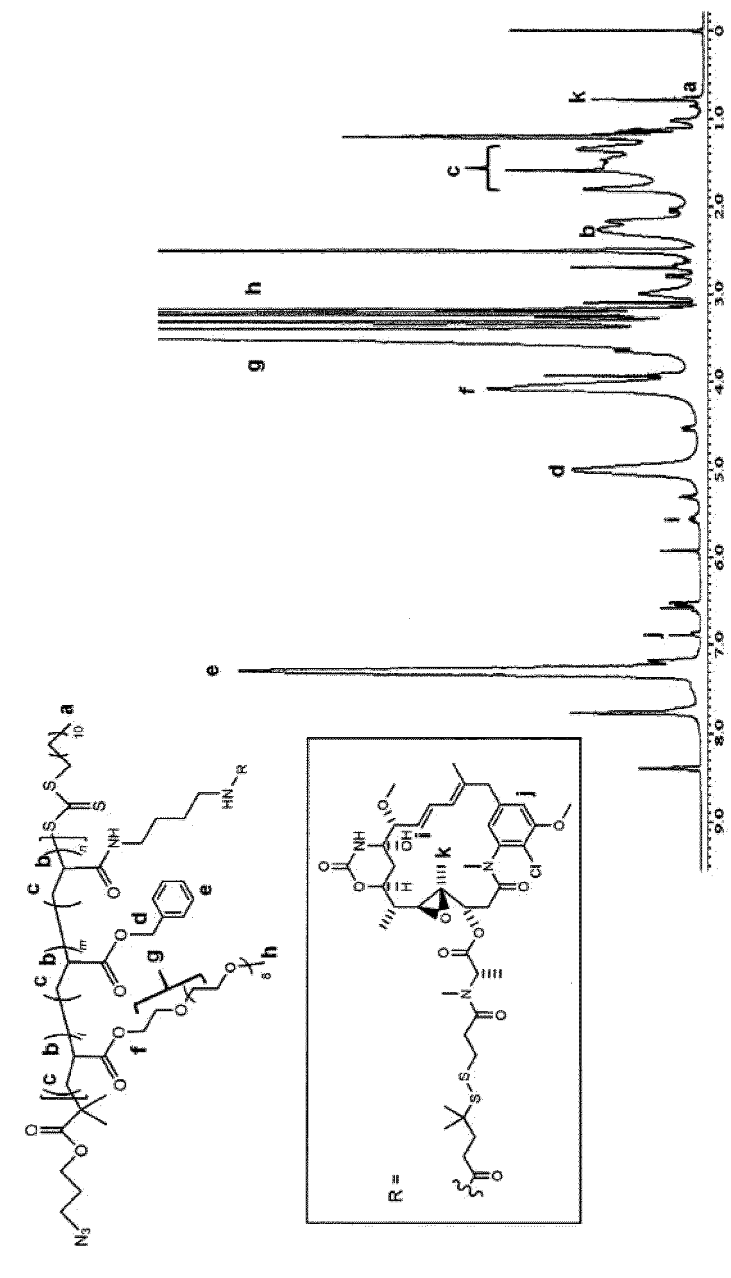

*FIG. 16*

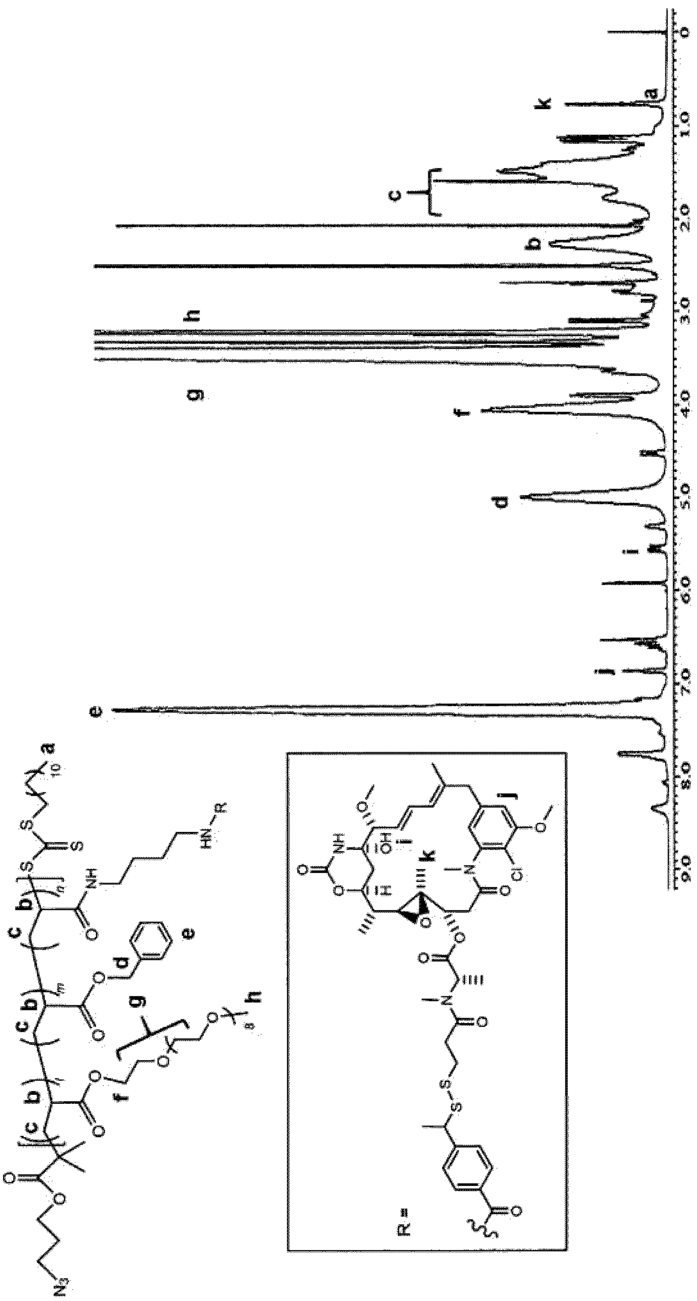

FIG. 17

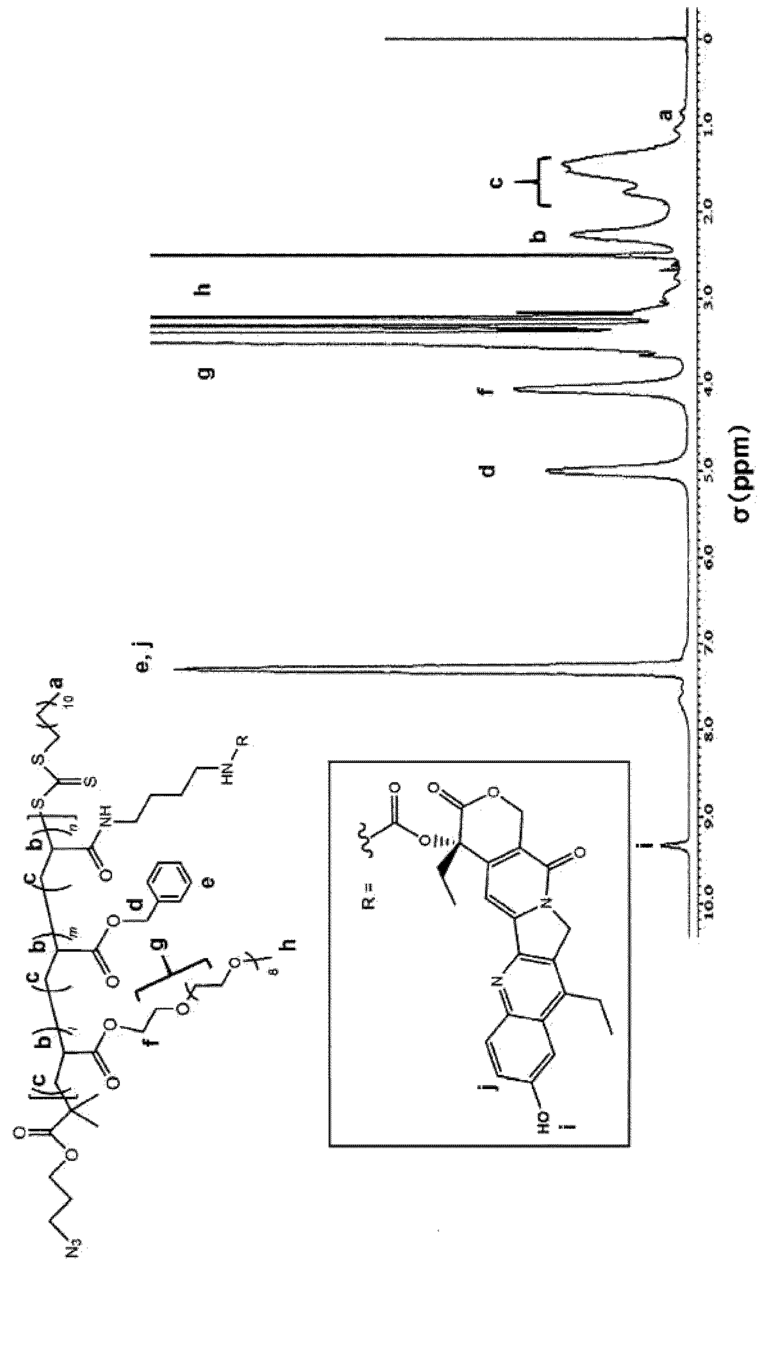

FIG. 18

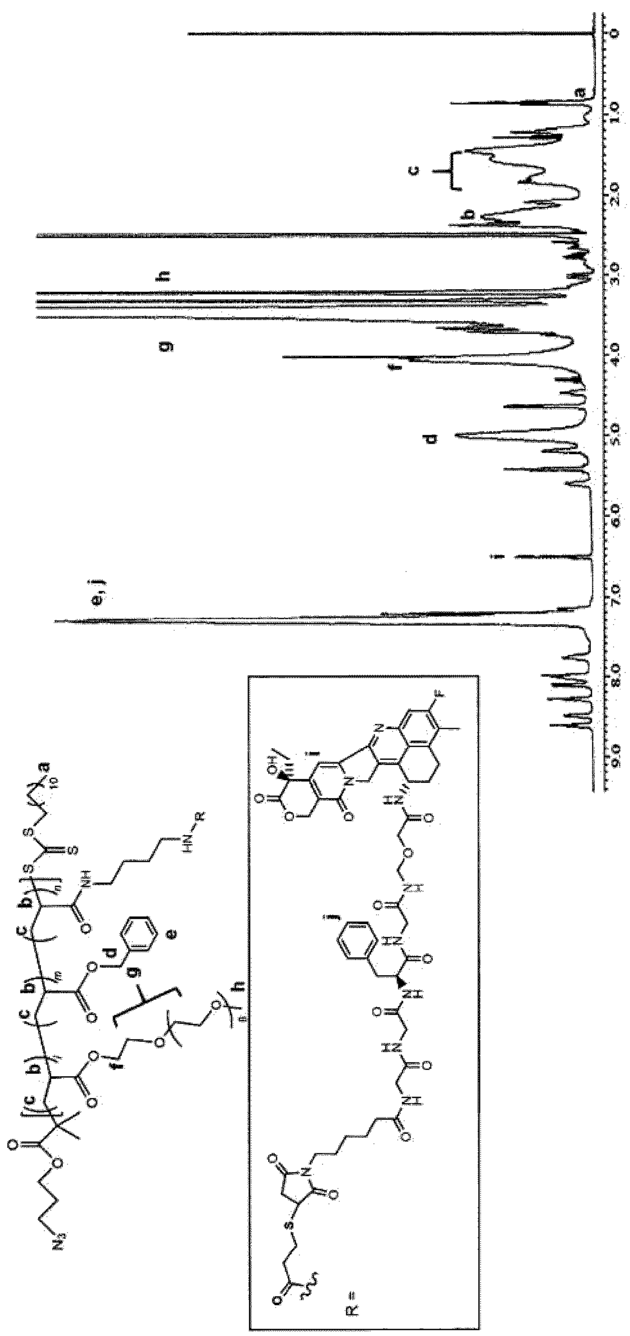

FIG. 19

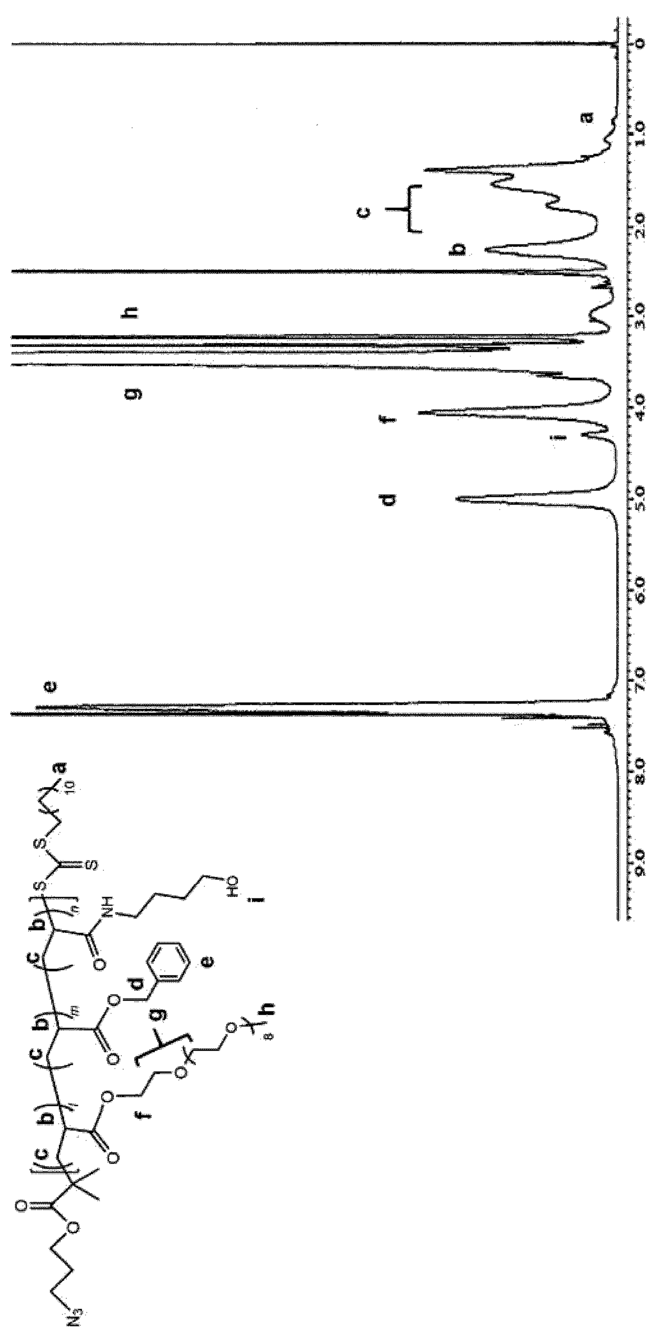

FIG. 20

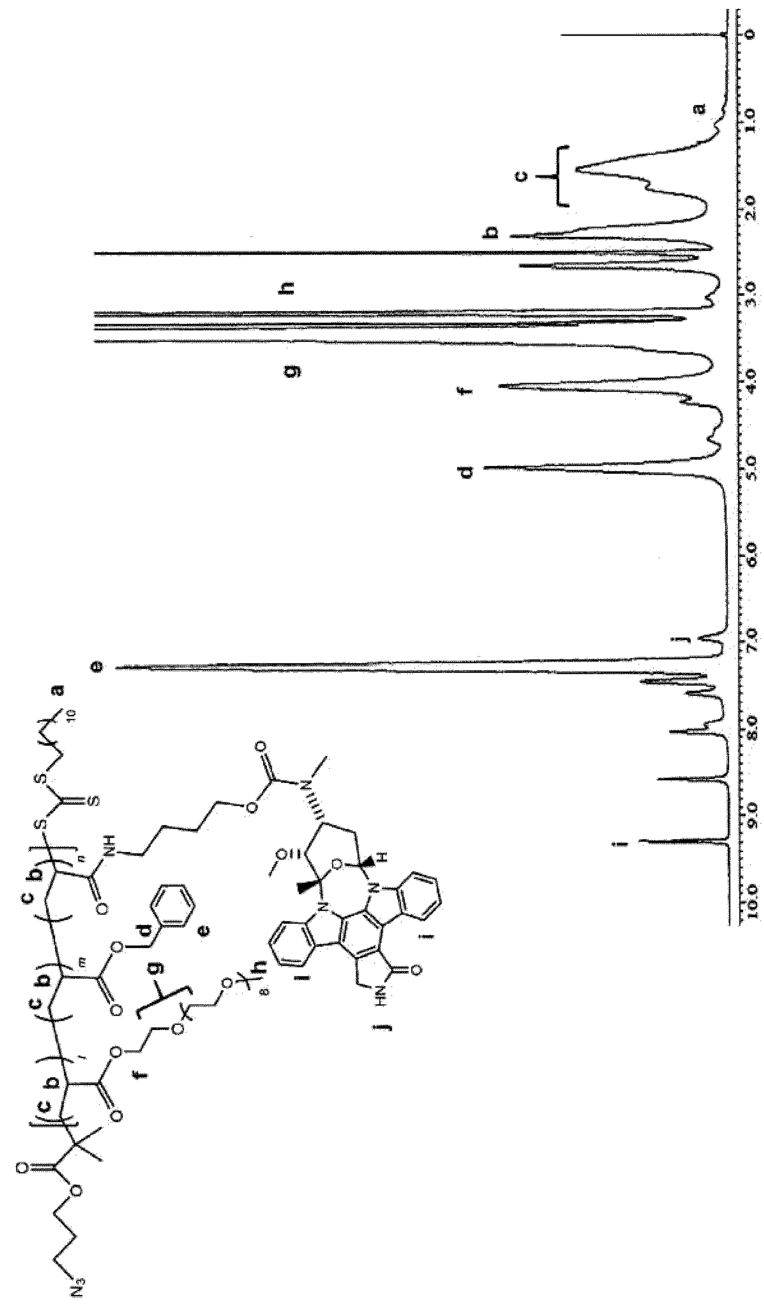

FIG. 21

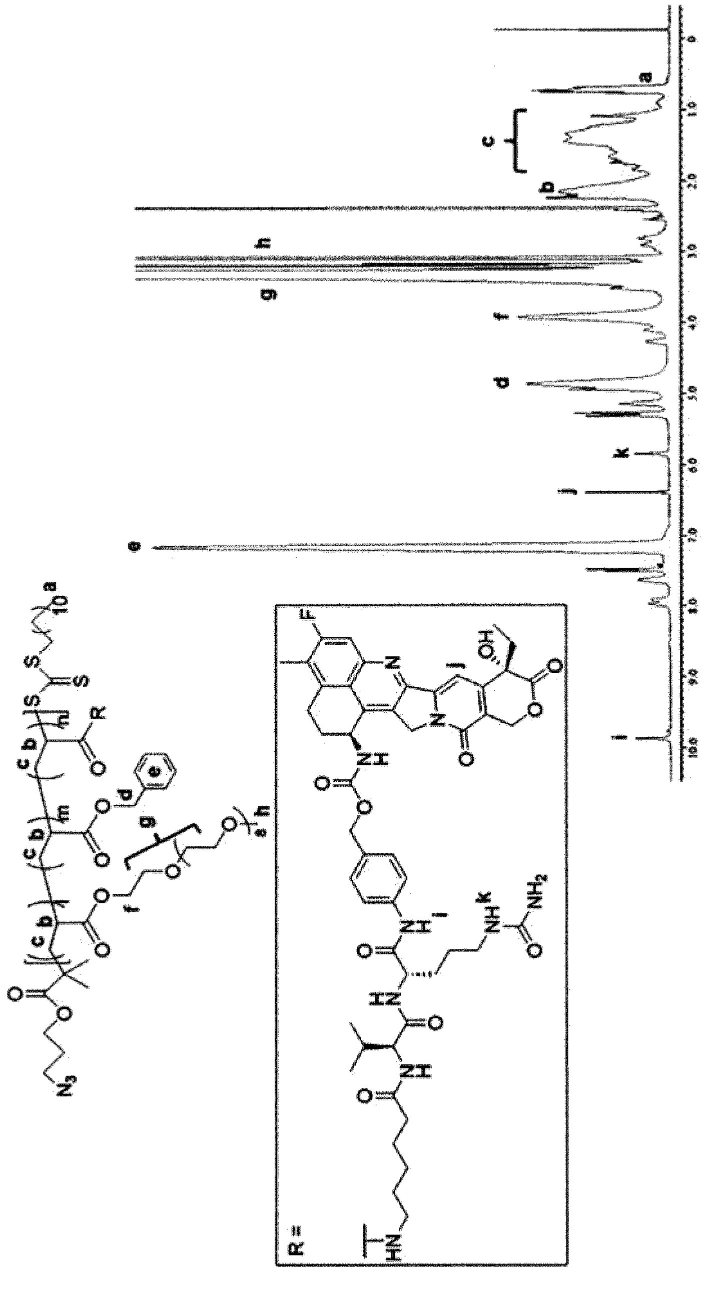

FIG. 22

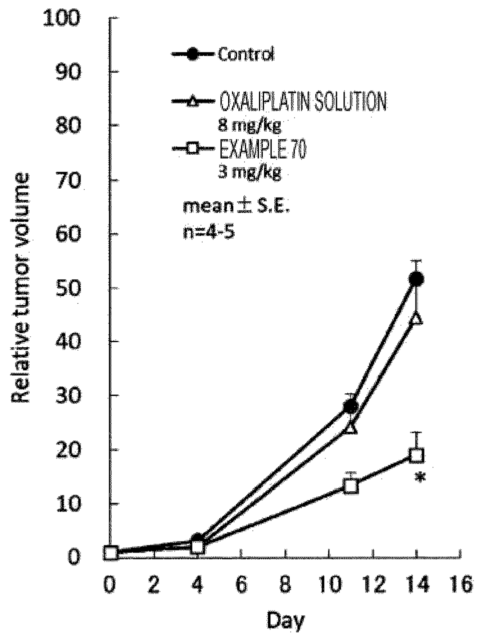


*; P < 0.05 (student' s t-test) vs. Control

FIG. 23

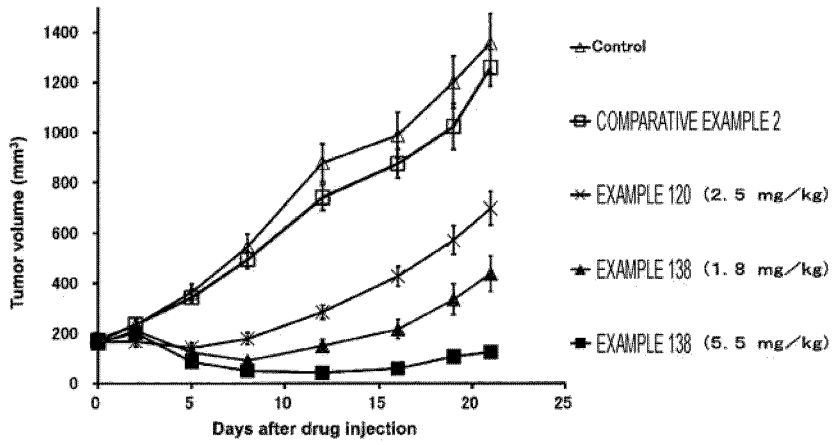

FIG. 24

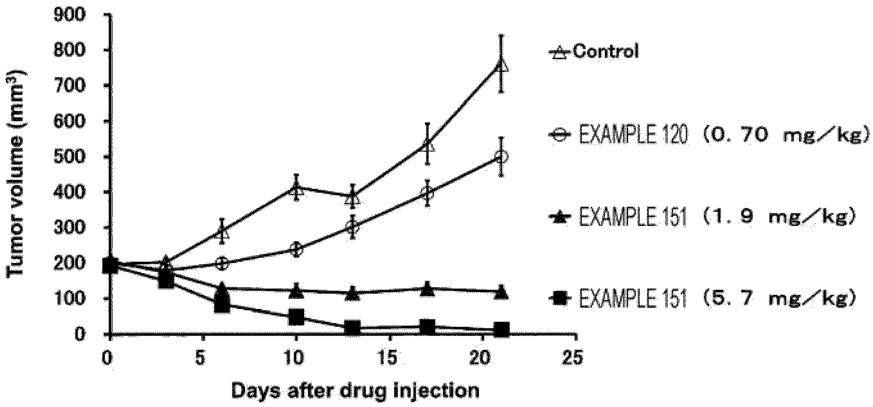
900
800
700
600
500
400
300
200
100
0
Tumor volume (mm³)
0
5
10
15
20
25
Days after drug injection
Control
EXAMPLE 120 (0. 70 mg/kg)
EXAMPLE 151 (1. 9 mg/kg)
EXAMPLE 151 (5. 7 mg/kg)

FIG. 25

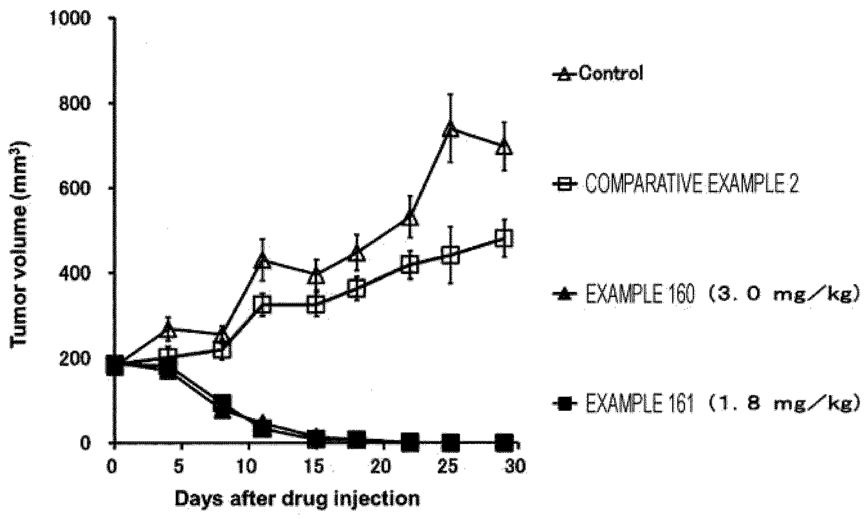
1000
800
600
400
200
0
Tumor volume (mm³)
0
5
10
15
20
25
30
Days after drug injection
Control
COMPARATIVE EXAMPLE 2
EXAMPLE 160 (3. 0 mg/kg)
EXAMPLE 161 (1. 8 mg/kg)

FIG. 26

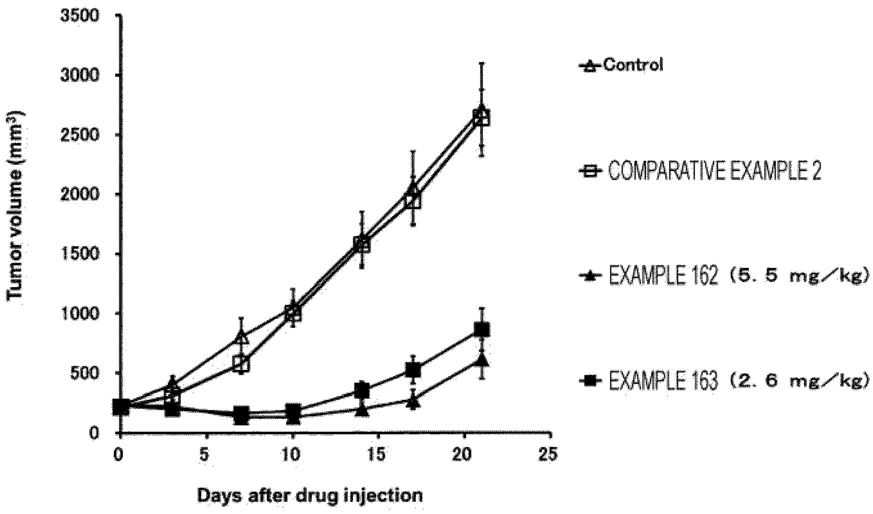
Tumor volume (mm³)
3500
3000
2500
2000
1500
1000
500
0
0
5
10
15
20
25
Days after drug injection
Control
COMPARATIVE EXAMPLE 2
EXAMPLE 162 (5. 5 mg/kg)
EXAMPLE 163 (2. 6 mg/kg)

**INTERNATIONAL SEARCH REPORT**

International application No. **PCT/JP2023/029356**

**A. CLASSIFICATION OF SUBJECT MATTER**

***A61K 47/69***(2017.01)i; ***A61K 9/51***(2006.01)i; ***A61K 31/282***(2006.01)i; ***A61K 39/395***(2006.01)i; ***A61K 47/32***(2006.01)i; ***A61K 47/68***(2017.01)i; ***A61P 35/00***(2006.01)i; ***C08F 220/10***(2006.01)i; ***C08F 220/30***(2006.01)i
FI: A61K47/69; A61K47/68; A61K9/51; A61K47/32; A61K31/282; A61P35/00; A61K39/395 L; C08F220/10; C08F220/30

According to International Patent Classification (IPC) or to both national classification and IPC

**B. FIELDS SEARCHED**

Minimum documentation searched (classification system followed by classification symbols)

A61K47/69; A61K9/51; A61K31/282; A61K39/395; A61K47/32; A61K47/68; A61P35/00; C08F220/10; C08F220/30

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | JP 2022-503966 A (NORTH CAROLINA STATE UNIVERSITY) 12 January 2022 (2022-01-12)<br>claims 1-2, 8-10, 18-26, 81-86, paragraphs [0079]-[0082], [0108], [0120] | 1-31 |
| Y | NISHIYAMA, Nobuhiro et al. Development of polymeric micelles for targeting intractable cancers, Cancer Sci., 2016, vol.107, pp. 867-874<br>fig. 1 | 1-31 |
| Y | CUNNINGHAM, Victoria J. et al. Poly(glycerol monomethacrylate)-Poly(benzyl methacrylate) Diblock Copolymer Nanoparticles via RAFT Emulsion Polymerization: Synthesis, Characterization, and Interfacial Activity. Macromolecules, 2014, vol. 47, pp. 5613-5623<br>column Abstract | 1-31 |
| X<br><br>Y | JACKSON, Alexander W. et al. Octreotide Functionalized Nano-Contrast Agent for Targeted Magnetic Resonance Imaging. Biomacromolecules, 2016, vol. 17, pp. 3902-3910<br>column Abstract, p. 3903, left column, last paragraph to right column, 1st paragraph | 1-6, 12, 23-24, 26, 28-31<br><br>1-19, 23-24, 26-31 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 November 2023** | **14 November 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2023/029356**

**C. DOCUMENTS CONSIDERED TO BE RELEVANT**

| Category* | Citation of document, with indication, where appropriate, of the relevant passages | Relevant to claim No. |
|---|---|---|
| A | MARSDEN, Catherine J. et al. Crosslinked p(MMA) particles by RAFT emulsion polymerisation: tuning size and stability, Polymer Chemistry, 01 June 2022, vol. 13, pp. 4124-4135 entire text | 1-31 |
| A | JP 2003-231648 A (JSR CORP.) 19 August 2003 (2003-08-19) entire text | 1-31 |
| P, A | WO 2022/168967 A1 (KOWA CO., LTD.) 11 August 2022 (2022-08-11) entire text | 1-31 |

Form PCT/ISA/210 (second sheet) (January 2015)

**INTERNATIONAL SEARCH REPORT**
**Information on patent family members**

International application No.
**PCT/JP2023/029356**

| Patent document cited in search report | Publication date (day/month/year) | Patent family member(s) | Publication date (day/month/year) |
|---|---|---|---|
| JP 2022-503966 A | 12 January 2022 | US 2022/0034873 A1<br>claims, paragraphs [0122], [0123], [0147], [0159]<br>EP 3861087 A1<br>CN 113166642 A | |
| JP 2003-231648 A | 19 August 2003 | (Family: none) | |
| WO 2022/168967 A1 | 11 August 2022 | TW 202245797 A<br>entire text | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3270592 B
- US 8114965 B
- WO 2009130575 A
- US 2010226884 A
- US 7151164 B
- US 7109304 B
- US 7300655 B
- US 7312318 B
- US 7074403 B
- US 7387773 B
- US 7612180 B
- US 6676924 B
- US 7541440 B
- US 7138496 B
- WO 2016142049 A


### Non-patent literature cited in the description


- **H. CABRAL et al.** *Nat. Nanotechnol.*, 2011, vol. 6, 815-823
- **JOSE A. POMPOSO**. *Single-Chain Polymer Nanoparticles: Synthesis, Characterization, Simulations, and Applications*, 2017
- **CHARI, R.V. et al.** *Angew.Chem.Int.Ed.*, 2014, vol. 53, 3796-3827
- **JAGADEESH, D.** ; **SMITH, M.R.** *Curr.Treat.Options Oncol.*, 2016, vol. 17, 55
- **DUCRY, L.** ; **STUMP, B.** *Bioconjugate Chem.*, 2010, vol. 21, 5-13
- **CASI, G.** ; **NERI, D.** *J.Controlled Release*, 2012, vol. 161, 422-428
- **WU, A. M.** ; **SENTER, P.D.** *Nat.Biotechnol.*, 2005, vol. 23, 1137-1146